# FASCICULE DE BREVET EUROPEEN

(11) **EP 1 280 936 B1**
(45) Date de publication et mention de la délivrance du brevet: **04.10.2006**
(21) Numéro de dépôt: 01929713.4
(22) Date de dépôt: 26.04.2001
(51) Int. Cl.: C12Q 1/68

(54) **PROCEDE DE DETECTION ET D'IDENTIFICATION DE LA PRESENCE DE MATIERES BIOLOGIQUES PROVENANT D'OISEAUX, ET OLIGONUCLEOTIDES POUR SA MISE EN OEUVRE**
VERFAHREN ZUM NACHWEIS UND ZUR IDENTIFIZIERUNG VON VOGELMATERIALEN , UND OLIGONUKLEOTIDEN ZUR AUSFÜHRUNG DAVON
METHOD FOR DETECTING AND IDENTIFYING THE PRESENCE OF BIOLOGICAL SUBSTANCES DERIVED FROM BIRDS, AND OLIGONUCLEOTIDES THEREFOR

(30) Priorité: 09.05.2000 FR 0005850
(43) Date de publication de la demande: 05.02.2003
(73) Titulaire: CENTRE NATIONAL DE LA RECHERCHE SCIENTIFIQUE (CNRS), 75794 Paris Cedex 16 (FR)
(72) Inventeur: DONNE-GOUSSE, Carole, F-76230 Bois-Guillaume (FR); LAUDET, Vincent, F-69002 Lyon (FR); HÄNNI, Catherine, F-69002 Lyon (FR)
(74) Mandataire: Grosset-Fournier, Chantal Catherine
(86) Numéro de dépôt international: PCT/FR2001/001279
(87) Numéro de publication internationale: WO 2001/084903

(56) Documents cités:
- KAHN N ET AL: "Molecular analysis of genetic variation among large- and small-bodied Sage Grouse using mitochondrial control-region sequences" THE AUK, vol. 116, juillet 1999 (1999-07), pages 819-24, XP000982665
- LEE J ET AL: "Random amplified polymorphic DNA polymerase chain reaction (RAPD PCR) fingerprints in forensic species identification" FORENSIC SCIENCE INTERNATIONAL, vol. 67, 1994, pages 103-107, XP002162233
- Biosis, AN=PREV199799378157 TI - Species identification of meats and meat products by PCR. AU - Fei Sha et al PUB - Animal science and Technology - 1996 Vol - 67 PG - 900-905 XP002162236
- WENINK P ET AL: "Hypervariable-control-region sequences reveal global population structuring in a lon-distance migrant shorebird, the Dunlin (Calidris alpina)" PROCEEDINGS OF THE NATIONAL ACADEMY OF SCIENCE, USA, vol. 90, janvier 1993 (1993-01), pages 94-98, XP002162234
- RANDI E ET AL: "Organization and evolution of the mitochondrial DNA control region in the avian genus Alectoris" MOLECULAR EVOLUTION, vol. 47, - 1998 pages 449-62, XP000982690
- FUMIHITO A ET AL: "The genetic link between the chinese bamboo partridge (Bambusicola thoracica) and the chicken and junglefowls of the genus Gallus" PROCEEDINGS OF THE NATIONAL ACADEMY OF SCIENCE, USA, vol. 92, novembre 1995 (1995-11), pages 11053-056, XP002162235
- LEFRANCOIS C. ET AL: "L'ADN anti-fraudes" BIOFUTUR, no. 165, - mars 1997 (1997-03) pages 27-30,

## Description

La présente invention a pour objet un procédé de détection et d'identification de la présence de matières biologiques provenant d'oiseaux, notamment d'ansériformes, de galliformes ou de struthioniformes, dans un échantillon de matière organique.

Elle a en outre pour objet les oligonucléotides pour la mise en oeuvre de ce procédé, et les fragments d'ADN obtenus à l'aide desdits oligonucléotides.

La valeur économique croissante de certaines espèces animales utilisées pour l'alimentation humaine est liée à un déséquilibre important entre l'offre et la demande du marché. Ceci a pour conséquence directe la mise en place d'une pratique accrue de l'adultération des aliments.

L'un des modes d'adultération des aliments le plus courant consiste en la substitution de composants provenant d'espèces animales à haute valeur commerciale par ceux provenant d'espèces de moindre valeur, ou même par des composants d'origine végétale comme le soja. Ainsi par exemple les viandes de porc, de poulet ou même de cheval sont fréquemment utilisées en remplacement du boeuf, le mouton à la place de la viande de chèvre, ou le canard à la place de l'oie.

L'adultération " inter-spécifique " des produits alimentaires est donc un problème à la fois économique, de santé publique et de préservation de l'environnement, qui concerne aussi bien les consommateurs, les distributeurs, les producteurs que les administrations chargées de l'hygiène alimentaire et de la répression des fraudes.

Il est donc important de pouvoir non seulement déterminer la présence dans les aliments de matières biologiques provenant d'espèces animales, mais également d'identifier l'origine de ces matières biologiques présentes dans les aliments.

Dans le domaine agro-alimentaire, la caractérisation d'espèces animales fait appel aux techniques biochimiques d'analyse des protéines (électrophorèse et immunoanalyse) ou aux techniques de la chimie (la chromatographie principalement).

Ainsi, l'électrophorèse sur gel de polyacrylamide en conditions dénaturantes a été essentiellement développée pour l'analyse des aliments cuits (analyse de peptides ou de protéines dénaturés et coagulés par la cuisson (Patterson R.L.S., 1985, Biochemical identification of meat species, Elsevier ed)). Cette technique est à présent supplantée par l'électrophorèse en gradient de pH (focalisation isoélectrique ou " IEF "), beaucoup plus résolutive ; cependant la focalisation isoélectrique est une technique délicate à mettre en oeuvre.

Quant à l'immunoanalyse, de nombreux travaux ont été consacrés à l'utilisation d'anticorps spécifiques pour la mise en évidence de l'adultération de produits camés par des protéines végétales, ainsi qu'à l'identification d'espèces animales dans les produits laitiers et les viandes (Hernandez *et al*., 1994, Food and Agricultural Immunology, 6, 95-104). Des anticorps dirigés contre des protéines du sarcoplasme musculaire ont été par exemple utilisés pour caractériser diverses espèces domestiques telles que le porc, le boeuf, le cheval ou le poulet. L'immunoanalyse présente cependant elle aussi d'importantes limitations.

Pour ce qui est de la chimie analytique, ce sont surtout les techniques de chromatographie en phase liquide ou gazeuse qui ont été utilisées pour le contrôle de produits carnés : dosage de peptides dans la viande (Barai *et al*., 1992, Trends in Food Science and Technology, 4, 395-401; Carnegie P.R. *et al*., 1983, Journal of chromatography, 261 : 153-157) ou d'acides gras dans les graisses par exemple (Youssef M.K.E. *et al*., 1988, Food Chemical, 30 : 167-180). Ces techniques sont cependant dépourvues d'une réelle spécificité et limitées aux espèces les plus communes telles que le porc.

Si ces techniques restent utilisées pour l'identification dans les aliments d'espèces communes (en particulier les espèces domestiquées), la multiplication du nombre d'espèces sauvages concernées (gibiers, poissons, crustacées, coquillages, mollusques) fait que la plupart de ces techniques trouvent aujourd'hui leurs limites.

L'analyse du génome au moyen de sondes nucléiques représente à l'évidence une nouvelle alternative pour l'identification d'espèces, encore peu développée à l'heure actuelle. En effet, les travaux menés sur l'ADN ancien (ou ADN fossile) depuis le début des années 1990 ont démontré que l'ADN est une molécule très stable après la mort, malgré l'action du temps et de l'environnement (Brown *et al*., 1994, Bioessays, 16 (10) : 719-26). Toutefois, quand il subsiste dans des substrats anciens, cet ADN est très dégradé et présent en petites quantités, sous formes de molécules abîmées et chimiquement modifiées (Pääbo *et al*., 1989, Journal of Biology Chemistry. 264, 9709-9712). Ces caractéristiques sont dues essentiellement aux phénomènes d'hydrolyse et d'oxydation (Lindahl, 1993, Nature, 362, 709-715). Grâce à la technique de PCR (" Polymerase Chain Reaction "), qui constitue un outil d'une puissance analytique remarquable, il est possible de multiplier " *in vitro* " de façon quasi exponentielle un fragment donné d'ADN. En amplifiant de l'ADN d'une préparation alimentaire, qui a subi des modifications telles que la cuisson, le fumage ..., des phénomènes d'hydrolyse et d'oxydation, il sera possible d'identifier chaque constituant d'origine animale ou végétale. La PCR a ainsi été récemment utilisée pour la caractérisation de la viande de porc cuite (Meyer *et al*., 1995, Journal of AOAC International, 78, (6) 1542 - 1551)), de mouton ou de chèvre (Chikuni *et al*., 1994, Animal Science and Technology, 65 (6), 571-579) par amplication de séquences spécifiques de l'espèce recherchée. De même, l'amplification de séquences spécifiques du chromosome Y a permis de déterminer le sexe de carcasses de boucherie d'origine bovine et ovine (Cotinot C, *et al*., 1991, Genomics. 10 (3) : 646-53, Apparao K.B.C., 1995, Meat Science, 39 (1) 123-126).

La demande internationale WO 98/50401 a pour objet un procédé de détection de la présence de matières biologiques d'origine bovine dans un échantillon de matière organique. Le procédé décrit dans cette référence fait appel à la PCR à l'aide d'oligonucléotides appropriés. Cependant, le procédé décrit dans cette référence permet uniquement de détecter la présence ou l'absence d'une seule espèce bovine, à savoir l'espèce *Bos taurus*, et ainsi ne permet pas de détecter et d'identifier la présence de plusieurs espèces bovines.

Les canards, oies, poulets, dindes, autruches sont des oiseaux très utilisés dans le domaine agro-alimentaire (foie gras, pâtés, viandes, graisses, plats cuisinés, oeufs etc....) et sont donc très exposés à l'adultération. Par exemple, le foie gras d'oie est plus cher et de meilleur goût que celui de canard et il existe une fraude importante à ce niveau.

Par ailleurs, l'adultération des espèces susmentionnées peut concerner également des produits manufacturés (plumes, duvets, parures à bases de plumes etc...), des oeufs de collection, plumes de collection, des animaux vivants d'élevages ou sauvages, des animaux taxidermisés etc....

Les canards, oies, poulets, dindes, autruches etc... appartiennent à la classe des Aves qui regroupe l'ensemble des oiseaux ; les Aves forment deux divisions : les paléognathes et les néognathes. A titre d'exemple, les canards, oies et cygnes font partie de la famille des anatidés, et appartiennent à l'ordre des ansériformes qui fait partie des néognathes. Les poulets, cailles et dindes font partie la famille des gallinacés et appartiennent à l'ordre des galliformes, qui fait partie lui aussi des néognathes. Les autruches font partie de la famille des struthionidés et appartiennent à l'ordre des struthioniformes, qui fait partie des paléognathes.

L'ADN mitochondrial (ADNmt) des oiseaux a la particularité de ne pas être organisé de la même façon que celui des mammifères. En effet, chez les oiseaux le gène ND6 qui code pour le composant 6 de la chaîne respiratoire se situe entre la région de contrôle de la réplication et le gène codant pour le cytochrome b, alors que chez les mammifères l'enchaînement est le suivant : région de contrôle de la réplication, gène codant pour le cytochrome b et gène ND6 (Desjardin *et al*., 1990, Current Genetic, 17, 515-518).

L'ADNmt est un excellent marqueur d'espèces qui est souvent utilisé en phylogénie. En effet, selon l'espèce que l'on étudie, certaines portions de l'ADNmt permettent de différencier des espèces entre elles, d'autres ont un pouvoir de résolution plus fin et permettent de distinguer des populations différentes (races géographiques, sous-espèces) : région de contrôle de la réplication de l'ADNmt, régions codant pour le cytochrome b ou codant pour les ARN mitochondriaux (ARN 12S ou ARN 16S).

D'un point de vue technique, des études ont été conduites sur l'ADNmt des anatidés. Chez les anatidés, l'ADNmt n'a été séquencé en entier que pour une seule espèce sauvage : *Aythya americana* ; toutefois, il a été de nombreuses fois séquencé au niveau des différentes régions décrites précédemment chez des espèces domestiques, comme le canard colvert (*Anas platyrhynchos*), le canard de barbarie (*Cairina moschata*), l'oie commune (*Anser anser*), mais aussi chez des espèces sauvages. La plupart de ces études proposent d'analyser la diversité génétique des espèces en comparant les séquences entres elles (Liu *et al*., 1996, Comp. Biochem. Physiol. 115B (2) : 209-214). D'autres études se basent sur une région précise de l'ADNmt et calculent des taux de divergence afin d'étudier l'évolution d'une espèce par rapport à une autre (Ramirez *et al*., 1993, Journal of Molecular Evolution, 37, : 296-310). Il est possible également de retracer l'histoire évolutive d'une espèce (Quinn *et al*., 1993, Journal of Molecular Evolution, 37 : 417 - 425; Shield *et al*., 1987, Evolution, 41 (3) : 662-666), et d'apporter de nouveaux éléments quant à la place d'une espèce sur un arbre phylogénique (Sraml *et al*., 1996, Autraslian Journal of Zoology, 44 : 47-58; Quinn, 1992, Molecular Ecololy, 1 : 105-117, Johnson *et al*., 1998, Molecular Phylogenetics and Evolution, 10 : 82-84).

L'article de Fei Sha et al. *(Animal Science and Technology,* 1996, vol. 67, pages 900-905) décrit l'application de la PCR à l'identification d'espèces de viandes ou de produits carnés. Il mentionne notamment l'utilisation de deux amorces définies dans le cas du poulet, et fabriquées d'après les séquences publiées d'ADN mitochondrial de poulet. Les produits d'amplification sont ensuite analysés par électrophorèse sur gel et donnent un fragment spécifique d'ADN de 400 bp en ce qui concerne le poulet. Ce procédé permet de déterminer, dans un produit carné, la présence de poulet, par comparaison des produits d'amplification obtenus. Cependant, les amorces utilisées dans cet article permettent de détecter l'éventuelle présence de poulet mais ne permet pas de détecter la présence de matière organique provenant d'oiseaux autres que le poulet.

L'article de Fumihito et al. *(Proc. Natl. Acad. Sci. USA,* 1995, vol. 92, pages 11053-11056) concerne le lien génétique entre le bambusicole de Chine et le poulet et les coqs sauvages du genre *Gallus.* Les auteurs de cet article ont comparé les séquences de la région de contrôle de l'ADN mitochondrial de 16 espèces d'oiseaux appartenant à la sous-famille *Phasianinae.* Leur but était de définir la position du genre *Gallus* dans la sous-famille *Phasianinae* par rapport aux autres membres de cette sous-famille. Cet article ne mentionne donc en aucun cas un procédé de détection et d'identification de la présence de matières biologiques provenant d'oiseaux, notamment d'ansériformes, de galliformes ou de struthioniformes, dans un échantillon de matière organique.

Il ressort donc de l'étude de l'état de la technique que des travaux d'analyses de l'ADN de certaines espèces d'oiseaux, notamment d'anatidés, ont déjà été effectués.

Néanmoins, aucun des documents de l'état de la technique ne décrit de méthode spécifique et sensible de détection et d'identification de la présence de matières biologiques provenant d'oiseaux dans un échantillon de matière organique, applicable à tous les genres d'oiseaux, et notamment espèces d'oiseaux, dans des échantillons de matière organique présentant des compositions très variées.

Le demandeur s'est donc attaché à développer une méthode sensible et fiable permettant de pallier à cette absence.

L'un des buts est de fournir une méthode de détection de la présence de matières biologiques provenant d'oiseaux dans un échantillon de matière organique.

L'un des autres buts est de fournir une méthode d'identifcation du genre, notamment de l'espèce d'oiseau présente dans un échantillon de matière organique.

Un autre but est de fournir une méthode permettant de distinguer des espèces d'oiseaux phylogénétiquement proches mais de valeurs commerciales différentes.

Un autre but est de fournir une méthode d'identification du genre, notamment de l'espèce d'oiseau présente dans des aliments frais ou transformés (cuits, lyophilisés, séchés, saumurés, appertisés ...).

Un procédé de détection de la présence de matières biologiques provenant d'oiseaux, notamment d'ansériformes, de galliformes ou de struthioniformes dans un échantillon de matière organique, est décrit, caractérisé en ce que l'on détermine la présence d'ADN provenant d'oiseaux, notamment d'ansériformes, de galliformes ou de struthioniformes dans ladite matière organique par amplification d'une séquence d'ADN spécifique du génome des oiseaux, notamment des ansériformes, des galliformes ou des struthioniformes, et contenue dans l'ADN extrait dudit échantillon, à savoir une séquence présente dans les génomes des oiseaux, et notamment des ansériformes, des galliformes ou des struthioniformes mais absente dans les génomes des autres genres animaliers, et notamment des autres espèces animales, ladite séquence étant une séquence d'ADN mitochondrial spécifique du génome des oiseaux amplifiée à l'aide d'au moins un oligonucléotide choisi parmi les oligonucléotides tels que définis ci-après.

Un procédé de détection de la présence de matières biologiques provenant d'oiseaux, et notamment d'ansériformes, de galliformes ou de struthioniformes dans un échantillon de matière organique et d'identification du genre, notamment de l'espèce d'oiseau, notamment d'ansériforme, de galliforme ou de struthioniforme présente dans ledit échantillon est décrit, caractérisé en ce que l'on détermine la présence d'ADN provenant d'oiseaux, et notamment d'ansériformes, de galliformes ou de struthioniformes dans ladite matière organique par amplification d'une séquence d'ADN spécifique du génome des oiseaux, et notamment des ansériformes, des galliformes ou des struthioniformes, et contenue dans l'ADN extrait dudit échantillon, à savoir une séquence présente dans les génomes des oiseaux, et notamment des ansériformes, des galliformes ou des struthioniformes mais absente dans les génomes des autres genres animaliers, notamment des autres espèces animales, et en ce que l'on compare la séquence d'ADN spécifique du génome des oiseaux, notamment des ansériformes, des galliformes ou des struthioniformes ainsi amplifiée avec d'autres séquences d'ADN du génome des oiseaux, et notamment des ansériformes, des galliformes ou des struthioniformes, ladite séquence d'ADN spécifique du génome des oiseaux, notamment des ansériformes, des galliformes ou des struthioniformes ainsi amplifiée présentant au moins 50% d'identité, notamment 60% d'identité avec les autres susdites séquences d'ADN du génome des oiseaux, et notamment des ansériformes, des galliformes ou des struthioniformes, ladite séquence étant une séquence d'ADN mitochondrial spécifique du génome des oiseaux amplifiée à l'aide d'au moins un oligonucléotide choisi parmi les oligonucléotides tels que définis ci-après.

On entend par genre une catégorie obligatoire à laquelle toute espèce doit appartenir et qui contient une espèce ou un groupe d'espèces (Wily, 1981).

On entend par espèce un groupe de population réellement ou partiellement capable de se croiser et qui est reproductivement isolé des autres groupes ayant la même propriété.

L'espèce animale se divise en sous-espèces, races, variétés et souches ; plusieurs espèces voisines forment un genre qui est lui-même une subdivision de la famille.

On entend par matière organique toute matière solide ou liquide que l'on suppose avoir au moins partiellement une origine organique.

Le pourcentage d'identité concerne le résultat de la comparaison des acides nucléiques de la séquence d'ADN que l'on cherche à identifier, avec ceux des séquences d'ADN connues des oiseaux, notamment des ansériformes, des galliformes ou des struthioniformes.

Ainsi, lorsque la séquence d'ADN spécifique du génome des oiseaux amplifiée présente au moins 50% d'identité, notamment 60% avec les autres séquences d'ADN connues du génome des oiseaux, on pourra en déduire que l'échantillon de matière organique à analyser contient des matières biologiques provenant d'oiseaux, notamment d'ansériformes, de galliformes ou de struthioniformes.

Si la séquence d'ADN spécifique du génome des oiseaux amplifiée présente moins de 50% d'identité avec les autres séquences d'ADN connues du génome des oiseaux, on pourra donc en déduire que l'échantillon de matière organique à analyser ne contient pas de matières biologiques provenant d'oiseaux, notamment d'ansériformes, de galliformes ou de struthioniformes.

Selon un mode de réalisation avantageux, le procédé permet de détecter et/ou d'identifier la présence d'ansériformes, notamment d'anatidés choisis dans le groupe constitué par les canards tels que le canard pilet (*Anas acuta*), le canard mandarin (*Aix galericulata*), le canard souchet *(Anas clypeata*), la sarcelle d'été (*Anas querquedula*), l'eider à duvet (*Somateria mollissima*), les oies telles que l'oie naine (*Anser erythropus*), l'oie des neiges (*Anser caerulescens*), la bernache du Canada *(Branta canadensis*), et les cygnes tels que le cygne sauvage *(Cygnus cygnus*), le cygne noir (*Cygnus atratus*), le cygne commun *(Cygnus color)* et notamment les espèces domestiques de canards telles que le canard colvert *(Anas platyrhynchos*), le canard de barbarie (*Cairina moschata*) ou l'espèce domestique d'oie telle que l'oie commune *(Anser anser)*.

Selon un autre mode de réalisation avantageux, le procédé permet de détecter et/ou d'identifier la présence de galliformes, notamment de gallinacés choisis dans le groupe constitué par les poulets, les cailles et les dindes, et notamment les espèces telles que *Gallus gallus, Coturnix coturnix* ou *Meleagris gallopavo*.

Selon un autre mode de réalisation avantageux, le procédé permet de détecter et/ou d'identifier la présence de struthioniformes, notamment de struthionidés tels que les autruches, et notamment les espèces telles que *Struthio camelus*.

La séquence amplifiée du génome des oiseaux, notamment des ansériformes, des galliformes ou des struthioniformes, est d'origine mitochondriale.

Le choix d'une séquence mitochondriale est particulièrement avantageux car, dans une cellule animale il y a environ 100 à 1000 copies d'ADN mitochondrial pour une copie d'ADN nucléaire. En cas de dégradation de l'ADN, la probabilité de détecter de l'ADN mitochondrial est donc beaucoup plus importante que la probabilité de détecter de l'ADN nucléaire. L'ADN mitochondrial peut donc être plus sûrement détecté dans des matières organiques dans lesquelles l'ADN est soumis à divers facteurs physiques (température, pression, ...) chimiques ou biochimiques tendant à sa dégradation.

Selon un mode de réalisation avantageux du procédé décrit, l'ADN extrait de l'échantillon de matière organique est :
- de l'ADN non dégradé provenant notamment d'un échantillon frais ou,
- de l'ADN dégradé, provenant notamment d'un échantillon transformé, notamment cuit, lyophilisé, séché, saumuré, appertisé ou pasteurisé.

Préférentiellement, l'amplification de la séquence d'ADN spécifique du génome des oiseaux, notamment d'ansériformes, de galliformes ou de struthioniformes est effectuée par la méthode d'amplification en chaîne par polymérase (PCR), comprenant une répétition du cycle des étapes suivantes :
- chauffage de l'ADN extrait de l'échantillon de matière organique, de façon à séparer l'ADN en deux brins monocaténaires,
- hybridation d'amorces oligonucléotidiques telles que définies ci-dessous aux brins d'ADN monocaténaires à une température adéquate et,
- élongation desdites amorces oligonucléotidiques par une polymérase à une température adéquate, pour obtenir une séquence d'ADN ou un fragment d'ADN amplifié(e) spécifique du génome des oiseaux, notamment d'ansériformes, de galliformes ou de struthioniformes.

Dans ce qui suit, les amorces oligonucléotidiques peuvent encore être appelées " oligonucléotides " ou " amorces ". La séquence d'ADN amplifiée obtenue à l'issue de la réaction de polymérisation en chaîne (PCR) à l'aide des amorces de l'invention, peut encore être dénommée dans ce qui suit " fragment d'ADN amplifié ", " fragment d'ADN " ou " produit d'amplification ".

Un procédé d'obtention d'un fragment d'ADN provenant d'oiseaux, notamment d'ansériformes et en particulier d'anatidés, présentant une taille et une séquence déterminées, spécifique des anatidés, notamment des genres *Anas, Cairina* et *Anser,* et en particulier des espèces *Anas platyrhynchos, Cairina moschata* et *Anser anser*, à partir d'un échantillon de matière organique, est également décrit, procédé par lequel on amplifie, par réaction de polymérisation en chaîne (PCR), une séquence déterminée du génome d'anatidés présente dans les génomes des anatidés mais absente dans les génomes des autres espèces animales.

Un procédé d'obtention d'un fragment d'ADN provenant d'oiseaux, notamment de galliformes et en particulier de gallinacés, présentant une taille et une séquence déterminées, spécifique des gallinacés, notamment des genres *Gallus, Coturnix* et *Meleagris,* et en particulier des espèces *Gallus gallus, Coturnix coturnix* et *Meleagris gallopavo*, à partir d'un échantillon de matière organique, est également décrit, procédé par lequel on amplifie, par réaction de polymérisation en chaîne (PCR), une séquence déterminée du génome des gallinacés, présente dans les génomes gallinacés mais absente dans les génomes des autres genres ou espèces animales.

Un procédé d'obtention d'un fragment d'ADN provenant d'oiseaux, notamment de struthioniformes, et en particulier des struthionidés présentant une taille et une séquence déterminées, spécifique des struthionidés, notamment du genre *Struthio,* et en particulier de l'espèce *Struthio camelus*, à partir d'un échantillon de matière organique, est également décrit procédé par lequel on amplifie, par réaction de polymérisation en chaîne (PCR), une séquence déterminée du génome des struthionidés, présente dans les génomes des struthionidés mais absente dans les génomes des autres espèces genres ou animales.

Selon un mode de réalisation avantageux du procédé décrit, la séquence amplifiée du génome des oiseaux, notamment des ansériformes, des galliformes ou des struthioniformes, est située dans la partie centrale de la région de contrôle de la réplication de l'ADN mitochondrial, délimitée par les nucléotides situés aux environs des positions 440 et 750, et notamment aux environs des positions 450 et 700, et de préférence aux environs des positions 459 et 665 de la région de contrôle de la réplication de l'ADN mitochondrial, lesdites positions étant définies d'après Desjardins et Morais, 1990, J. Mol. Biol., 599-634.

Selon un autre mode de réalisation avantageux du procédé décrit, la séquence amplifiée du génome des oiseaux, notamment des ansériformes, des galliformes ou des struthioniformes est située dans la partie 5' de la région de contrôle de la réplication de l'ADN mitochondrial, délimitée par les nucléotides situés aux environs des positions 1 et 439, et notamment aux environs des positions 80 et 433, et de préférence aux environs des positions 150 et 433 de la région de contrôle de la réplication de l'ADN mitochondrial, ou dans la partie 3' de la région de contrôle de la réplication de l'ADN mitochondrial, délimitée par les nucléotides situés aux environs des positions 750 et 1227, et notamment aux environs des positions 780 et 1227, et de préférence aux environs des positions 800 et 1227 de la région de contrôle de la réplication de l'ADN mitochondrial, lesdites positions étant définies d'après Desjardins et Morais, 1990, J. Mol. Biol., 599-634.

Sont également décrits les oligonucléotides choisis parmi ceux :
(1) - présentant une identité de séquence d'au moins 80%, préférentiellement 90% et avantageusement 95% avec un oligonucléotide constitué d'une séquence de 15 à 25 nucléotides, notamment de 20 à 25 nucléotides, comprise dans la séquence SEQ ID N°32 suivante (positions 455 à 479 d'après Desjardins et Morais, 1990, J. Mol. Biol., 212, 599-634) :
   ACG TGA CYA GCY TCA GGC CCA TAC G
   dans laquelle Y est C ou T,
   - ou comprenant la séquence SEQ ID N°33 suivante Desjardins et Morais, 1990, J. Mol. Biol., 212, 599-634) :
      CTT CAG GCC CAT ACG
   - ou constitués par la séquence SEQ ID N°1 suivante (positions 465 à 479 d'après Desjardins et Morais, 1990, J. Mol. Biol., 212, 599-634) :
      ACT AGC TTC AGG CCC ATA CG
   ou ceux,
(2) - présentant une identité de séquence d'au moins 80%, préférentiellement 90% et avantageusement 95% avec un oligonucléotide constitué d'une séquence de 15 à 25 nucléotides, notamment de 20 à 25 nucléotides, comprise dans la séquence SEQ ID N°34 suivante (positions 484 à 508 d'après Desjardins et Morais, 1990, J. Mol. Biol., 212, 599-634) :
   CCC TAA ACC CCT CGC CCT CCT CAC A
   - ou comprenant la séquence SEQ ID N°35 suivante (positions 493 à 508 d'après Desjardins et Morais, 1990, J. Mol. Biol., 212, 599-634) :
      CTC GCC CTC CTC ACA
   - ou constitués par la séquence SEQ ID N°2 suivante (positions 488 à 508 d'après Desjardins et Morais, 1990, J. Mol. Biol., 212, 599-634) :
      AAC CCC TCG CCC TCC TCA CA
   ou ceux,
(3) - présentant une identité de séquence d'au moins 80%, préférentiellement 90% et avantageusement 95% avec un oligonucléotide constitué d'une séquence de 15 à 25 nucléotides, notamment de 20 à 25 nucléotides, comprise dans la séquence SEQ ID N°36 suivante (positions 537 à 561 d'après Desjardins et Morais, 1990, J. Mol. Biol., 212, 599-634) :
   GGG CCA TCM ATT GGG TTC ACT CAC C
   dans laquelle M est A ou C,
   - ou comprenant la séquence SEQ ID N°37 suivante (positions 547 à 561 d'après Desjardins et Morais, 1990, J. Mol. Biol., 212, 599-634) :
      TTG GGT TCA CTC ACC
   - ou constitués par la séquence SEQ ID N°3 suivante (positions 542 à 561 d'après Desjardins et Morais, 1990, J. Mol. Biol., 212, 599-634) :
      ATC AAT TGG GTT CAC TCA CC
   ou ceux,
(4) - présentant une identité de séquence d'au moins 80%, préférentiellement 90% et avantageusement 95% avec un oligonucléotide constitué d'une séquence de 15 à 25 nucléotides, notamment de 20 à 25 nucléotides, comprise dans la séquence SEQ ID N°38 suivante (positions 574 à 599 d'après Desjardins et Morais, 1990, J. Mol. Biol., 212, 599-634) :
   GGT RRA VST MNT CCA CAG ATG CCA C
   dans laquelle R est A ou G, V est A, C ou G, N est A, C, G ou T, S est C ou G, M est A ou C,
   - ou comprenant la séquence SEQ ID N°39 suivante (positions 584 à 599 d'après Desjardins et Morais, 1990, J. Mol. Biol., 212,599-634) :
      TTC CAC AGA TGC CAC
   - ou constitués par la séquence SEQ ID N°4 suivante (positions 580 à 599 d'après Desjardins et Morais, 1990, J. Mol. Biol., 212, 599-634) :
      AAG TAT TCC ACA GAT GCC AC
   ou ceux,
(5) - présentant une identité de séquence d'au moins 80%, préférentiellement 90% et avantageusement 95% avec un oligonucléotide constitué d'une séquence de 15 à 25 nucléotides, notamment de 20 à 25 nucléotides, comprise dans la séquence SEQ ID N°40 suivante (positions 639 à 664 d'après Desjardins et Morais, 1990, J. Mol. Biol., 212, 599-634) :
   CMG GAA RAR ADA ARW RGA ACC AGA G
   dans laquelle R est A ou G, D est A, G ou T, M est A ou C, W est A ou T,
   - ou comprenant la séquence SEQ ID N°41 suivante (positions 648 à 664 d'après Desjardins et Morais, 1990, J. Mol. Biol., 212, 599-634) :
      A TAA AAA GAA CCA GAG
   - ou constitués par la séquence SEQ ID N°5 suivante (positions 645 à 664 d'après Desjardins et Morais, 1990, J. Mol. Biol., 212, 599-634) :
      AAA AAT AAA AGG AAC CAG AG
   ou ceux,
(6) - présentant une identité de séquence d'au moins 80%, préférentiellement 90% et avantageusement 95% avec un oligonucléotide constitué d'une séquence de 15 à 25 nucléotides, notamment de 20 à 25 nucléotides, comprise dans la séquence SEQ ID N°42 suivante (positions 797 à 820 d'après Desjardins et Morais, 1990, J. Mol. Biol., 212, 599-634):
   WVK CAY MYY KRY ACT GAT GCA CTT T
   dans laquelle Y est C ou T, K est G ou T, R est A ou G, M est A ou C, W est A ou T, V est A, C ou G, M est A ou C,
   - ou comprenant la séquence SEQ ID N°43 suivante (positions 807 à 820 d'après Desjardins et Morais, 1990, J. Mol. Biol., 212, 599-634) :
      ACA CTG ATG CAC TTT
   - ou constitués par la séquence SEQ ID N°11 suivante (positions 802 à 820 d'après Desjardins et Morais, 1990, J. Mol. Biol., 212, 599-634) :
      CCT TGA CAC TGA TGC ACT TT
   ou ceux,
(7) - présentant une identité de séquence d'au moins 80%, préférentiellement 90% et avantageusement 95% avec un oligonucléotide constitué d'une séquence de 15 à 25 nucléotides, notamment de 20 à 25 nucléotides, comprise dans la séquence SEQ ID N°44 suivante (positions 1220 à 1224 d'après Desjardins et Morais, 1990, J. Mol. Biol., 212, 599-634) :
   GCY TTD DND KKR WRS WAT GTG GAC G
   dans laquelle Y est C ou T, D est A, G ou T, B est C, G ou T, K est G ou T, W est A ou T, N est A, C, G ou T, R est A ou G, et S est C ou G,
   - ou comprenant la séquence SEQ ID N°45 suivante (positions 1210 à 1224 d'après Desjardins et Morais, 1990, J. Mol. Biol., 212, 599-634) :
      TAA GCT ATG TGG ACG
   - ou constitués par la séquence SEQ ID N°12 suivante (positions 1205 à 1224 d'après Desjardins et Morais, 1990, J. Mol. Biol., 212, 599-634) :
      TGT GGT AAG CTA TGT GGA CG
   ou ceux,
(8) - présentant une identité de séquence d'au moins 80%, préférentiellement 90% et avantageusement 95% avec un oligonucléotide constitué d'une séquence de 15 à 25 nucléotides, notamment de 20 à 25 nucléotides, comprise dans la séquence SEQ ID N°46 suivante (positions 180 à 206 d'après Desjardins et Morais, 1990, J. Mol. Biol., 212, 599-634) :
   MTN MHM BDN HMV HVN NCA TAC CCT A
   dans laquelle Y est C ou T, M est A ou C, H est A, C ou T, B est C, G ou T, D est A, G ou T, N est A, C, G ou T, W est A ou T, V est A, C ou G,
   - ou comprenant la séquence SEQ ID N°47 suivante (positions 190 à 206 d'après Desjardins et Morais, 1990, J. Mol. Biol., 212, 599-634) :
      AAC GGA CAT ACC CTA
   - ou constitués par la séquence SEQ ID N°14 suivante (positions 185 à 206 d'après Desjardins et Morais, 1990, J. Mol. Biol., 212, 599-634) :
      CAT GTC AAC GGA CAT ACC CTA
   ou ceux,
(9) - présentant une identité de séquence d'au moins 80%, préférentiellement 90% et avantageusement 95% avec un oligonucléotide constitué d'une séquence de 15 à 25 nucléotides, notamment de 20 à 25 nucléotides, comprise dans la séquence SEQ ID N°48 suivante (positions 355 à 376 d'après Desjardins et Morais, 1990, J. Mol. Biol., 212, 599-634) :
   CAT RAG NWA WKG KYG GGT GGA GAG G
   dans laquelle R est A ou G, N est A, C, G ou T, W est A ou T, K est G ou T, Y est C ou T,
   - ou comprenant la séquence SEQ ID N°49 suivante (positions 365 à 376 d'après Desjardins et Morais, 1990, J. Mol. Biol., 212, 599-634) :
      GGG TGG GTG GAG AGG
   - ou constitués par la séquence SEQ ID N°15 suivante (positions 361 à 376 d'après Desjardins et Morais, 1990, J. Mol. Biol., 212, 599-634) :
      TAA TGG GTG GGT GGA GAG G
   ou ceux,
(10) - présentant une identité de séquence d'au moins 80%, préférentiellement 90% et avantageusement 95% avec un oligonucléotide constitué d'une séquence de 15 à 25 nucléotides, notamment de 20 à 25 nucléotides, comprise dans la séquence SEQ ID N°50 suivante (positions 833 à 855 d'après Desjardins et Morais, 1990, J. Mol. Biol., 212, 599-634) :
   GTT AWK GYY MYN CTC CGC RGC CCT T
   dans laquelle W est A ou T, K est G ou T, Y est C ou T, M est A ou C, Y est C ou T, N est A, C, G ou T, R est A ou G,
   - ou comprenant la séquence SEQ ID N°51 suivante (positions 843 à 855 d'après Desjardins et Morais, 1990, J. Mol. Biol., 212, 599-634) :
      TCC TCC GCA GCC CTT
   - ou constitués par la séquence SEQ ID N°16 suivante (positions 837 à 855 d'après Desjardins et Morais, 1990, J. Mol. Biol., 212, 599-634) :
      ATG CTC TCC TCC GCA GCC CTT
   ou ceux,
(11) - présentant une identité de séquence d'au moins 80%, préférentiellement 90% et avantageusement 95% avec un oligonucléotide constitué d'une séquence de 15 à 25 nucléotides, notamment de 20 à 25 nucléotides, comprise dans la séquence SEQ ID N°52 suivante (positions 1120 à 1150 d'après Desjardins et Morais, 1990, J. Mol. Biol., 212, 599-634) :
   DTW ATG ATT HTY RTT ADA TTA CGC T
   dans laquelle D est A, G ou T, W est A ou T, H est A, C ou T, Y est C ou T, R est A ou G,
   - ou comprenant la séquence SEQ ID N°53 suivante (positions 1130 à 1150 d'après Desjardins et Morais, 1990, J. Mol. Biol., 212, 599-634) :
      TCG TTA GAT TAC GCT
   - ou constitués par la séquence SEQ ID N°17 suivante (positions 1125 à 1150 d'après Desjardins et Morais, 1990, J. Mol. Biol., 212, 599-634) :
      GAT TGT CGT TAG ATT ACG CT
   ou ceux,
(12) - présentant une identité de séquence d'au moins 80%, préférentiellement 90% et avantageusement 95% avec un oligonucléotide constitué d'une séquence de 15 à 25 nucléotides, notamment de 20 à 25 nucléotides, comprise dans la séquence SEQ ID N°54 suivante (positions 249 à 272 d'après Desjardins et Morais, 1990, J. Mol. Biol., 212, 599-634) :
   AAY GHA TGA AYG HYC AGN DAC CAT
   dans laquelle Y est C ou T, H est A, C ou T, N est A, C, G ou T, D est A, G ou T,
   - ou comprenant la séquence SEQ ID N°55 suivante (positions 256 à 272 d'après Desjardins et Morais, 1990, J. Mol. Biol., 212, 599-634) :
      ATG TTC TAG GAC CAT
   - ou constitués par la séquence SEQ ID N°18 suivante (positions 252 à 272 d'après Desjardins et Morais, 1990, J. Mol. Biol., 212, 599-634) :
      ATG AAT GTT CTA GGA CCA T
   ou ceux,
(13) - présentant une identité de séquence d'au moins 80%, préférentiellement 90% et avantageusement 95% avec un oligonucléotide constitué d'une séquence de 15 à 25 nucléotides, notamment de 20 à 25 nucléotides, comprise dans la séquence SEQ ID N°56 suivante (positions 409 à 433 d'après Desjardins et Morais, 1990, J. Mol. Biol., 212, 599-634) :
   CGG GTT CTG ATT TCA CGT GAG GAG T
   - ou comprenant la séquence SEQ ID N°57 suivante (positions 419 à 433 d'après Desjardins et Morais, 1990, J. Mol. Biol., 212, 599-634) :
      TTT CAC GTG AGG AGT
   - ou constitués par la séquence SEQ ID N°19 suivante (positions 414 à 433 d'après Desjardins et Morais, 1990, J. Mol. Biol., 212, 599-634) :
      TCT GAT TTC ACG TGA GGA GT.

Les oligonucléotides ou amorces tels que définis ci-dessus permettent de détecter et d'identifier des fragments d'ADN provenant d'oiseaux appartenant à l'ordre des ansériformes, et en particulier à la famille des anatidés, ou à l'ordre des galliformes et en particulier à la famille des gallinacés.

Sont également décrits les couples d'amorces constitués :
- par l'un quelconque des oligonucléotides SEQ ID N°1, SEQ ID N°32, SEQ ID N°33, SEQ ID N°2, SEQ ID N°34, SEQ ID N°35, SEQ ID N°3, SEQ ID N°36, SEQ ID N°37 et l'un quelconque des oligonucléotides SEQ ID N°4, SEQ ID N°38, SEQ ID N°39, SEQ ID N°5, SEQ ID N°40, SEQ ID N°41 tels que définis ci-dessus,
- par l'un quelconque des oligonucléotides SEQ ID N°11, SEQ ID N°42, SEQ ID N°43 et l'un quelconque des oligonucléotides SEQ ID N°12, SEQ ID N°44, SEQ ID N°45 tels que définis ci-dessus,
- par l'un quelconque des oligonucléotides SEQ ID N°14, SEQ ID N°46, SEQ ID N°47, et l'un quelconque des oligonucléotides SEQ ID N°15, SEQ ID N°48, SEQ ID N°49 tels que définis ci-dessus,
- par l'un quelconque des oligonucléotides SEQ ID N°16, SEQ ID N°50, SEQ ID N°51, et l'un quelconque des oligonucléotides SEQ ID N°17, SEQ ID N°52, SEQ ID N°53 tels que définis ci-dessus,
- par l'un quelconque des oligonucléotides SEQ ID N°18, SEQ ID N°54, SEQ ID N°55, et l'un quelconque des oligonucléotides SEQ ID N°19, SEQ ID N°56, SEQ ID N°57 tels que définis ci-dessus,
et avantageusement constitués par le couple d'oligonucléotides choisis parmi les couples suivants :
- SEQ ID N° 1 et SEQ ID N° 5,
- SEQ ID N° 2 et SEQ ID N° 5,
- SEQ ID N° 1 et SEQ ID N° 4,
- SEQ ID N° 2 et SEQ ID N° 4,
- SEQ ID N° 3 et SEQ ID N° 5,
- SEQ ID N° 11 et SEQ ID N° 12,
- SEQ ID N° 14 et SEQ ID N° 15,
- SEQ ID N° 16 et SEQ ID N° 17,
- SEQ ID N° 18 et SEQ ID N° 19.

Les couples d'amorces tels que défmis ci-dessus permettent d'obtenir, par réaction PCR, un fragment d'ADN provenant d'oiseaux appartenant à l'ordre des ansériformes, et en particulier à la famille des anatidés, présentant une taille et une séquence déterminées, spécifique des anatidés. En outre, le couple d'amorces (SEQ ID N°11, SEQ ID N° 12) permet d'obtenir par réaction PCR, un fragment d'ADN provenant d'oiseaux appartenant à l'ordre des galliformes, et en particulier à la famille des gallinacés, présentant une taille et une séquence déterminées, spécifique des gallinacés. Sont également décrits les oligonucléotides choisis parmi ceux :
(1) - présentant une identité de séquence d'au moins 80%, préférentiellement 90% et avantageusement 95% avec un oligonucléotide constitué d'une séquence de 15 à 25 nucléotides, notamment de 20 à 25 nucléotides, comprise dans la séquence SEQ ID N°58 suivante (positions 145 à 170 d'après Desjardins et Morais, 1990, J. Mol. Biol., 212, 599-634) :
   ATA TGK MVN CGG CAT TAA CCT ATA T
   dans laquelle K est G ou T, M est A ou C, V est A, C ou G, N est A, C, G ou T,
   - ou comprenant la séquence SEQ ID N°59 suivante (positions 155 à 170 d'après Desjardins et Morais, 1990, J. Mol. Biol., 212, 599-634) :
      GGC ATT AAC CTA TAT
   - ou constitués par la séquence SEQ ID N°23 suivante (positions 151 à 170 d'après Desjardins et Morais, 1990, J. Mol. Biol., 212, 599-634) :
      ATA CGG GCA TTA ACC TAT AT
   ou ceux,
(2) - présentant une identité de séquence d'au moins 80%, préférentiellement 90% et avantageusement 95% avec un oligonucléotide constitué d'une séquence de 15 à 25 nucléotides, notamment de 20 à 25 nucléotides, comprise dans la séquence SEQ ID N°60 suivante (positions 356 à 380 d'après Desjardins et Morais, 1990, J. Mol. Biol., 212, 599-634):
   GCG AGY ATA ACC AAA TSG GTT ACA T
   dans laquelle Y est C ou T, S est C ou G,
   - ou comprenant la séquence SEQ ID N°61 suivante (positions 366 à 380 d'après Desjardins et Morais, 1990, J. Mol. Biol., 212, 599-634) :
      CCA AAT GGG TTA CAT
   - ou constitués par la séquence SEQ ID N°24 suivante (positions 361 à 380 d'après Desjardins et Morais, 1990, J. Mol. Biol., 212, 599-634) :
      CAT AAC CAA ATG GGT TAC AT
   ou ceux,
(3) - présentant une identité de séquence d'au moins 80%, préférentiellement 90% et avantageusement 95% avec un oligonucléotide constitué d'une séquence de 15 à 25 nucléotides, notamment de 20 à 25 nucléotides, comprise dans la séquence SEQ ID N°62 suivante (positions 1014 à 1037 d'après Desjardins et Morais, 1990, J. Mol. Biol., 212, 599-634) :
   TAC ATA CAW ACY MWC CGC ACA AAT A
   dans laquelle W est A ou T, Y est C ou T, M est A ou C,
   - ou comprenant la séquence SEQ ID N°63 suivante (positions 1024 à 1037 d'après Desjardins et Morais, 1990, J. Mol. Biol., 212, 599-634) :
      CTC ACC GCA CAA ATA
   - ou constitués par la séquence SEQ ID N°25 suivante (positions 1019 à 1037 d'après Desjardins et Morais, 1990, J. Mol. Biol., 212, 599-634) :
      ACA AAC TCA CCG CAC AAA TA
   ou ceux,
(4) - présentant une identité de séquence d'au moins 80%, préférentiellement 90% et avantageusement 95% avec un oligonucléotide constitué d'une séquence de 15 à 25 nucléotides, notamment de 20 à 25 nucléotides, comprise dans la séquence SEQ ID N°64 suivante (positions 1167 à 1192 d'après Desjardins et Morais, 1990, J. Mol. Biol., 212, 599-634) :
   TGT AAR TAW CAA TAT AAA TAA TAT A
   dans laquelle R est A ou G, W est A ou T,
   - ou comprenant la séquence SEQ ID N°65 suivante (positions 1177 à 1192 d'après
      AAT ATA AAT AAT ATA
   - ou constitués par la séquence SEQ ID N°26 suivante (positions 1172 à 1191 d'après Desjardins et Morais, 1990, J. Mol. Biol., 212, 599-634) :
      TTA ACA ATA TAA ATA ATA TA
   ou ceux,
(5) - présentant une identité de séquence d'au moins 80 %, préférentiellement 90 % et avantageusement 95 %, avec un oligonucléotide constitué d'une séquence de 15 à 25 nucléotides, notamment de 20 à 25 nucléotides, comprise dans la séquence SEQ ID N° 70 suivante (position 684 à 709 d'après Desjardins et Morais, 1990, J. Mol. Biol., 212, 599-634) :
   CCT TCM CAG TGM BKK YGS CRG RGT V
   dans laquelle M est A ou C, B est C, G ou T, K est G ou T, Y est C ou T, S est C ou G, R est A ou G, V est A, C ou G,
   - ou comprenant la séquence SED ID N°71 suivante (positions 694 à 709 d'après Desjardins et Morais, 1990, J. Mol. Biol., 212, 599-634) :
      GCC GTC GCC AGA GTA
   - ou constitués par la séquence SED ID N°72 suivante (positions 689 à 709 d'après Desjardins et Morais, 1990, J. Mol. Biol., 212, 599-634) :
      ACA GTG CCG TCG CCA GAG TA
   ou ceux,
(6) - présentant une identité de séquence d'au moins 80 %, préférentiellement 90 % et avantageusement 95 %, avec un oligonucléotide constitué d'une séquence de 15 à 25 nucléotides, notamment de 20 à 25 nucléotides, comprise dans la séquence SEQ ID N° 73 suivante (position 898 à 923 d'après Desjardins et Morais, 1990, J. Mol. Biol., 212, 599-634) :
   RRW WRT RGW KGR RGK GRR WWT TNR Y
   dans laquelle R est A ou G, W est A ou T, K est G ou T, N est A, C, G ou T, Y est C ou T,
   - ou comprenant la séquence SED ID N°74 suivante (positions 898 à 913 d'après Desjardins et Morais, 1990, J. Mol. Biol., 212, 599-634)
      GAA GTG AAA ATT AGC
   - ou constitués par la séquence SED ID N°75 suivante (positions 898 à 918 d'après Desjardins et Morais, 1990, J. Mol. Biol., 212, 599-634)
      TAG AGG AAG TGA AAA TTA GC
   ou ceux,
(7) - présentant une identité de séquence d'au moins 80%, préférentiellement 90% et avantageusement 95% avec un oligonucléotide constitué d'une séquence de 15 à 25 nucléotides, notamment de 20 à 25 nucléotides, comprise dans la séquence SEQ ID N°66 suivante (positions 879 à 898 d'après Desjardins et Morais, 1990, J. Mol. Biol., 212, 599-634) :
   TGA ATG CTT GYY GGA CAT WAA TAC C
   dans laquelle Y est C ou T, W est A ou T,
   - ou comprenant la séquence SEQ ID N°67 suivante (positions 888 à 898 d'après Desjardins et Morais, 1990, J. Mol. Biol., 212, 599-634) :
      CTG GAC ATA AAT ACC
   - ou constitués par la séquence SEQ ID N°27 suivante (positions 884 à 898 d'après Desjardins et Morais, 1990, J. Mol. Biol., 212, 599-634) :
      GCT TGC TGG ACA TAA ATA CC
   ou ceux,
(8) - présentant une identité de séquence d'au moins 80%, préférentiellement 90% et avantageusement 95% avec un oligonucléotide constitué d'une séquence de 15 à 25 nucléotides, notamment de 20 à 25 nucléotides, comprise dans la séquence SEQ ID N°68 suivante (positions 1077 à 1101 d'après Desjardins et Morais, 1990, J. Mol. Biol., 212, 599-634) :
   CTA TAA TAK GRT GTG GGG CGT GTT G
   dans laquelle K est G ou T, R est A ou G
   - ou comprenant la séquence SEQ ID N°69 suivante (positions 1087 à 1101 d'après Desjardins et Morais, 1990, J. Mol. Biol., 212, 599-634) :
      TTG TGG GGC GTG TTG
   - ou constitués par la séquence SEQ ID N°28 suivante (positions 1082 à 1101 d'après Desjardins et Morais, 1990, J. Mol. Biol., 212, 599-634) :
      ATA AGT TGT GGG GCG TGT TG

Les oligonucléotides ou amorces tels que définis ci-dessus permettent de détecter et d'identifier des fragments d'ADN provenant d'oiseaux appartenant à l'ordre des galliformes, et en particulier à la famille des gallinacés, ou à l'ordre des struthioniformes, et en particulier à la famille des struthionidés.

Sont également décrits les couples d'amorces constitués :
- par l'un quelconque des oligonucléotides SEQ ID N°23, SEQ ID N°58, SEQ ID N°59, et l'un quelconque des oligonucléotides SEQ ID N°24, SEQ ID N°60, SEQ ID N°61 tels que définis ci-dessus ou,
- par l'un quelconque des oligonucléotides SEQ ID N°25, SEQ ID N°62, SEQ ID N°63, et l'un quelconque des oligonucléotides SEQ ID N°26, SEQ ID N°64, SEQ ID N°65 tels que définis ci-dessus ou,
- par l'un quelconque des oligonucléotides SEQ ID N°70, SEQ ID N°71, SEQ ID N°72, et l'un quelconque des oligonucléotides SEQ ID N°73, SEQ ID N°74, SEQ ID N°75 tels que définis ci-dessus ou,
- par l'un quelconque des oligonucléotides SEQ ID N°27, SEQ ID N°66, SEQ ID N°67, et l'un quelconque des oligonucléotides SEQ ID N°28, SEQ ID N°68, SEQ ID N°69 tels que définis ci-dessus ou,
et avantageusement constitués par le couple d'oligonucléotides choisis parmi les couples suivants :
- SEQ ID N°23 et SEQ ID N°24,
- SEQ ID N°25 et SEQ ID N°26,
- SEQ ID N°72 et SEQ ID N°75,
- SEQ ID N°27 et SEQ ID N°28.

Les couples d'amorces (SEQ ID N°23, SEQ ID N°24), (SEQ ID N°25, SEQ ID N°26) et (SEQ ID N°72, SEQ ID N°75), tels que définis ci-dessus permettent d'obtenir, par réaction PCR, un fragment d'ADN provenant d'oiseaux appartenant à l'ordre des galliformes, et en particulier à la famille des gallinacés, présentant une taille et une séquence déterminées, spécifique des gallinacés.

Le couple d'amorces (SEQ ID N°27, SEQ ID N°28) tel que défini ci-dessus permet d'obtenir, par réaction PCR, un fragment d'ADN provenant d'oiseaux appartenant à l'ordre des struthioniformes, et en particulier à la famille des struthionidés, présentant une taille et une séquence déterminées, spécifique des struthionidés.

Sont encore décrits les fragments d'ADN susceptibles d'être obtenus selon le procédé tel que défini ci-dessus, comprenant environ 100 à environ 500 paires de bases.

Le fragment d'ADN décrit présente avantageusement une identité de séquence d'au moins 80%, préférentiellement 90% et avantageusement 95% avec :
- un oligonucléotide constitué d'une séquence d'environ 15 à 25 nucléotides, comprise dans la SEQ ID N°6 suivante : 
ACYAGCTTCA GGCCCATACG TTCCCCCTAA ACCCCTCGCC CTCCTCACAT TTTTGCGCCT
CTGGTTCCTC GGTCAGGGCC ATCMATTGGG TTCACTCACC YCYMYTYGCC YTTCAAAGTG
GCATCTGTGG ANKACBTYCA CCWYYYCRRT GCGTWATCGC GGCATBYTYM ASYWTTTWSM
CGCCTYTGGT TCYMYTTHTY TYT
 dans laquelle Y est C ou T, M est A ou C, K est G ou T, B est C, G ou T, R est A ou G, W est A ou T, S est C ou G, H est A, C ou T, N est A, C, G ou T,
   et notamment fragment de 203 paires de bases comprenant ou constitué par la SEQ ID N°6 définie ci-dessus,
- ou un oligonucléotide constitué d'une séquence d'environ 15 à 25 nucléotides, comprise dans la SEQ ID N°7 suivante : 
ACYAGCTTCA GGCCCATACG TTCCCCCTAA ACCCCTCGCC CTCCTCACAT TTTTGCGCCT
CTGGTTCCTC GGTCAGGGCC ATCMATTGGG TTCACTCACC YCYMYTYGCC YTTCAAAGTG
GCATCTGTGG ANKACBT
dans laquelle Y est C ou T, M est A ou C, N est A, C, G ou T, K est G ou T, B est C, G ou T,
et notamment fragment de 137 paires de bases comprenant ou constitué par la SEQ ID N° 7 définie ci-dessus,
- ou un oligonucléotide constitué d'une séquence d'environ 15 à 25 nucléotides, comprise dans la SEQ ID N°8 suivante : 
AACCCCTCGC CCTCCTCACA TTTTTGCGCC TCTGGTTCCT CGGTCAGGGC CATCMATTGG
GTTCACTCAC CYCYMYTYGC CYTTCAAAGT GGCATCTGTG GANKACBTYC ACCWYYYCRR
TGCGTWATCG CGGCATBYTY MASYWTTTWS MCGCCTYTGG TTCYMYTTHT YTYT
 dans laquelle Y est C ou T, M est A ou C, N est A, C, G ou T, K est G ou T, B est C, G ou T, R est A ou G, W est A ou T, S est C ou G, H est A, C ou T,
   et notamment fragment de 174 paires de bases comprenant ou constitué par la SEQ ID N° 8 définie ci-dessus,
- ou un oligonucléotide constitué d'une séquence d'environ 15 à 25 nucléotides, comprise dans la SEQ ID N°9 suivante : 
AACCCCTCGC CCTCCTCACA TTTTTGCGCC TCTGGTTCCT CGGTCAGGGC CATCMATTGG
GTTCACTCAC CYCYMYTYGC CYTTCAAAGT GGCATCTGTG GANKACBT
 dans laquelle Y est C ou T, M est A ou C, N est A, C, G ou T, K est G ou T, B est C, G ou T,
   et notamment fragment de 108 paires de bases comprenant ou constitué par la SEQ ID N° 9 défmie ci-dessus,
- ou un oligonucléotide constitué d'une séquence d'environ 15 à 25 nucléotides, comprise dans la SEQ ID N° 10 suivante : 
ATCMATTGGG TTCACTCACC YCYMYTYGCC YTTCAAAGTG GCATCTGTGG ANKACBTYCA
CCWYYYCRRT GCGTWATCGC GGCATBYTYM ASYWTTTWSM CGCCTYTGGT TCYMYTTHTY
TYT
 dans laquelle Y est C ou T, M est A ou C, N est A, C, G ou T, K est G ou T, B est C, G ou T, R est A ou G, W est A ou T, S est C ou G, H est A, C ou T,
   et notamment fragment de 123 paires de bases comprenant ou constitué par la SEQ ID N° 10 définie ci-dessus,
- ou un oligonucléotide constitué d'une séquence d'environ 15 à 25 nucléotides, comprise dans la SEQ ID N° 13 suivante : 
YMYYKRYACT GATGCACTTK GDYCDCATTY RGTTRWKGYY MYNTCCRCHV HYCYABNBWH
WVRTGYTRYY NRGTGAATGC TYGHHGGACA TADYWYWAWM MAWHWWMHYW YYHWSYRYWW
YYYHYRSRMH HHMNRAAYDM AACCARHRDW YTTYMDCHRW THWDHWWHMM WHYWYVTWDY
RTMWWHYHHV HMHHHHMHWM HHBHAWWHHH HNHNHYNHWA YYBCHNNYHH HWAWYNYSYY
WHHYNWBVHW HHNBYBDMDM DWCHDWMMNR WWHDMAYDDH DYVDHHYAWM HTTATTAKAG
RAACWCCAGY ACYARVVVHD VWHNHAMHYN RRHRWHHMYY AYWAHYYHVH YNTYNMNAMH
HNBNVYHYRD HYHRYYARYB RMHYVHHHYR YYCDCMTWSY WYMMHNHH
 dans laquelle Y est C ou T, M est A ou C, N est A, C, G ou T, K est G ou T, B est C, G ou T, R est A ou G, W est A ou T, S est C ou G, D est A, G ou T, V est A, C ou G, H est A, C ou T,
   et notamment fragment de 408 paires de bases comprenant ou constitué par la SEQ ID N° 13 définie ci-dessus ou,
   fragments résultants d'une ou plusieurs délétions de nucléotides contigus ou non contigus dudit fragment de 408 paires de bases comprenant ou constitué par SEQ ID N°13, et susceptible d'être obtenu par amplification d'ADNmt provenant d'oiseaux, à l'aide des oligonucléotides SEQ ID N°11 et SEQ ID N°12 tels que définis précédemment, et notamment fragments de 331 paires de bases, de 328 paires de bases ou 288 paires de bases,
- ou un oligonucléotide constitué d'une séquence d'environ 15 à 25 nucléotides, comprise dans la SEQ ID N°20 suivante : 
HWBDNHMRMG RNCATHHHHN NYHVHBDNAH NNNHCHHCCA VVRMHHHNNV RTGMAYGHYC
YHRNDVHHHH NHVNHVVMHV VMHVNVHNHN NHHHNHVHVV RWNMACMARR RCMMHDHNNR
VTGAATGCTH NMHRRACAWR DMNYNDVHAH HMHTCYDDNB YYBHHTCCAC CCRMMCATWH
 dans laquelle Y est C ou T, M est A ou C, N est A, C, G ou T, B est C, G ou T, R est A ou G, W est A ou T, D est A, G ou T, V est A, C ou G, H est A, C ou T,
   et notamment fragment de 180 paires de bases comprenant ou constitué par la SEQ ID N° 20 définie ci-dessus,
- ou un oligonucléotide constitué d'une séquence d'environ 15 à 25 nucléotides, comprise dans la SEQ ID N°21 suivante : 
WKGYYMYNTC CRCHVHYCYA BNBWHWVRTG YTRYYNRGTG AATGCTYGHH GGACATAMHH
HMNRAAYDMA ACHRHYHHVH MHHHNHNHNH HNNYHHHWAY WBVHWHWCHD WMMNRWWHDM
AYDDHDYVDH HYAWMHTTAT TAKAGRAACW CCAGYACYAR VVVHDVWHNH AMHYNRRHRW
HHMYYAYWAH Y
 dans laquelle Y est C ou T, M est A ou C, N est A, C, G ou T, B est C, G ou T, R est A ou G, W est A ou T, D est A, G ou T, V est A, C ou G, H est A, C ou T, K est G ou T,
   et notamment fragment de 191 paires de bases comprenant ou constitué par la SEQ ID N° 21 définie ci-dessus,
- ou un oligonucléotide constitué d'une séquence d'environ 15 à 25 nucléotides, comprise dans la SEQ ID N°22 suivante : 
RTGAAYGHYC YHRNDVHHNH MVHNHNVMHV VNHNHHNHNH VVVSWNMARD SCMNHNNTTM
VTGAATGCTH NMHRRACAWD MNYNDVHAHH MHTCYNDNBH YBHHTCCACC CRMMCATWHM
TYATGRAVYY NCGTAYCARR TGGATTTATT RRYCGTACWC CTCACGTGAA ATCAGC
 dans laquelle Y est C ou T, M est A ou C, N est A, C, G ou T, B est C, G ou T, R est A ou G, W est A ou T, D est A, G ou T, V est A, C ou G, H est A, C ou T, S est C ou G,
   et notamment fragment de 176 paires de bases comprenant ou constitué par la SEQ ID N° 22 définie ci-dessus.

Les fragments d'ADN tels que définis ci-dessus sont spécifiques d'oiseaux, notamment d'ansériformes ou des galliformes, et plus particulièrement d'anatidés et de gallinacés.

A titre d'exemple, la séquence nucléotidique du fragment SEQ ID N° 6 telle que définie ci-dessous, est spécifique du canard mandarin (*Aix galericulata*) et est obtenue par amplification à l'aide des amorces SEQ ID N°1 et SEQ ID N°5 :

ACTAGCTTCA GGCCCATACG TTCCCCCTAA ACCCCTCGCC CTCCTCACAT TTTTGCGCCT
CTGGTTCCTC GGTCAGGGCC ATCAATTGGG TTCACTCACC TCCTCCTTGC CTTTCAAAGT
GGCATCTGTG GAAGACCTCC ACCATCTCAA TGCGTAATCG CGGCATCCTC CAGCTTTTTG
GCGCCTCTGG TTCCTCTTAT TTT

Est également décrit le fragment d'ADN présentant avantageusement une identité de séquence d'au moins 80%, préférentiellement 90% et avantageusement 95% avec :
- un oligonucléotide constitué d'une séquence d'environ 15 à 25 nucléotides, comprise dans la SEQ ID N°29 suivante : 
KMAWVNGGHM WTVACHYATA TTCCMCWTYY CKYMCHAHVB MCHYTVDATG MAYSMTSHHR
KRCMHVCTMH NTTHCVYDHM CCATAGABAG TTCCWAACHA CWATCRRKVM MCMTAACTAT
GAATGGTTRC VGHACATAAA TCTCACTCTC ATGTTCTCCC CCCAACAAGT CACCTAACTA
TGAATGGTTA CAGGACATAC ATTTDACTAY CATGTTCTAA CCSATTTGGT T
 dans laquelle Y est C ou T, M est A ou C, N est A, C, G ou T, B est C, G ou T, R est A ou G, W est A ou T, D est A, G ou T, V est A, C ou G, H est A, C ou T, S est C ou G, K est G ou T
   et notamment fragment de 231 paires de bases comprenant ou constitué par la SEQ ID N°29 définie ci-dessus,
- ou un oligonucléotide constitué d'une séquence d'environ 15 à 25 nucléotides, comprise dans la SEQ ID N°30 suivante : 
TACAWACYMW CGYAWWRRWD CCCTYAMAYY AYAHAAMCGY YTATCGYWTA DTATATATAC
ATTRYWRYDY AYYMYAYMAT TATTAKAGAA ACYCCMCTAC CRAAACMAWC AWHARARRCW
WAHADHWAHA TKCMRCDYAW VBYHCYTCAS AWRCMWWBRT KRTWTATATT GTTAA
 dans laquelle Y est C ou T, M est A ou C, B est C, G ou T, R est A ou G, W est A ou T, D est A, G ou T, V est A, C ou G, H est A, C ou T, S est C ou G, K est G ou T,
   et notamment fragment de 175 paires de bases comprenant ou constitué par la SEQ ID N°30 définie ci-dessus,
- ou un oligonucléotides constitué d'une séquence d'environ 15 à 25 nucléotides, comprise dans la SEQ ID N°76 suivante : 
MCAGTGMSKK YGSCRGRGTV CYAYTCAAKY KRAGCCTGGA YTACWCCTGC GTTRCGYSCT
ATYCTAKWYY TCHVGKRTYM CTCRATGAKA CGGTTKGCGT RTATKKGGWA TCAYYTTGAC
ACTGATGCAC TTTGGAYCRC ATTYRGTTAW KGYTCTYCCR CMSYYCYNKW WARTRGKGYT
ATWTAGTGAA TGCTYGHYGG ACATAYYTTA WSYNAAWWYY CMCYYCMWCY A
 dans laquelle M est A ou C, S est C ou G, K est G ou T, Y est C ou T, R est A ou G, V est A, C ou G, W est A ou T, N est A, C, T ou G,
   et notamment fragment de 231 paires de bases comprenant ou constitué par la SEQ ID N°76 définie ci-dessus,
- ou un oligonucléotide constitué d'une séquence d'environ 15 à 25 nucléotides, comprise dans la SEQ ID N°31 suivante : 
TGAATGCTTG YYGGACATWW TWYMAYYWAT TWTCACTTCC WCTGAYTTTY YTMACAMMAC
YAGGAYWMAW YTYYTWYDTY DKTWYWAAYW YWTKWTTTVW TYAYWTYAAW MACATYTTTT
AMYTAWAYTA MCYWTACAWA CYMWCMGYAY WRRWTDTYAM AYYAAHAAMC GYYCGYWTAD
TATATATACA TARYWRYTGY RYYTMYAYCM ATTATT
 dans laquelle Y est C ou T, M est A ou C, R est A ou G, W est A ou T, D est A, G ou T, V est A, C ou G, H est A, C ou T,
   et notamment fragment de 216 paires de bases comprenant ou constitué par la SEQ ID N°31 définie ci-dessus.

Les fragments d'ADN SEQ ID N°29, SEQ ID N°30 et SEQ ID N°76 tels que définis ci-dessus sont spécifiques d'oiseaux appartenant à l'ordre des galliformes, et plus particulièrement à la famille des gallinacés ; le fragment d'ADN SEQ ID N°31 tel que défini ci-dessus est spécifique d'oiseaux appartenant à l'ordre des struthioniformes, et plus particulièrement à la famille des struthionidés.

Selon un mode de réalisation avantageux du procédé décrit, le fragment d'ADN amplifié obtenu est identifié :
- par séquençage dudit fragment amplifié,
- par électrophorèse sur gel des fragments obtenus après digestion à l'aide d'enzymes de restriction dudit fragment,
- directement, par simple visualisation de la présence dudit fragment amplifié par électrophorèse sur gel.

Selon un mode de réalisation avantageux, la méthode d'identification par séquençage du fragment amplifié obtenu permet d'identifier les espèces d'oiseaux appartenant à l'ordre des ansériformes, et plus particulièrement à la famille des anatidés.

La méthode d'identification par électrophorèse sur gel des fragments obtenus après digestion à l'aide d'enzymes de restriction permet d'identifier les genres d'oiseaux appartenant à l'ordre des ansériformes, et plus particulièrement à la famille des anatidés, et/ou à l'ordre des galliformes, et plus particulièrement à la famille des gallinacés.

La méthode d'identification directe, par simple visualisation de la présence de la séquence amplifiée obtenue, permet d'identifier les espèces d'oiseaux appartenant à l'ordre des ansériformes, et plus particulièrement à la famille des anatidés, à l'ordre des galliformes, et plus particulièrement à la famille des gallinacés, et/ou à l'ordre des struthioniformes, et plus particulièrement à la famille des struthionidés.

Chacune de ces trois méthodes d'identification est décrite plus en détails ci-dessous.

### 1) La méthode d'identification par séquençage de la séquence ou du fragment d'ADN amplifié(e) est dénommée dans ce qui suit " stratégie globale ", car elle permet de détecter et d'identifier la présence ou l'absence de tous les anatidés existants. La liste de tous les anatidés existants pouvant être détectés et identifiés par le procédé de l'invention est donnée dans le tableau 1 ci-après.

Les amorces utilisées afin d'effectuer l'amplification par la méthode PCR d'une séquence d'ADN provenant d'anatidés, éventuellement présente dans un échantillon de matière organique à analyser, sont les 15 amorces listées ci-dessous et telles que définies ci-dessus :
SEQ ID N°32, SEQ ID N°33, SEQ ID N°1, SEQ ID N°34, SEQ ID N°35, SEQ ID N°2, SEQ ID N°36, SEQ ID N°37, SEQ ID N°3, SEQ ID N° 38, SEQ ID N°39, SEQ ID N°4, SEQ ID N°40, SEQ ID N°41, SEQ ID N°5,
et plus particulièrement les 5 amorces SEQ ID N°1, SEQ ID N°2, SEQ ID N°3, SEQ ID N°4 et SEQ ID N°5.

Chacun des oligonucléotides SEQ ID N°1 à SEQ ID N°3 peut être utilisé en couple avec l'un quelconque des oligonucléotides SEQ ID N°4 et SEQ ID N°5.

Selon un mode de mise en oeuvre particulièrement avantageux, une partie des étapes d'hybridation des cycles constituant la réaction d'amplification est effectuée à une température d'environ 50°C à environ 58 °C. En outre, le Demandeur a trouvé qu'une température de 55°C était particulièrement appropriée pour obtenir une amplification spécifique. Un tel mode de mise en oeuvre permet d'obtenir une plus grande spécificité d'amplification.

On notera à ce sujet que le Demandeur a résolu un certain nombre de problèmes techniques pour la mise en oeuvre de ce procédé. Tout d'abord, le choix des amorces constituait un réel problème, car il fallait sélectionner d'une part des séquences communes aux différentes espèces d'anatidés mais pas à d'autres espèces d'oiseaux, et d'autre part s'hybridant de manière stable, dans des conditions physico-chimiques très variables, représentatives de la grande variabilité des matières organiques susceptibles de contenir des matières biologiques provenant d'anatidés. Les températures des étapes de séparation des brins et d'élongation sont avantageusement respectivement d'environ 94°C et d'environ 72°C.

Le procédé décrit ci-dessus est spécifique aux anatidés mais global entre toutes les espèces d'anatidés car il ne donne aucune réaction d'amplification détectable en présence d'ADN d'origine autre que les anatidés.

Selon un mode de réalisation avantageux du procédé décrit, l'utilisation des couples d'amorces oligonucléotidiques suivants :
- (SEQ ID N°1, SEQ ID N°5),
- (SEQ ID N°2, SEQ ID N°5),
- (SEQ ID N°1, SEQ ID N°4),
- (SEQ ID N°2, SEQ ID N°4) et,
- (SEQ ID N°3, SEQ ID N°5),
permettent respectivement d'obtenir :
- un fragment d'ADN SEQ ID N°6 de 203 paires de bases tel que défini ci-dessus,
- un fragment d'ADN SEQ ID N°8 de 174 paires de bases tel que défini ci-dessus,
- un fragment d'ADN SEQ ID N°7 de 137 paires de bases tel que défini ci-dessus,
- un fragment d'ADN SEQ ID N°9 de 108 paires de bases tel que défini ci-dessus et,
- un fragment d'ADN SEQ ID N°10 de 123 paires de bases tel que défini ci-dessus.

Les produits d'amplification décrits ci-dessus peuvent être détectés même lorsqu'une fraction importante de l'ADN est dégradée, à savoir après action de facteurs physiques, chimiques et/ou biochimiques, et lors de transformations diverses des échantillons de matières organiques. L'expérimentateur recherche préférentiellement la présence de la séquence SEQ ID n°6 décrite ci-dessus à l'aide des amorces appropriées, et dans le cas où la détection serait négative, il recherche les fragments de taille inférieure et notamment ceux d'environ 174, 137, 123 et 108 paires de bases.

L'unicité des produits d'amplification représente un autre avantage, celle-ci permettant d'obtenir une sensibilité importante et facilitant grandement l'interprétation des résultats. Le procédé décrit présente un grand nombre d'avantages par rapport aux techniques d'identification déjà connues.

Ainsi, le procédé décrit présente une grande simplicité d'interprétation, en raison de la production d'un seul et unique produit, spécifique de l'ADN d'anatidés et qui donc ne se retrouve pas dans les produits d'amplifications d'ADN d'autres espèces.

Le produit d'amplification peut être mis en évidence par toute méthode connue de l'homme de métier, et en particulier par simple électrophorèse sur un gel d'agarose. La lecture des profils de migration des produits d'amplification obtenus avec le procédé de l'invention consiste donc simplement à déterminer la présence d'une seule et unique bande de migration dans un gel d'électrophorèse. En l'absence d'une telle bande, on peut considérer qu'il n'y a pas de traces détectables d'ADN d'anatidés. La présence d'une bande signifie au contraire que l'ADN d'anatidés est présent dans l'échantillon, et donc que l'échantillon en question contient de la matière biologique à base d'anatidés.

Le produit d'amplification obtenu peut ensuite être séquencé afin de déterminer sa séquence nucléotidique. La comparaison de cette séquence avec toutes les séquences nucléotidiques connues des anatidés permet de déterminer l'espèce d'anatidés présente dans l'échantillon de matière organique, et ainsi de différencier les espèces les unes par rapport aux autres.

Le produit d'amplification obtenu, identifié à l'aide de la stratégie globale, est généralement situé dans la partie centrale de la région de contrôle de la réplication de l'ADN mitochondrial, délimitée par les nucléotides situés aux environs des positions 440 et 750, notamment aux environs des positions 450 et 700, et de préférence aux environs des positions 459 et 665 de la région de contrôle de la réplication de l'ADN mitochondrial, lesdites positions étant définies d'après Desjardins et Morais, 1990, J. Mol. Biol., 599-634.

### 2) La méthode d'identification par électrophorèse sur gel des fragments obtenus à l'aide d'enzymes de restriction dudit fragment amplifié, est appelée " stratégie intermédiaire ", car elle permet de détecter et d'identifier les genres d'anatidés et de gallinacés les plus communs.

La stratégie intermédiaire permet d'obtenir un résultat plus rapidement que la " stratégie globale ", car la phase de séquençage est évitée. Cette méthode d'identification consiste à mettre un produit d'amplification obtenu par PCR en présence d'enzymes de restriction. Les oligonucléotides sont choisis de façon à avoir un fragment d'amplification assez variable afin de pouvoir distinguer suffisamment de genres d'anatidés et de gallinacés après coupure enzymatique.

Les amorces utilisées afin d'effectuer l'amplification par la méthode PCR d'une séquence d'ADN provenant d'anatidés ou de gallinacés, éventuellement présente dans un échantillon de matière organique à analyser, sont les 6 amorces listées ci-après et telles que définies ci-dessus : SEQ ID N°42, SEQ ID N°43, SEQ ID N°11, SEQ ID N°44, SEQ ID N°45 et SEQ ID N°12, et plus particulièrement les 2 amorces SEQ ID N°11 et SEQ ID N°12.

Selon un mode de réalisation avantageux du procédé décrit, l'utilisation du couple d'oligonucléotides SEQ ID N°11 et SEQ ID N°12 permet d'obtenir un fragment d'amplification SEQ ID N°13 d'une taille variable, en fonction du genre d'anatidés ou de gallinacés présent dans l'échantillon de matière organique à analyser.

Ainsi, un fragment d'amplification SEQ ID N°13 d'une taille de 408 pb, obtenu à l'aide du couple (SEQ ID N°11, SEQ ID N°12), est caractéristique de l'oie du genre *Anser,* et notamment de l'espèce *Anser anser* (oie commune). La séquence nucléotidique de ce fragment a été définie ci-dessus.

Un fragment d'amplification SEQ ID N°79 d'une taille de 288 pb, obtenu à l'aide du couple (SEQ ID N°11, SEQ ID N°12), est caractéristique du canard du genre *Anas,* et notamment de l'espèce *Anas platyrhynchos* (canard colvert) ou du genre *Cairina,* et notamment de l'espèce *Cairina moschata* (canard de barbarie), et présente la séquence nucléotidique suivante : 
YMYYKRYACT GATGCACTTK GDYCDCATTY RGTTRWKGYY MYNTCCRCHV HYCYABNBWH
WVRTGYTRYY NRGTGAATGC TYGHHGGACA TAMHHHMNRA AYDMAACHRH YHHVHMHHHN
HNHYNHHNNY HHHWAYWBVH WHWCHDWMMN RWWHDMAYDD HDYVDHHYAW MHTTATTAKA
GRAACWCCAG YACYARVVVH DVWHNHAMHY NRRHRWHHMY YAYWAHYYHV HYNTYNMNMH
HNBNVYHYRD HYHRYYARYB RMHYVHHHYR YYCDCMTWSY WYMMHNHH
 dans laquelle Y est C ou T, M est A ou C, N est A, C, G ou T, K est G ou T, B est C, G ou T, R est A ou G, W est A ou T, S est C ou G, D est A, G ou T, V est A, C ou G, H est A, C ou T.

Un fragment d'amplification SEQ ID N°78 d'une taille de 328 pb, obtenu à l'aide du couple (SEQ ID N°11, SEQ ID N°12), est caractéristique du cygne du genre *Cygnus* et notamment de l'espèce *Cygnus cygnus* (cygne sauvage), et présente la séquence nucléotidique suivante : 
YMYYKRYACT GATGCACTTK GDYCDCATTY RGTTRWKGYY MYNTCCRCHV HYCYABNBWH
WVRTGYTRYY NRGTGAATGC TYGHHGGACA TAMHHHMNRA AYDMAACCAY DMAACHRHYH
HVHMHHHNHN HYNHHNNYRH HWAYYBCHNN YHHWAWYMYS YYWHHYNWBV HWHNBYBDMD
MDWCHDWMNR WWHDMAYDDH DYVDHHYAWM HTTATTAKAG RAACWCCAGY ACYARVVVHD
VWHNHAMHYN RRHRWHHMYY AYWAHYYHVH YNTYNMNAMH HNBNVYHYRD HYHRYYARYB
RMHYVHHHYR YYCDCMTWSY WYMMHNHH
 dans laquelle Y est C ou T, M est A ou C, N est A, C, G ou T, K est G ou T, B est C, G ou T, R est A ou G, W est A ou T, S est C ou G, D est A, G ou T, V est A, C ou G, H est A, C ou T.

Un fragment SEQ ID N°77 d'une taille de 331 pb, obtenu à l'aide du couple (SEQ ID N°11, SEQ ID N°12), est caractéristique du poulet du genre *Gallus,* et notamment de l'espèce *Gallus gallus* (poulet commun) et présente la séquence nucléotidique suivante : 
YMYYKRCTGA TGCACTTKGD YCDCATTYRG TTRWKGYYMY CCRCHVHYCY ABNBWHWVRT
GYTRYYNRGT GAATGCTYGH HGGACATADY WYWAWMMAWH WMHYWYYHWS YRYWWYYYHY
RSRMHHHMRA AYDMAACCAR HRDWYTTYMD CHRWTHDYRT MWWHYHVHMH HNHWAYYBCH
NNYHDMDWCH DWMMRWWHDM AYDDHDYVDH HYAWHTTATT AKAGRAACWC CAGYACYARV
VVHDVWHNHA MHYNRRHRWH MYYAYWAHYY HVHYNTYNMN AMHHNBNVYH YRDHYHRYYA
RYBRMHYVHH HYRYYCDCMT WSYWYMMHNH H
 dans laquelle Y est C ou T, M est A ou C, N est A, C, G ou T, K est G ou T, B est C, G ou T, R est A ou G, W est A ou T, S est C ou G, D est A, G ou T, V est A, C ou G, H est A, C ou T.

En effet, les amorces SEQ ID N°11 et SEQ ID N°12 encadrent une partie de la région de contrôle de l'ADMmt qui varie du fait d'une délétion d'ADN respectivement chez le canard, le cygne et le poulet par rapport à l'oie.

Selon un mode de mise en oeuvre particulièrement avantageux, une partie des étapes d'hybridation des cycles constituant la réaction d'amplification est effectuée à une température d'environ 50°C à environ 58 °C. En outre, le demandeur a trouvé qu'une température de 55°C était particulièrement appropriée pour obtenir une amplification spécifique. Un tel mode de mise en oeuvre permet d'obtenir une plus grande spécificité d'amplification.

Les fragments SEQ ID N° 13, SEQ ID N° 79, SEQ ID N° 78 et SEQ ID N° 77 amplifiés obtenus sont soumis à différentes enzymes de restriction spécifiques de différents genres d'oiseaux de la famille des anatidés ou des gallinacés. Les enzymes ont été choisies afin d'avoir des sites de coupures différents sur les séquences des genres choisis.

A titre d'exemple on pourra citer, lorsqu'il s'agit d'identifier une séquence d'ADN amplifiée provenant d'anatidés, les enzymes de restriction choisies dans le groupe constitué par Nsi I, Acc I, Hha I ou Nde I ou leurs mélanges.

Les enzymes Nsi I, Acc I, Hha I et Nde I sont spécifiques des genres d'anatidés les plus fréquemment trouvés dans des échantillons de matière organique. Plus particulièrement, l'enzyme Nsi I est spécifique du genre *Anas,* regroupant notamment les espèces *Anas platyrhynchos* (canard pilet), *Anas acuta* (canard pilet), *Anas querquedula* (sarcelle) ; l'enzyme Acc I est spécifique du genre *Cairina*, englobant notamment l'espèce *Cairina moschata* (canard de barbarie) ; l'enzyme Hha I est spécifique du genre *Anser,* regroupant notamment l'espèce *Anser anser* (oie commune), *Anser caerulescens* (oie des neiges) ; l'enzyme Nde I est spécifique du genre *Cygnus* regroupant notamment les espèces *Cygnus cygnus* (cygne sauvage), *Cygnus atratus* (cygne noir).

Lorsqu'il s'agit d'identifier une séquence d'ADN amplifiée provenant de gallinacés, on utilisera l'enzyme de restriction BsaJ I.

L'enzyme BsaJ I est spécifique du genre de gallinacés le plus fréquemment trouvé dans un échantillon de matière organique, à savoir le genre *Gallus*, englobant notamment l'espèce *Gallus gallus* (poulet).

On détermine ainsi, en fonction de la taille des fragments obtenus à l'aide des enzymes de restriction susmentionnées, l'espèce d'anatidés ou de gallinacés présente dans l'échantillon de matière organique, et on peut ainsi différencier les espèces les unes par rapport aux autres.

A titre d'exemple on obtient, à l'aide de l'enzyme Nsi I, deux fragments d'une taille respective de 154 pb et 134 pb, caractéristiques du genre *Anas.* L'enzyme Acc I conduit à deux fragments d'une taille respective de 147 pb et 141 pb, caractéristiques du genre *Cairina*. L'enzyme Hha I conduit à trois fragments d'une taille respective de 11 pb, 113 pb et 284 pb, caractéristiques du genre *Anser.* L'enzyme Nde I conduit à deux fragments d'une taille respective de 132 pb et 196 pb, caractéristiques du genre *Cygnus.* L'enzyme BsaJ I conduit à deux fragments d'une taille de 50 pb et 281 pb, caractéristiques du genre *Gallus.*

### 3) La méthode d'identification directe, par simple visualisation de la présence du fragment d'ADN amplifié à l'aide d'une électrophorèse sur gel, est dénommée dans ce qui suit " stratégie spécifique ", car elle permet de détecter et d'identifier directement des espèces d'anatidés, de gallinacés et de struthionidés bien spécifiques. Dans la " stratégie spécifique ", l'étape de détection et d'identification est une étape simultanée.

En effet, cette méthode ne requiert pas, contrairement aux deux méthodes décrites ci-dessus, de procéder à une étape supplémentaire après l'obtention de la séquence amplifiée et permet ainsi de détecter et d'identifier directement après amplification certaines espèces d'oiseaux bien particulières, qui sont les espèces domestiques les plus courantes car les plus utilisées dans les préparations alimentaires ou autres. Ces espèces d'oiseaux spécifiques pouvant être directement détectées et identifiées sont :
- le canard colvert ou commun (*Anas platyrhynchos)*, le canard de barbarie *(Cairina moschata*) et l'oie commune *(Anser anser*) pour les oiseaux appartenant à la famille des anatidés (ordre des ansériformes),
- le poulet *(Gallus gallus*), la caille (*Coturnix coturnix)* et la dinde *(Meleagris gallopavo*) pour les oiseaux appartenant à la famille des gallinacés (ordre des galliformes),
- l'autruche *(Struthio camelus*) pour les oiseaux appartenant à la famille des struthionidés (ordre des struthioniformes).

L'amplification de l'ADN est effectuée par la méthode d'amplification en chaîne par polymérase (PCR) comme décrit précédemment.

La stratégie spécifique permettant de détecter et d'identifier la présence d'anatidés dans un échantillon de matière organique est décrite plus en détails ci-dessous.

### a) Stratégie spécifique permettant d'identifier les anatidés.

Les amorces utilisées afin d'effectuer l'amplification par la méthode PCR d'une séquence d'ADN provenant d'anatidés, éventuellement présente dans un échantillon de matière organique à analyser, sont les 18 amorces listées ci-dessous et telles que définies ci-dessus :
SEQ ID N°46, SEQ ID N°47, SEQ ID N°14, SEQ ID N°48, SEQ ID N°49, SEQ ID N°15, SEQ ID N°50, SEQ ID N-51, SEQ ID N°16, SEQ ID N°52, SEQ ID N°53, SEQ ID N°17, SEQ ID N°54, SEQ ID N°55, SEQ ID N°18, SEQ ID N°56, SEQ ID N°57 et SEQ ID N°19,
et plus particulièrement les 6 amorces SEQ ID N°14, SEQ ID N°15, SEQ ID N°16, SEQ ID N°17, SEQ ID N°18 et SEQ ID N°19.

Selon un mode de réalisation avantageux du procédé décrit, l'utilisation des couples d'oligonucléotides suivants :
- (SEQ ID N°14, SEQ ID N°15),
- (SEQ ID N°16, SEQ ID N°17) et
- (SEQ ID N°18, SEQ ID N°19),
permettent respectivement d'obtenir :
- un fragment SEQ ID N°20 de 180 paires de bases tel que défini ci-dessus,
- un fragment SEQ ID N°21 de 191 paires de bases tel que défini ci-dessus et,
- un fragment SEQ ID N°22 de 176 paires de bases tel que défini ci-dessus.

Selon un mode de mise en oeuvre particulièrement avantageux, une partie des étapes d'hybridation des cycles constituant la réaction d'amplification est effectuée à une température de 63 °C qui est particulièrement appropriée pour obtenir une amplification spécifique. Un tel mode de mise en oeuvre permet d'obtenir une plus grande spécificité d'amplification.

La présence des fragments SEQ ID N°20, SEQ ID N°21 et SEQ ID N°22 après amplification est détectée par simple visualisation par électrophorèse sur gel d'agarose.

La visualisation du fragment SEQ ID N°20 permet de détecter et d'identifier la présence de canard colvert *(Anas platyrhynchos*). La visualisation du fragment SEQ ID N°21 permet de détecter et d'identifier la présence de canard de barbarie *(Cairina moschata*). La visualisation du fragment SEQ ID N°22 permet de détecter et d'identifier la présence d'oie commune *(Anser anser*).

La stratégie spécifique permettant de détecter et d'identifier la présence de gallinacés ou de struthionidés dans un échantillon de matière organique est décrite plus en détails ci-dessous.

### b) Stratégie spécifique permettant d'identifier les gallinacés ou les struthionidés.

Les amorces utilisées afin d'effectuer l'amplification par la méthode PCR d'une séquence d'ADN provenant de galliformes, éventuellement présente dans un échantillon de matière organique à analyser, sont les 12 amorces listées ci-dessous et telles que définies ci-dessus :
SEQ ID N°58, SEQ ID N°59, SEQ ID N°23, SEQ ID N°60, SEQ ID N°61, SEQ ID N°24, SEQ ID N°62, SEQ ID N°63, SEQ ID N°25, SEQ ID N°64, SEQ ID N°65, SEQ ID N°26, SEQ ID N°70, SEQ ID N°71, SEQ ID N°72, SEQ ID N°73, SEQ ID N°74 et SEQ ID N°75,
et plus particulièrement les 6 amorces SEQ ID N°23, SEQ ID N°24, SEQ ID N°25, SEQ ID N°26, SEQ ID N°72 et SEQ ID N°75.

Selon un mode de réalisation avantageux du procédé décrit, l'utilisation des couples d'amorces oligonucléotidiques suivants :
- (SEQ ID N°23, SEQ ID N°24),
- (SEQ ID N°25, SEQ ID N°26),
- (SEQ ID N°72, SEQ ID N°75) et,
permettent respectivement d'obtenir :
- le fragment SEQ ID N°29 de 231 paires de bases tel que défini ci-dessus,
- le fragment SEQ ID N°30 de 175 paires de bases tel que défini ci-dessus,
- le fragment SEQ ID N°76 de 231 paires de bases tel que défini ci-dessus.

Les amorces utilisées afin d'effectuer l'amplification par la méthode PCR d'une séquence d'ADN provenant de struthioniformes, éventuellement présente dans un échantillon de matière organique à analyser, sont les 6 amorces listées ci-dessous et telles que définies ci-dessus :
SEQ ID N°66, SEQ ID N°67, SEQ ID N°27, SEQ ID N°68, SEQ ID N°69 et SEQ ID N°28, et plus particulièrement les amorces SEQ ID N°27 et SEQ ID N°28.

Selon un mode de réalisation avantageux du procédé décrit, l'utilisation du couple d'oligonucléotides (SEQ ID N°27, SEQ ID N°28), permet d'obtenir le fragment SEQ ID N°31 de 216 paires de bases tel que défini ci-dessus.

Selon un mode de mise en oeuvre particulièrement avantageux du procédé décrit, une partie des étapes d'hybridation des cycles constituant la réaction d'amplification est effectuée à une température de 55 °C qui est particulièrement appropriée pour obtenir une amplification spécifique. Un tel mode de mise en oeuvre permet d'obtenir une plus grande spécificité d'amplification.

La visualisation du fragment SEQ ID N°29 permet de détecter et d'identifier la présence de poulet (*Gallus gallus*). La visualisation du fragment SEQ ID N°30 permet de détecter et d'identifier la présence de caille (*Coturnix coturnix*). La visualisation du fragment SEQ ID N°76 permet de détecter et d'identifier la présence de dinde *(Meleagris gallopavo*).

La visualisation du fragment SEQ ID N°31 permet de détecter et d'identifier la présence d'autruche (*Struthio camelus*).

Le produit d'amplification obtenu, identifié à l'aide de la stratégie intermédiaire ou de la stratégie spécifique, est généralement situé dans la partie 5' de la région de contrôle de la réplication de l'ADN mitochondrial, délimitée par les nucléotides situés aux environs des positions 1 et 439, notamment aux environs des positions 80 et 433, et de préférence aux environs des positions 150 et 433 de la région de contrôle de la réplication de l'ADN mitochondrial, ou dans la partie 3' de la région de contrôle de la réplication de l'ADN mitochondrial, délimitée par les nucléotides situés aux environs des positions 750 et 1227, notamment aux environs des positions 780 et 1227, et de préférence aux environs des positions 800 et 1227 de la région de contrôle de la réplication de l'ADN mitochondrial, lesdites positions étant définies d'après Desjardins et Morais, 1990, J. Mol. Biol., 599-634.

Est également décrite l'utilisation de séquences nucléotidiques choisies parmi les amorces oligonucléotidiques telles que définies ci-dessus, ou les fragments d'ADN tels que définis ci-dessus, pour la mise en oeuvre d'une méthode de détection de la présence de matières biologiques provenant d'oiseaux, notamment d'ansériformes, de galliformes ou de struthioniformes, et/ou d'identification de l'espèce d'oiseaux, notamment d'ansériformes, de galliformes ou de struthioniformes présente, dans un échantillon de matière organique susceptible de contenir de telles matières biologiques.

A titre d'exemple d'échantillon de matière organique, on peut citer les produits agro-alimentaires tels que le foie gras, les pâtés, les viandes, les graisses, les plats cuisinés, les oeufs etc...., les produits manufacturés tels que plumes, les duvets, les parures a bases de plumes etc..., les oeufs de collection, les plumes de collection, les animaux vivants d'élevages ou sauvages, les animaux taxidermisés etc....

Est également décrite l'utilisation telle que définie ci-dessus pour la mise en oeuvre d'une méthode de détection de la présence de matières biologiques provenant d'oiseaux, notamment d'ansériformes, de galliformes ou de struthioniformes, et/ou d'identification de l'espèce d'oiseaux, notamment d'ansériformes, de galliformes ou de struthioniformes présente, dans des échantillons de matières organiques frais ou transformés tels que les produits agro-alimentaires, notamment le foie gras, les pâtés, les viandes, les graisses, les plats cuisinés, les oeufs, les produits manufacturés tels que ceux à base de plumes.

### Description des figures.

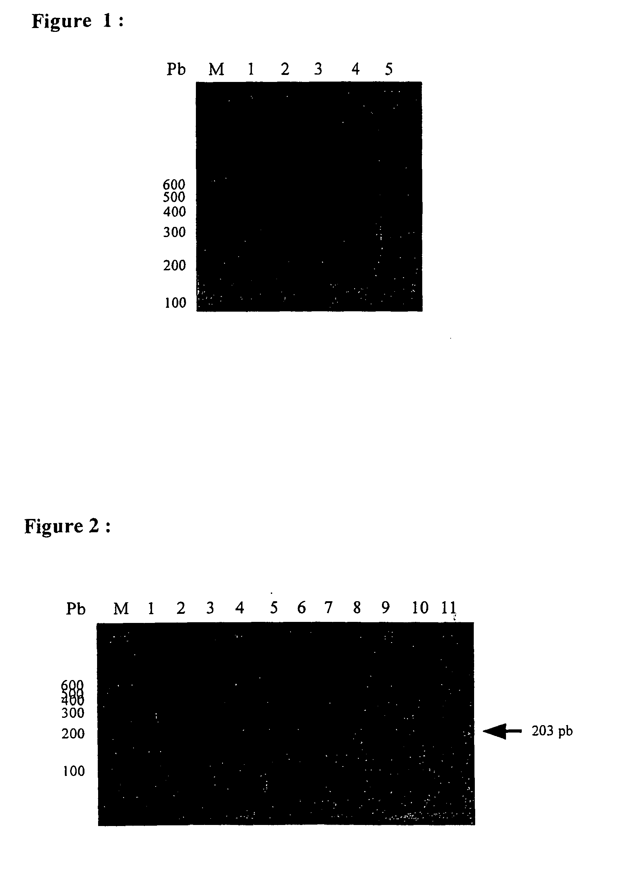

La **figure 1** représente un gel d'agarose coloré par du bromure d'éthidium. Un marqueur de taille de 100 paires de bases (pb) (M) est déposé sur le gel. Les ADN sont extraits à partir de différents échantillons de matières organiques tels que des plumes (puits 1), du sang (puits 2), des os (puits 3), de la mousse de canard (puits 4), des plats cuisinés (puits 5), et sont déposés sur le gel.
La **figure 2** représente un gel d'agarose coloré par du bromure d'éthidium. Un marqueur de taille de 100 pb (M) est déposé sur le gel. Les ADN de canard colvert (puits 1), de canard de barbarie (puits 2), de canard pilet (puits 3), d'eider (puits 4), d'oie (puits 5), de cygne (puits 6), de poulet (puits 7), de caille (puits 8), de dinde (puits 9) et d'autruche (puits 10) ont été amplifiés à l'aide des amorces SEQ ID N°1 et SEQ ID N°5. Le produit d'amplification obtenu SEQ ID N°6 migre en formant une bande de 203 paires de bases (pb). Le puits 11 correspond à un témoin négatif d'amplification.

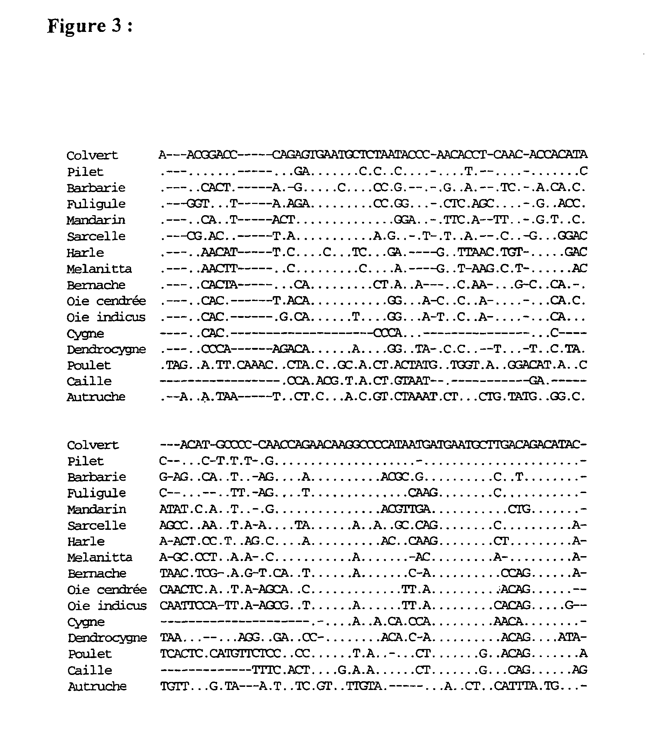

La **figure 3** représente des alignements de séquences nucléotidiques de 120 paires de bases (120 pb) de la région de contrôle de la réplication de l'ADN mitochondrial de différentes espèces d'oiseaux, notamment d'ansériformes, de galliformes ou de struthioniformes. Les séquences obtenues après séquençage sont alignées et comparées avec les séquences déjà connues. Les séquences des différentes espèces d'oiseaux représentées sont respectivement les suivantes (en allant de haut en bas de la figure) : canard colvert, canard pilet, canard de barbarie, fuligule, canard mandarin, sarcelle, harle, melanitta, bernache, oie cendrée, oie indicus, cygne, dendrocygne, poulet, caille et autruche. Les points représentent les bases conservées avec celles du canard colvert (séquence de référence). Les tirets représentent des délétions de bases.

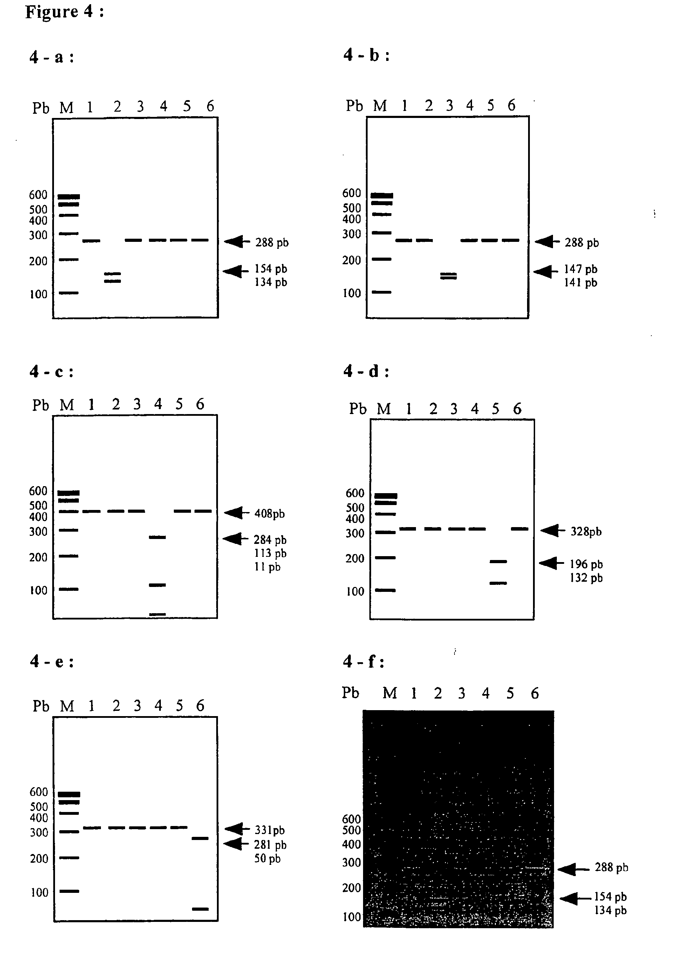

Les **figures 4-a, 4-b, 4-c, 4-d, 4-e** représentent les profils de migration attendus après digestion enzymatique du fragment SEQ ID N°13 et dépôt sur un gel d'agarose à 2% après coloration par du bromure d'éthidium. Un marqueur de taille de 100 bp (M) est déposé sur le gel. La **figure 4-f** représente le profil de migration obtenu après digestion enzymatique du fragment SEQ ID N°13 et dépôt sur un gel d'agarose à 2% après coloration par du bromure d'éthidium. Un marqueur de taille de 100 bp (M) est déposé sur le gel. Le fragment SEQ ID N°13 est obtenu à l'aide des amorces SEQ ID N°11 et SEQ ID N°12, par amplification d'ADNmt extrait d'un plat cuisiné dont on cherche à détecter et identifier le genre d'oiseau présent dans ledit plat.
La **figure 4-a** représente plus particulièrement le profil attendu pour le genre *Anas.* Le fragment SEQ ID N°13 de 288 pb est déposé dans le puits 1 sans avoir été digéré, et sert donc de contrôle en le comparant avec les autres fragments mis en présence des différentes enzymes. Le puits 2 est le même fragment SEQ ID N°13 digéré avec l'enzyme Nsi I, spécifique du genre *Anas :* on observe une coupure engendrant deux fragments de 154 et 134 paires de bases, qui sont bien les fragments spécifiques du genre *Anas.* Le puits 3 est le même fragment SEQ ID N°13 digéré par l'enzyme Acc I, spécifique du genre *Cairina :* aucune coupure n'est observée, étant donnée que Acc I n'est pas spécifique du genre *Anas.* Le puits 4 est le même fragment SEQ ID N°13 digéré par l'enzyme Hha I, spécifique du genre *Anser :* aucune coupure n'est observée. Le puits 5 est le même fragment SEQ ID N°13 digéré par l'enzyme Nde I, spécifique du genre *Cygnus :* aucune coupure n'est observée. Le puits 6 est le même fragment SEQ ID N°13 digéré par l'enzyme BsaJ I, spécifique du genre *Gallus* : aucune coupure n'est observée.

Le fragment SEQ ID N°13 obtenu après amplification est à chaque fois mis en présence de toutes les enzymes, car il peut arriver qu'il y ait un mélange de genres, et ainsi on peut obtenir un profil où l'enzyme spécifique du genre *Anas* coupe SEQ ID N°13, et l'enzyme spécifique du genre *Cairina* coupe aussi SEQ ID N°13. Les deux genres sont donc présents, il y a donc un mélange d'espèces dans l'échantillon de matière organique.

La **figure 4-b** est le profil attendu pour le genre *Cairina*. Le fragment SEQ ID N°13 de 288 pb est déposé dans le puits 1 sans avoir été digéré (contrôle). Le puits 2 est le fragment SEQ ID N°13 digéré avec l'enzyme Nsi I : aucune coupure n'est observée, étant donnée que Nsi I n'est pas spécifique du genre *Cairina*. Le puits 3 est le fragment SEQ ID N°13 digéré par l'enzyme Acc I : on observe une coupure engendrant deux fragments de 147 et 141 paires de bases, qui sont bien les fragments spécifiques du genre *Cairina.* Le puits 4 est le fragment SEQ ID N°13 digéré par l'enzyme Hha I : aucune coupure n'est observée. Le puits 5 est le fragment SEQ ID N°13 digéré par l'enzyme Nde I : aucune coupure n'est observée. Le puits 6 est le fragment SEQ ID N°13 digéré par l'enzyme BsaJ I : aucune coupure n'est observée.

La **figure 4-c** est le profil attendu pour le genre *Anser.* Le fragment SEQ ID N°13 de 408 pb est déposé dans le puits 1 sans avoir été digéré (contrôle). Les puits 2 et 3 représentent respectivement le fragment SEQ ID N°13 digéré avec les enzymes Nsi I et Acc I : aucune coupure n'est observée. Le puits 4 représente le fragment SEQ ID N°13 digéré avec Hha I : on observe une coupure engendrant trois fragments de 11, 113 et 284 paires de bases, qui sont bien les fragments spécifiques du genre *Anser*. Les puits 5 et 6 représentent respectivement le fragment SEQ ID N°13 digéré par l'enzyme Nde I et BsaJ I : aucune coupure n'est observée.

La **figure 4-d** est le profil attendu pour le genre *Cygnus.* Le fragment SEQ ID N°13 de 328 pb est déposé dans le puits 1 sans avoir été digéré (contrôle). Les puits 2, 3 et 4 représentent respectivement le fragment SEQ ID N°13 digéré avec les enzymes Nsi I, Acc I et Hha I : aucune coupure n'est observée. Le puits 5 représente le fragment SEQ ID N°13 digéré avec Nde I : on observe une coupure engendrant deux fragments de 132 et 196 paires de bases, qui sont bien les fragments spécifiques du genre *Cygnus.* Le puits 6 représentent le fragment SEQ ID N°13 digéré par l'enzyme BsaJ I : aucune coupure n'est observée.

La **figure 4-e** est le profil attendu pour le genre *Gallus.* Le fragment SEQ ID N°13 de 331 pb est déposé dans le puits 1 sans avoir été digéré (contrôle). Les puits 2, 3, 4 et 5 représentent respectivement le fragment SEQ ID N°13 digéré avec les enzymes Nsi I, Acc I, Hha I et Nde I : aucune coupure n'est observée. Le puits 6 représente le fragment SEQ ID N°13 digéré avec BsaJ I : on observe une coupure engendrant deux fragments de 50 et 281 paires de bases, qui sont bien les fragments spécifiques du genre *Gallus.*

Dans la **figure 4-f**, l'exemple choisi est celui d'un plat cuisiné dont l'ADN a été extrait et amplifié à l'aide des amorces SEQ ID N°11 et SEQ ID N°12. Le puits 1 correspond au fragment d'amplification SEQ ID N 13 non digéré, d'une taille de 288 pb (contrôle). Le profil obtenu à la figure 4-f, après amplification et digestion on obtient le profil 4-f, est comparé avec les différents profils attendus : on constate que le profil 4-f correspond à celui de la figure 4-a. Ainsi, le plat cuisiné analysé contient donc un oiseau du genre *Anas,* appartenant à la famille des anatidés.

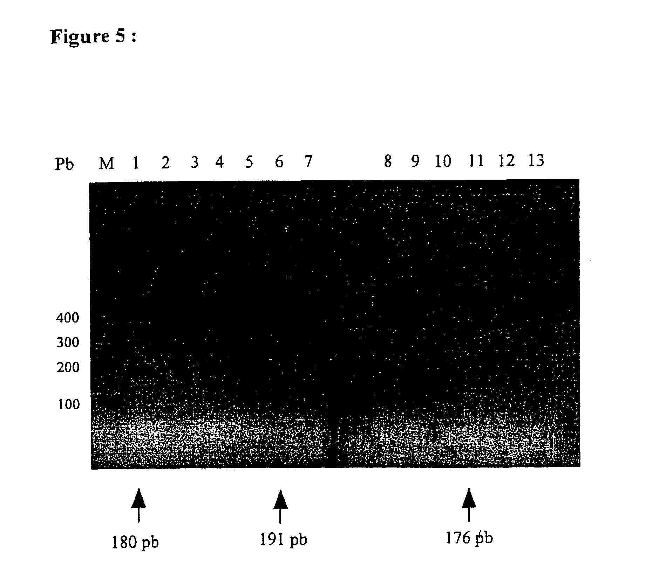

La **figure 5** représente un gel d'agarose coloré par du bromure d'éthidium sur lequel est déposé un marqueur de taille de 100 pb (M).

Les ADN de canard colvert (puits 1), de canard de barbarie (puits 2), d'oie (puits 3) et de poulet (puits 4) ont été amplifiés à l'aide des amorces SEQ ID N°14 et SEQ ID N°15, qui permettent d'obtenir un fragment d'ADN SEQ ID N°20 d'une taille de 180 pb. Seul un fragment d'amplification est observé pour le canard Colvert.

Les ADN de canard colvert (puits 5), de canard de barbarie (puits 6), d'oie (puits 7) et de poulet (puits 8) ont été amplifiés à l'aide des amorces SEQ ID N°16 et SEQ ID N°17, qui permettent d'obtenir un fragment d'ADN SEQ ID N°21 d'une taille de 191 pb. Seul un fragment d'amplification est observé pour le canard de Barbarie.

Les ADN de canard colvert (puits 9), de canard de barbarie (puits 10), d'oie (puits 11) et de poulet (puits 12) ont été amplifiés à l'aide des amorces SEQ ID N°18 et SEQ ID N°19, qui permettent d'obtenir un fragment d'ADN SEQ ID N°22 d'une taille de 176 pb. Seul un fragment d'amplification est observé pour l'oie commune.

Le puits 13 représente un témoin négatif d'amplification.

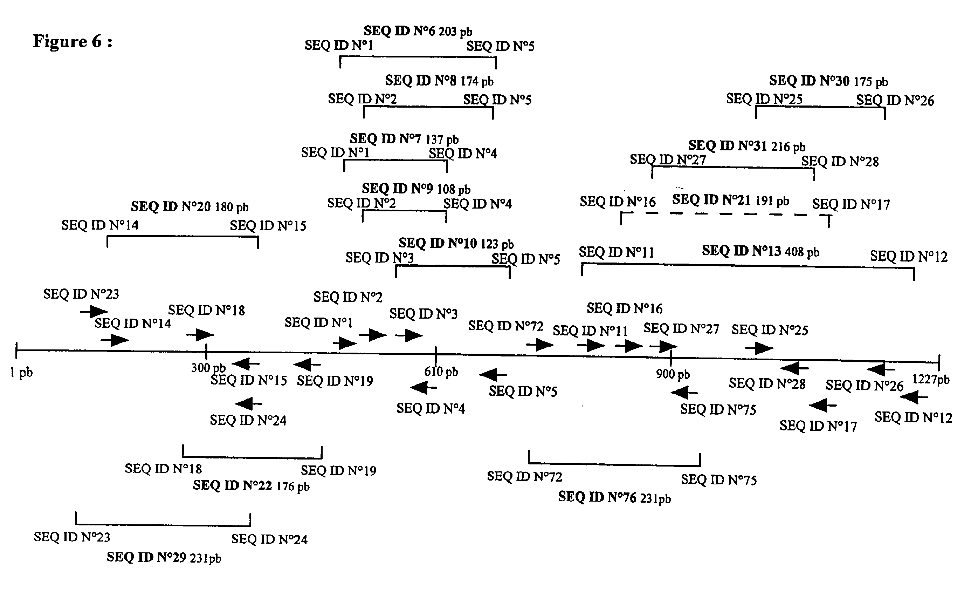

La **figure 6** représente les positions, sur la région de contrôle de la réplication de l'ADN mitochondrial, lesdites positions étant définies par Desjardins et Morais, 1990, J. Mol. Biol., 599-634 :
- des amorces oligonucléotidiques de l'invention, à savoir SEQ ID N°1, SEQ ID N°2, SEQ ID N°3, SEQ ID N°4, SEQ ID N°5, SEQ ID N°11, SEQ ID N°12, SEQ ID N°14, SEQ ID N°15, SEQ ID N°16, SEQ ID N°17, SEQ ID N°18, SEQ ID N°19, SEQ ID N°23, SEQ ID N°24, SEQ ID N°25, SEQ ID N°26, SEQ ID N°72, SEQ ID N°75, SEQ ID N° 27, SEQ ID N° 28 et,
- des fragments d'ADN amplifiés, à l'aide des amorces de l'invention, à savoir SEQ ID N°6, SEQ ID N°7, SEQ ID N°8, SEQ ID N°9, SEQ ID N°10, SEQ ID N°13, SEQ ID N°20, SEQ ID N°21, SEQ ID N°22, SEQ ID N°29, SEQ ID N°30, SEQ ID N°76 et SEQ ID N°31,

Le **tableau 1** ci-après représente la liste de tous les anatidés existants, pouvant être détectés et identifiés à l'aide du procédé décrit (Stratégie globale).

**Tableau 1**

| **NOM LATIN** | **NOM VERNACULAIRE** |
|---|---|
| *Aix galericulata* | Canard Mandarin |
| *Aix sponsa* | Canard carolin |
| *Alopochen oegyptiacus* | Oie d'Egypte |
| *Amazonetta brasiliensis* | Sarcelle du Brésil |
| *Anas acuta* | Canard Pilet |
| *Anas americana* | Canard siffleur d'Amérique |
| *Anas aucklandica* | Sarcelle brune |
| *Anas bahamensis* | Canard Pilet des Bahamas |
| *Anas bernieri* | Sarcelle malgache |
| *Anas capensis* | Canard du Cap |
| *Anas castenea* | Sarcelle d'Australie |
| *Anas clypeata* | Canard souchet d'Europe |
| *Anas crecca* | Sarcelle d'hiver |
| *Anas cyanoptera* | Sarcelle à ailes bleues |
| *Anas discors* | Sarcelle soucrourou |
| *Anas erythrorhyncha* | Canard à bec rouge |
| *Anas falcata* | Sarcelle à faucilles |
| *Anas flavirostris* | Sarcelle mouchetée |
| *Anas formosa* | Sarcelle élégante |
| *Anas georgica* | Pilet de Georgie ou du Chili |
| *Anas gibberifrons* | Sarcelle grise |
| *Anas hottenta* | Sarcelle hottentote |
| *Anas laysanensis* | Canard de Laysan |
| *Anas luzonica* | Canard des Philippines |
| *Anas melleri* | Cnard de Meller |
| *Anas penelope* | Canard siffleur d'Europe |
| *Anas platalea* | Souchet roux |
| *Anas plathyrhynchos* | Canard Colvert |
| *Anas poecilorhyncha* | Canard à bec tacheté |
| *Anas querquedula* | Sarcelle d'été |
| *Anas rhynchotis* | Souchet d'Australie |
| *Anas rubripes* | Canard noirâtres |
| *Anas sibilatrix* | Siffleur du Chili |
| *Anas smithi* | Souchet du Cap |
| *Anas sparsa* | Canard tacheté |
| *Anas specularis* | Canard à ailes bronzées |
| *Anas strepera* | Canard chipeau |
| *Anas superciliosa* | Canard à sourcils |
| *Anas undulata* | Canard à bec jaune |
| *Anas versicolor* | Sarcelle versicolore |
| *Anas waigiuensis* | Canard de Salvadori |
| *Anas wywilliana* | Canard des Hawaï |
| *Anseranas semipalmata* | Oie semi palmée |
| *Anser albifrons* | Oie a front blanc |
| *Anser anser* | Oie cendrée |
| *Anser brachyrhynchus* | Oie à bec court |
| *Anser caerulescens* | Oie des neiges |
| *Anser cygnoïdes* | Anser cygnoide |
| *Anser erythropus* | Oie naine |
| *Anser fabalis* | Oie des moissons |
| *Anser indicus* | Oie à tête barrée |
| *Anser rosii* | Oie de Ross |
| *Anser canagicus* | Oie empereur |
| *Anseranas semipalmata* | Oie semi-palmée |
| *Aythya affinis* | Petit milouinan d'Amérique |
| *Aythya americana* | Milouin d'Amérique |
| *Aythya australis* | Fuligule nycora du Sud |
| *Aythya baeri* | Fuligule nycora de Baer |
| *Aythya collaris* | Fuligule morillon à collier |
| *Aythya fuligula* | Fuligule morillon |
| *Aythya innotata* | Fuligule nycora de Madagascar |
| *Aythya marila* | Fuligule milouinan |
| *Aythya novoe-seelandioe* | Fuligule nycora de Nouvelle-Zélande |
| *Aythya nycora* | Fuligule nycora |
| *Aythya vasilineria* | Milouin à dos blanc |
| *Biziura lobata* | Erismature à barbillons |
| *Branta bernicla* | Bernache cravant |
| *Branta canadensis* | Bernache du canada |
| *Branta leucopsis* | Bernache nonnette |
| *Branta ruficollis* | Bernache à cou roux |
| *Branta sandvicensis* | Bernacge néné |
| *Bucephala albeola* | Garrot albéole |
| *Bucephala clangula* | Garrot à oeil d'or |
| *Bucephala islandica* | garrot d'Islande |
| *Cairina moschata* | Canard de Barbarie |
| *Cairina scutulata* | Canard musqué |
| *Calonetta leucophrys* | Sarcelle à collier |
| *Calonetta leucophrys* | Sarcelle à collier |
| *Cereopse novoe-hollandioe* | Oie cereopse |
| *Chauna torquota* | Kamichi à collier |
| *Chenonetta jubata* | Bernache à Crinière |
| *Chloëphaga hybrida* | Petite Bernache antarctique |
| *Chloëphaga melanoptera* | Bernache des Andes |
| *Chloëphaga picta* | Bernache de Magellan |
| *Chloëphaga poliocephala* | Bernache à tête grise |
| *Chloëphaga rubidiceps* | Bernache à tête rousse |
| *Clangula hyemalis* | Harelde de Miquelon |
| *Coscoroba coscoroba* | Cygne coscoroba |
| *Cyanochen cyanopterus* | Bernache à ailes bleues |
| *Cygnus atratus* | Cygne noir |
| *Cygnus columbianus* | Cygne siffleur |
| *Cygnus cygnus* | Cygne sauvage |
| *Cygnus melanocoryphus* | Cygne à col noir |
| *Cygnus olor* | Cygne tuberculé |
| *Dendrocygna arborea* | Dendrocygne des Antilles |
| *Dendrocygna arcuata* | Dendrocygne à lunules |
| *Dendrocygna autumnalis* | Dendrocygne à ventre noir |
| *Dendrocygna bicolor* | Dendrocygne bicolore |
| *Dendrocygna eytoni* | Dendrocygne d'Eyton |
| *Dendrocygna guttata* | Dendrocygne tacheté |
| *Dendrocygna javanica* | Dendrocygne de l'Inde |
| *Dendrocygna viduata* | Dendrocygne veuf |
| *Erismature vittata* | Erismature d'Argentine |
| *Heteronetta atricapilla* | Hétéronette à tête noire |
| *Histrionicus histrionicus* | Garrot arlequin |
| *Hymenolaimus malacorhynchus* | Canard bleu de Nouvelle-Zélande |
| *Lophonetta specularoides* | Canard huppée |
| *Malacorhynchus membranaceus* | Canard à oreillons roses |
| *Marmaronetta angustirostris* | Sarcelle marbrée |
| *Melanitta fusca* | Macreuse brune |
| *Melanitta nigra* | Macreuse noire |
| *Melanitta perspicillata* | Macreuse à lunettes |
| *Merganetta armata* | Merganette d'Amérique |
| *Mergus albellus* | Harle piette |
| *Mergus cucullatus* | Harle couronnée |
| *Mergus merganser* | Harle bièvre |
| *Mergus octosetaceus* | Harle du Brésil |
| *Mergus serrator* | Harle huppée |
| *Mergus squamatus* | Harle écaillé |
| *Neochen jubatus* | Oie de l'Orénoque |
| *Netta erythrophthalma* | Nette d'Amérique ou Afrique |
| *Netta peposaca* | Nette à bec rosé |
| *Netta rufina* | Nette rousse |
| *Nettapus auritus* | Anserelle naine |
| *Nettapus coromandelianus* | Anserelle de Coromandel |
| *Nettapus pulchellus* | Anserrele élégante |
| *Oxyura australis* | Erismature australe |
| *Oxyura dominica* | Erismature masquée |
| *Oxyura jamaicensis* | Erismature Rousse |
| *Oxyura leucocephala* | Erismature à tête blanche |
| *Oxyura maccoa* | Erismature d'Afrique |
| *Oxyura vittata* | Erismature d'Argentine |
| *Plectropterus gambensis* | Oie de Gambie |
| *Polysticta stelleri* | Eider de Steller |
| *Pteronetta hartlaubi* | Canard de Hartlaud |
| *Sarkidiornis melanotos* | Sarcidione d'Afrique et d'Amérique |
| *Somateria fischeri* | Eider à lunettes |
| *Somateria mollissima* | Eider à duvet |
| *Somateria spectabilis* | Eider à tête grise |
| *Stictonetta noevosa* | Canard moucheté |
| *Tachyeres brachypterus* | Canard vapeur des îles Falkland |
| *Tachyeres patachonicus* | Canard vapeur de Patagonie |
| *Tachyeres pteneres* | Canard vapeur magellanique |
| *Tadorna cana* | Tadorne du Cap |
| *Tadorna ferruginea* | Tadorne casarca |
| *Tadorna tadorna* | Tadorne de Belon |
| *Tadorna tadornoides* | Casarca d'Australie |
| *Tadorna radjah* | Tadorne radjah |
| *Tadorna variegata* | Tadorne de Paradis |
| *Thalassornis leuconotus* | Canard à dos blanc |

Les exemples ci-après illustrent l'invention. Ils ne la limitent en aucune façon.

### EXEMPLE 1 : Extraction d'ADN.

Afin de pouvoir mettre au point une méthode de détection par amplification génique de matières biologiques provenant d'anatidés dans des produits utilisés en production agro-alimentaire et dans tous les autres domaines utilisant de la matière organique, les expériences décrites dans ce premier exemple ont été réalisées avec divers types d'échantillons représentant des sources potentielles de présence de matières biologiques provenant d'anatidés.

### 1) Extraction d'ADN par la méthode au phénol/chloroforme

Cette méthode s'adresse à tous les types d'échantillons susceptibles de contenir de la matière organique, tels que les plumes, les tissus frais, les produits transformés, le sang, les graisses, les os.

Cette méthode fait appel à des techniques décrites dans les références HÄNNI et al., 1990, C.R. Acad.Sci.Paris., 310, 365-370 et HÄNNI et al., 1995, Nucl.Acids Res., 23, 881-882, concernant l'extraction d'ADN à partir d'os et de dents.

Une quantité d'environ 1 à 2 g d'échantillon de matière organique est incubée pendant deux heures à 37°C dans 400µl de tampon de lyse de composition suivante :
- STE 1X (NaCl 100mM, Tris 10mM à pH 7,4, EDTA (acide éthylènediaminetétraacétique) 1mM),
- SDS 2%,
- protéinase K à 0,5mg/ml.

La protéinase K permet de dégrader les protéines et de libérer les acides nucléiques. Le lysat est ensuite extrait deux fois par un volume de phénol/chloroforme (1/1). On centrifuge pendant 15 minutes à 1000 g, la phase organique est éliminée ce qui permet de se débarrasser de la partie protéique du lysat. L'ADN est précipité par 1/10 de volume d'acétate de sodium 2M puis par 2,5 volumes d'isopropanol et centrifugé 30 minutes à 10 000 g. L'ADN est repris dans de l'eau : on obtient ainsi un extrait d'ADN. Le volume d'eau est fonction de la quantité d'ADN récupéré.

La migration des ADN provenant des divers échantillons donne des traînées caractéristiques d'un ADN dégradé, qui est cependant parfaitement amplifiable (figure 1).

### 2) Extraction d'ADN par la méthode kit d'extraction

Cette méthode est réalisée dans les conditions décrites par le fournisseur Qiagen.

### EXEMPLE 2 : Amplification d'ADN

La PCR est effectuée avec le couple d'amorces SEQ ID N°1 et SEQ ID N°5 défini ci-dessus. Les amplifications sont réalisées dans un volume total de 50µl contenant :
200µg/ml de BSA (Sérum Albumine Bovine),
250 mM de dNTP (désoxynucléotide Triphosphate),
300 ng de chaque amorce,
1,5 mM de chlorure de magnésium (MgCl₂),
tampon de PCR 10X (100mM Tris-HCl pH 8,3 ; 500mM KCl),
1 unité de Taq Polymerase,
qsp 50 µl d'eau distillée stérile,
1µl d'extrait d'ADN.

Le mélange réactionnel est effectué dans une pièce stérile sous hotte à flux horizontal, pour éviter autant que possible les contaminations. Les PCR sont effectuées sur un appareil PCR Ependorff. Chaque PCR est découpée comme suit :
- 1 cycle initial à une température de 94°C pendant 2 minutes puis,
- 40 cycles à une température de 94°C pendant 1 minute, à une température de 55°C à 63°C pendant 1 minute, à une température de 72°C pendant 2 minutes ; au dernier cycle on effectue une élongation terminale à une température de 72°C pendant 7 minutes.

Les produits d'amplification sont analysés par électrophorèse sur gel d'agarose à 2 % sous tension constante de 100 V pendant 30 min, et à l'aide de bromure d'éthidium pour la visualisation des amplifications obtenues.

La réaction de PCR utilisant le couple d'amorces SEQ ID N°1 et SEQ ID N°5 a été effectuée sur une série d'extraits d'ADN d'oiseaux. On observe un produit d'amplification unique (fragment SEQ ID N°6 d'une longueur de 203 paires de bases) pour les ADN d'ansériformes, et notamment d'anatidés. Par contre, aucune bande d'amplification n'est observée pour le poulet (puits 7), la caille (puits 8), la dinde (puits 9) et l'autruche (puits 10) : ainsi, les amorces SEQ ID N°1 et SEQ ID N°5 sont bien spécifiques des ansériformes (**figure 2**).

La totalité du produit de PCR peut être purifiée directement grâce au système "QIAquick PCR Purification Kit" de Qiagen selon les conditions annoncées. La totalité du produit de PCR est passée sur une colonne de gel de silice. Les acides nucléiques sont retenus sur la colonne alors que les autres résidus de la PCR sont élués à la microcentrifugation. Les impuretés sont éliminées et l'ADN est élué par du tampon Tris ou de l'eau. L'ADN ainsi purifié est visualisé et quantifié sur gel d'agarose 1%.

### EXEMPLE 3 : Caractérisation des fragments par séquençage direct (" Stratégie globale ")

Le séquençage automatique est effectué sur les produits de PCR purifiés.

Les amorces utilisées sont celles qui ont servi à amplifier le fragment en question. Le Kit " Perkin -Elmer " est utilisé pour la réaction de PCR, qui est effectuée sur 25 cycles dans les conditions énoncées par le fournisseur. Les produits d'amplification obtenus à l'issue de la réaction PCR sont précipités à l'éthanol et déposés sur un gel de polyacrylamide. Les séquences obtenues sont alors comparées avec celles des banques ou avec les séquences déjà obtenues (figure 3).

### EXEMPLE 4 : Caractérisation des fragments extraits par digestion enzymatique (" Stratégie intermédiaire ")

Une carte de restriction est construite pour chacun des genres les plus fréquemment utilisés dans la composition de substances organiques à base d'anatidés et de gallinacés. Les genres choisis comme références sont les suivants :
- le genre *Anas* pour le canard colvert *(Anas platyrhynchos*), le canard pilet *(Anas acuta*) ou bien la sarcelle *(Anas querquedula*),
- le genre *Cairina* pour le canard de barbarie (*Cairina moschata*),
- le genre *Anser* pour l'oie commune *(Anser anser*), l'oie des neiges *(Anser caerulescens*),
- le genre *Cygnus* pour le cygne sauvage *(Cygnus cygnus*), le cygne noir *(Cygnus atratus*),
- le genre *Gallus* pour le poulet (*Gallus gallus*).

L'exemple choisi est celui d'un plat cuisiné dont on veut détecter et identifier la présence de matières biologiques provenant d'oiseaux (cf **figures 4-a** à **4-f**). L'ADN est extrait du plat cuisiné et est amplifié à l'aide des amorces SEQ ID N°11 et SEQ ID N°12.

Les enzymes ont été choisies afin d'avoir des sites de coupures différents et spécifiques des genres analysés pour le fragment SEQ ID N°13, obtenu à l'aide des amorces SEQ ID N°11 et SEQ ID N°12 par amplification. Rappelons que la taille du fragment SEQ ID N°13 varie entre les genres d'anatidés ou de gallinacés, du fait d'insertions ou de délétions de quelques bases d'ADN pour le canard, l'oie, la cygne et le poulet.

La spécificité des enzymes de restriction utilisées est rappelée dans le **tableau 2** ci-dessous. Un dixième des réactions de PCR a été utilisé directement pour réaliser chaque digestion enzymatique avec les tampons spécifiques des enzymes citées (Sambrook *et al*., 1987). Les digestions ont été réalisées pendant 30 minutes à 37°C. Les produits de digestion ont été testés sur un gel d'agarose 3%.

On obtient deux fragments d'une taille de 154 pb et 134 pb. Le plat cuisiné analysé contient donc du canard du genre *Anas.* Le même fragment SEQ ID N°13 est digéré par d'autres enzymes de restriction spécifiques d'autres genres : aucune coupure n'est observée pour les autres enzymes de restriction. Ainsi, le plat cuisiné est donc uniquement à base d'anas (**figure 4-a à 4-f**).

Le **tableau 2** ci-dessous représente la liste des enzymes de restrictions utilisées pour le fragment SEQ ID N°13.

| **anatidés gallinacés** | **Enzymes de restriction utilisées** | **Nombre de bandes** | **Taille des bandes en paires de bases** |
|---|---|---|---|
| canard | Nsi I | 2 | 154 |
| (genre *Anas)* | | | 134 |
| canard | Acc I | 2 | 147 |
| (genre *Cairina)* | | | 141 |
| oie | Hha I | 3 | 11 |
| | | | 113 |
| (genre *Anser*) | | | 284 |
| cygne | Nde I | 2 | 132 |
| (genre *Cygnus*) | | | 196 |
| poulet | BsaJ I | 2 | 50 |
| (genre *Gallus*) | | | 281 |

### EXEMPLE 5 : " Stratégie spécifique "

La PCR est effectuée avec les couples d'amorces (SEQ ID N°14, SEQ ID N°15), (SEQ ID N°16, SEQ ID N°17) et (SEQ ID N°18, SEQ ID N°19).

Les amplifications sont réalisées dans un volume total de 50µl contenant :
200µg/ml de BSA,
250 mM de dNTP,
300 ng de chaque amorce,
1,5 mM de MgCl₂,
tampon de PCR 10X (100mM Tris-HCl pH 8,3; 500mM KCl),
1 unité de Taq Polymerase,
qsp 50 µl d'eau distillée stérile,
1µl d'extrait d'ADN

Le mélange réactionnel est effectué dans une pièce stérile sous hotte à flux horizontal, pour éviter autant que possible les contaminations. Les PCR sont effectuées sur un appareil PCR Ependorff.

Chaque PCR est découpée comme suit :
- 1 cycle initial à une température de 94°C pendant 2 minutes puis,
- 40 cycles à une température de 94°C pendant 1 minute, à une température de 63°C pendant 1 minute, à une température de 72°C pendant 2 minutes ; au dernier cycle on effectue une élongation terminale à une température de 72°C pendant 7 minutes.

Les produits d'amplification sont analysés par électrophorèse sur gel d'agarose à 2 % sous tension constante de 100 V pendant 30 min, et à l'aide de bromure d'éthidium pour la visualisation des amplifications obtenues (figure 5).

L'utilisation des amorces SEQ ID N°14 et SEQ ID N°15 permet d'obtenir un produit d'amplification SEQ ID N°20 qui migre en formant une bande de 180 paires de bases : ce fragment est bien spécifique du canard colvert (puits 1) puisque aucune autre bande n'est observée pour les autres espèces. Les amorces SEQ ID N°14 et SEQ ID N°15 sont donc bien spécifiques du canard Colvert. L'utilisation des amorces SEQ ID N°16 et SEQ ID N°17 permet d'obtenir un fragment d'amplification SEQ ID N°21 qui migre en formant une bande de 191 paires de bases : ce fragment est bien spécifique du canard de Barbarie (puits 6) puisque aucune autre bande n'est observée pour les autres espèces. Les amorces SEQ ID N°16 et SEQ ID N°17 sont donc bien spécifiques du canard de Barbarie. L'utilisation des amorces SEQ ID N°18 et SEQ ID N°19 permet d'obtenir un fragment d'amplification SEQ ID N°22 qui migre en formant une bande de 176 paires de bases : ce fragment est bien spécifique pour l'oie commune (puits 11) puisque aucune autre bande n'est observée pour les autres espèces. Les amorces SEQ ID N°18 et SEQ ID N°19 sont donc bien spécifiques de l'oie commune.

Le poulet est témoin (puits 13) et permet de montrer que les trois couples d'amorces sont bien spécifiques des ansériformes **(figure 5).**

### LISTE DE SEQUENCES

<110> CNRS
<120> PROCEDE DE DETECTION ET D'IDENTIFICATION DE LA PRESENCE DE MATIERES BIOLOGIQUES PROVENANT D'OISEAUX, ET OLIGONUCLEOTIDES POUR SA MISE EN OEUVRE
<130> WOB 99 CNR ANAT
<140> PCT/FR01/01279
   <141> 2001-04-26
<150> FR 00 05850
   <151> 2000-05-09
<160> 79
<170> PatentIn Ver. 2.1
<210> 1
   <211> 20
   <212> ADN
   <213> Séquence artificielle
<220>
   <223> Description de la séquence artificielle: amorce PCR
<400> 1
   actagcttca ggcccatacg 20
<210> 2
   <211> 20
   <212> ADN
   <213> Séquence artificielle
<220>
   <223> Description de la séquence artificielle: amorce PCR
<400> 2
   aacccctcgc cctcctcaca 20
<210> 3
   <211> 20
   <212> ADN
   <213> Séquence artificielle
<220>
   <223> Description de la séquence artificielle: amorce
   PCR
<400> 3
   atcaattggg ttcactcacc 20
<210> 4
   <211> 20
   <212> ADN
   <213> Séquence artificielle
<220>
   <223> Description de la séquence artificielle: amorce
   PCR
<400> 4
   aagtattcca cagatgccac 20
<210> 5
   <211> 20
   <212> ADN
   <213> Séquence artificielle
<220>
   <223> Description de la séquence artificielle: amorce
   PCR
<400> 5
   aaaaataaaa ggaaccagag 20
<210> 6
   <211> 203
   <212> ADN
   <213> Séquence artificielle
<220>
   <223> Description de la séquence artificielle: fragment d'ADN amplifié à partir du génome d'oiseaux
<400> 6 
acyagcttca ggcccatacg ttccccctaa acccctcgcc ctcctcacat ttttgcgcct 60
ctggttcctc ggtcagggcc atcmattggg ttcactcacc ycymytygcc yttcaaagtg 120
gcatctgtgg ankacbtyca ccwyyycrrt gcgtwatcgc ggcatbytym asywtttwsm 180
cgcctytggt tcymytthty tyt 203
 dans laquelle Y est C ou T, M est A ou C, K est G ou T, B est C, G ou T, R est A ou G, W est A ou T, S est C ou G, H est A, C ou T, N est A, C, G ou T,
<210> 7
   <211> 137
   <212> ADN
   <213> Séquence artificielle
<220>
   <223> Description de la séquence artificielle: fragment d'ADN amplifié à partir du génome d'oiseaux
<400> 7 
acyagcttca ggcccatacg ttccccctaa acccctcgcc ctcctcacat ttttgcgcct 60
ctggttcctc ggtcagggcc atcmattggg ttcactcacc ycymytygcc yttcaaagtg 120
gcatctgtgg ankacbt 137
 dans laquelle Y est C ou T, M est A ou C, N est A, C, G ou T, K est G ou T, B est C, G ou T,
<210> 8
   <211> 174
   <212> ADN
   <213> Séquence artificielle
<220>
   <223> Description de la séquence artificielle: fragment d'ADN amplifié à partir du génome d'oiseaux
   <400> 8 
aacccctcgc cctcctcaca tttttgcgcc tctggttcct cggtcagggc catcmattgg 60
gttcactcac cycymytygc cyttcaaagt ggcatctgtg gankacbtyc accwyyycrr 120
tgcgtwatcg cggcatbyty masywtttws mcgcctytgg ttcymyttht ytyt 174
 dans laquelle Y est C ou T, M est A ou C, N est A, C, G ou T, K est G ou T, B est C, G ou T, R est A ou G, W est A ou T, S est C ou G, H est A, C ou T,
<210> 9
   <211> 108
   <212> ADN
   <213> Séquence artificielle
<220>
   <223> Description de la séquence artificielle: fragment d'ADN amplifié à partir du génome d'oiseaux
<400> 9
   aacccctcgc cctcctcaca tttttgcgcc tctggttcct cggtcagggc catcmattgg 60
   gttcactcac cycymytygc cyttcaaagt ggcatctgtg gankacbt 108
   dans laquelle Y est C ou T, M est A ou C, N est A, C, G ou T, K est G ou T, B est C, G ou T,
<210> 10
   <211> 123
   <212> ADN
   <213> Séquence artificielle
<220>
   <223> Description de la séquence artificielle: fragment d'ADN amplifié à partir du génome d'oiseaux
   <400> 10 
atcmattggg ttcactcacc ycymytygcc yttcaaagtg gcatctgtgg ankacbtyca 60
ccwyyycrrt gcgtwatcgc ggcatbytym asywtttwsm cgcctytggt tcymytthty 120
tyt 123
 dans laquelle Y est C ou T, M est A ou C, N est A, C, G ou T, K est G ou T, B est C, G ou T, R est A ou G, W est A ou T, S est C ou G, H est A, C ou T,
<210> 11
   <211> 20
   <212> ADN
   <213> Séquence artificielle
<220>
   <223> Description de la séquence artificielle: amorce
   PCR
<400> 11
   ccttgacact gatgcacttt 20
<210> 12
   <211> 20
   <212> ADN
   <213> Séquence artificielle
<220>
   <223> Description de la séquence artificielle: amorce
   PCR
<900> 12
   tgtggtaagc tatgtggacg 20
<210> 13
   <211> 408
   <212> ADN
   <213> Séquence artificielle
<220>
   <223> Description de la séquence artificielle: fragment d'ADN amplifié à partir du génome d'oiseaux
   <400> 13 
ymyykryact gatgcacttk gdycdcatty rgttrwkgyy myntccrchv hycyabnbwh 60
wvrtgytryy nrgtgaatgc tyghhggaca tadywywawm mawhwwmhyw yyhwsyryww 120
yyyhyrsrmh hhmnraaydm aaccarhrdw yttymdchrw thwdhwwhmm whywyvtwdy 180
rtmwwhyhhv hmhhhhmhwm hhbhawwhhh hnhnhynhwa yybchnnyhh hwawynysyy 240
whhynwbvhw hhnbybdmdm dwchdwmmnr wwhdmayddh dyvdhhyawm httattakag 300
raacwccagy acyarvvvhd vwhnhamhyn rrhrwhhmyy aywahyyhvh yntynmnamh 360
hnbnvyhyrd hyhryyaryb rmhyvhhhyr yycdcmtwsy wymmhnhh 408
 dans laquelle Y est C ou T, M est A ou C, N est A, C, G ou T, K est G ou T, B est C, G ou T, R est A ou G, W est A ou T, S est C ou G, D est A, G ou T, V est A, C ou G, H est A, C ou T,
<210> 14
   <211> 21
   <212> ADN
   <213> Séquence artificielle
<220>
   <223> Description de la séquence artificielle: amorce
   PCR
<400> 14
   catgtcaacg gacataccct a 21
<210> 15
   <211> 19
   <212> ADN
   <213> Séquence artificielle
<220>
   <223> Description de la séquence artificielle: amorce
   PCR
<400> 15
   taatgggtgg gtggagagg 19
<210> 16
   <211> 21
   <212> ADN
   <213> Séquence artificielle
<220>
   <223> Description de la séquence artificielle: amorce
   PCR
<400> 16
   atgctctcct ccgcagccct t 21
<210> 17
   <211> 20
   <212> ADN
   <213> Séquence artificielle
<220>
   <223> Description de la séquence artificielle: amorce
   PCR
<400> 17
   gattgtcgtt agattacgct 20
<210> 18
   <211> 19
   <212> ADN
   <213> Séquence artificielle
<220>
   <223> Description de la séquence artificielle: amorce
   PCR
<400> 18
   atgaatgttc taggaccat 19
<210> 19
   <211> 20
   <212> ADN
   <213> Séquence artificielle
<220>
   <223> Description de la séquence artificielle: amorce
   PCR
<400> 19
   tctgatttca cgtgaggagt 20
<210> 20
   <211> 180
   <212> ADN
   <213> Séquence artificielle
<220>
   <223> Description de la séquence artificielle: fragment d'ADN amplifié à partir du génome d'oiseaux
   <400> 20 hwbdnhmrmg rncathhhhn nyhvhbdnah nnnhchhcca vvrmhhhnnv rtgmayghyc 60 
yhrndvhhhh nhvnhvvmhv vmhvnvhnhn nhhhnhvhvv rwnmacmarr rcmmhdhnnr 120
vtgaatgcth nmhrracawr dmnyndvhah hmhtcyddnb yybhhtccac ccrmmcatwh 180
 dans laquelle Y est C ou T, M est A ou C, N est A, C, G ou T, B est C, G ou T, R est A ou G, W est A ou T, D est A, G ou T, V est A, C ou G, H est A, C ou T,
<210> 21
   <211> 191
   <212> ADN
   <213> Séquence artificielle
<220>
   <223> Description de la séquence artificielle: fragment d'ADN amplifié à partir du génome d'oiseaux
<400> 21 
wkgyymyntc crchvhycya bnbwhwvrtg ytryynrgtg aatgctyghh ggacatamhh 60
hmnraaydma achrhyhhvh mhhhnhnhnh hnnyhhhway wbvhwhwchd wmmnrwwhdm 120
ayddhdyvdh hyawmhttat takagraacw ccagyacyar vvvhdvwhnh amhynrrhrw 180
hhmyyaywah y 191
 dans laquelle Y est C ou T, M est A ou C, N est A, C, G ou T, B est C, G ou T, R est A ou G, W est A ou T, D est A, G ou T, V est A, C ou G, H est A, C ou T, K est G ou T,
<210> 22
   <211> 176
   <212> ADN
   <213> Séquence artificielle
<220>
   <223> Description de la séquence artificielle: fragment d'ADN amplifié à partir du génome d'oiseaux
   <400> 22 
rtgaayghyc yhrndvhhnh mvhnhnvmhv vnhnhhnhnh vvvswnmard scmnhnnttm 60
vtgaatgcth nmhrracawd mnyndvhahh mhtcyndnbh ybhhtccacc crmmcatwhm 120
tyatgravyy ncgtaycarr tggatttatt rrycgtacwc ctcacgtgaa atcagc 176
 dans laquelle Y est C ou T, M est A ou C, N est A, C, G ou T, B est C, G ou T, R est A ou G, W est A ou T, D est A, G ou T, V est A, C ou G, H est A, C ou T, S est C ou G,
<210> 23
   <211> 20
   <212> ADN
   <213> Séquence artificielle
<220>
   <223> Description de la séquence artificielle: amorce
   PCR
<400> 23
   atacgggcat taacctatat 20
<210> 24
   <211> 20
   <212> ADN
   <213> Séquence artificielle
<220>
   <223> Description de la séquence artificielle: amorce
   PCR
<400> 24
   cataaccaaa tgggttacat 20
<210> 25
   <211> 20
   <212> ADN
   <213> Séquence artificielle
<220>
   <223> Description de la séquence artificielle: amorce
   PCR
<400> 25
   acaaactcac cgcacaaata 20
<210> 26
   <211> 20
   <212> ADN
   <213> Séquence artificielle
<220>
   <223> Description de la séquence artificielle: amorce
   PCR
<400> 26
   ttaacaatat aaataatata 20
<210> 27
   <211> 20
   <212> ADN
   <213> Séquence artificielle
<220>
   <223> Description de la séquence artificielle: amorce
   PCR
<400> 27
   gcttgctgga cataaatacc 20
<210> 28
   <211> 20
   <212> ADN
   <213> Séquence artificielle
<220>
   <223> Description de la séquence artificielle: amorce
   PCR
<400> 28
   ataagttgtg gggcgtgttg 20
   <210> 29
   <211> 231
   <212> ADN
   <213> Séquence artificielle
<220>
   <223> Description de la séquence artificielle: fragment d'ADN amplifié à partir du génome d'oiseaux
   <400> 29 
kmawvngghm wtvachyata ttccmcwtyy ckymchahvb mchytvdatg maysmtshhr 60
krcmhvctmh ntthcvydhm ccatagabag ttccwaacha cwatcrrkvm mcmtaactat 120
gaatggttrc vghacataaa tctcactctc atgttctccc cccaacaagt cacctaacta 180
tgaatggtta caggacatac atttdactay catgttctaa ccsatttggt t 231
 dans laquelle Y est C ou T, M est A ou C, N est A, C, G ou T, B est C, G ou T, R est A ou G, W est A ou T, D est A, G ou T, V est A, C ou G, H est A, C ou T, S est C ou G, K est G ou T
<210> 30
   <211> 175
   <212> ADN
   <213> Séquence artificielle
<220>
   <223> Description de la séquence artificielle: fragment d'ADN amplifié à partir du génome d'oiseaux
   <400> 30 
tacawacymw cgyawwrrwd ccctyamayy ayahaamcgy ytatcgywta dtatatatac 60
attrywrydy ayymyaymat tattakagaa acyccmctac craaacmawc awhararrcw 120
wahadhwaha tkcmrcdyaw vbyhcytcas awrcmwwbrt krtwtatatt gttaa 175
 dans laquelle Y est C ou T, M est A ou C, B est C, G ou T, R est A ou G, W est A ou T, D est A, G ou T, V est A, C ou G, H est A, C ou T, S est C ou G, K est G ou T,
<210> 31
   <211> 216
   <212> ADN
   <213> Séquence artificielle
<220>
   <223> Description de la séquence artificielle: fragment d'ADN amplifié à partir du génome d'oiseaux
   <400> 31 
tgaatgcttg yyggacatww twymayywat twtcacttcc wctgayttty ytmacammac 60
yaggaywmaw ytyytwydty dktwywaayw ywtkwtttvw tyaywtyaaw macatytttt 120
amytawayta mcywtacawa cymwcmgyay wrrwtdtyam ayyaahaamc gyycgywtad 180
tatatataca tarywrytgy ryytmyaycm attatt 216
 dans laquelle Y est C ou T, M est A ou C, R est A ou G, W est A ou T, D est A, G ou T, V est A, C ou G, H est A, C ou T,
<210> 32
   <211> 25
   <212> ADN
   <213> Séquence artificielle
<220>
   <223> Description de la séquence artificielle: amorce
   PCR
<400> 32
   acgtgacyag cytcaggccc atacg 25
   dans laquelle Y est C ou T,
<210> 33
   <211> 15
   <212> ADN
   <213> Séquence artificielle
<220>
   <223> Description de la séquence artificielle: amorce
   PCR
<400> 33
   cttcaggccc atacg 15
<210> 34
   <211> 25
   <212> ADN
   <213> Séquence artificielle
<220>
   <223> Description de la séquence artificielle: amorce
   PCR
<400> 34
   ccctaaaccc ctcgccctcc tcaca 25
<210> 35
   <211> 15
   <212> ADN
   <213> Séquence artificielle
<220>
   <223> Description de la séquence artificielle: amorce
   PCR
<400> 35
   ctcgccctcc tcaca 15
<210> 36
   <211> 25
   <212> ADN
   <213> Séquence artificielle
<220>
   <223> Description de la séquence artificielle: amorce
   PCR
<400> 36
   gggccatcma ttgggttcac tcacc 25
   dans laquelle M est A ou C,
<210> 37
   <211> 15
   <212> ADN
   <213> Séquence artificielle
<220>
   <223> Description de la séquence artificielle: amorce
   PCR
<400> 37
   ttgggttcac tcacc 15
<210> 38
   <211> 25
   <212> ADN
   <213> Séquence artificielle
<220>
   <223> Description de la séquence artificielle: amorce
   PCR
<400> 38
   ggtrravstm ntccacagat gccac 25
   dans laquelle R est A ou G, V est A, C ou G, N est A, C, G ou T, S est C ou G, M est A ou C,
<210> 39
   <211> 15
   <212> ADN
   <213> Séquence artificielle
<220>
   <223> Description de la séquence artificielle: amorce
   PCR
<400> 39
   ttccacagat gccac 15
<210> 40
   <211> 25
   <212> ADN
   <213> Séquence artificielle
<220>
   <223> Description de la séquence artificielle: amorce
   PCR
<400> 40
   cmggaarara daarwrgaac cagag 25
   dans laquelle R est A ou G, D est A, G ou T, M est A ou C, W est A ou T,
<210> 41
   <211> 16
   <212> ADN
   <213> Séquence artificielle
<220>
   <223> Description de la séquence artificielle: amorce
   PCR
<400> 41
   ataaaaagaa ccagag 16
<210> 42
   <211> 25
   <212> ADN
   <213> Séquence artificielle
<220>
   <223> Description de la séquence artificielle: amorce
   PCR
<400> 42
   wvkcaymyyk ryactgatgc acttt 25
   dans laquelle Y est C ou T, K est G ou T, R est A ou G, M est A ou C, W est A ou T, V est A, C ou G, M est A ou C,
<210> 43
   <211> 15
   <212> ADN
   <213> Séquence artificielle
<220>
   <223> Description de la séquence artificielle: amorce
   PCR
<400> 43
   acactgatgc acttt 15
<210> 44
   <211> 25
   <212> ADN
   <213> Séquence artificielle
<220>
   <223> Description de la séquence artificielle: amorce
   PCR
<400> 44
   gcyttddndk krwrswatgt ggacg 25
   dans laquelle Y est C ou T, D est A, G ou T, B est C, G ou T, K est G ou T, W est A ou T, N est A, C, G ou T, R est A ou G, et S est C ou G,
<210> 45
   <211> 15
   <212> ADN
   <213> Séquence artificielle
<220>
   <223> Description de la séquence artificielle: amorce
   PCR
<400> 45
   taagctatgt ggacg 15
<210> 46
   <211> 25
   <212> ADN
   <213> Séquence artificielle
<220>
   <223> Description de la séquence artificielle: amorce
   PCR
<400> 46
   mtnmhmbdnh mvhvnncata cccta 25
   dans laquelle Y est C ou T, M est A ou C, H est A, C ou T, B est C, G ou T, D est A, G ou T, N est A, C, G ou T, W est A ou T, V est A, C ou G,
<210> 47
   <211> 15
   <212> ADN
   <213> Séquence artificielle
<220>
   <223> Description de la séquence artificielle: amorce
   PCR
<400> 47
   aacggacata cccta 15
<210> 48
   <211> 25
   <212> ADN
   <213> Séquence artificielle
<220>
   <223> Description de la séquence artificielle: amorce
   PCR
<400> 48
   catragnwaw kgkygggtgg agagg 25
   dans laquelle R est A ou G, N est A, C, G ou T, W est A ou T, K est G ou T, Y est C ou T,
<210> 49
   <211> 15
   <212> ADN
   <213> Séquence artificielle
<220>
   <223> Description de la séquence artificielle: amorce
   PCR
<400> 49
   gggtgggtgg agagg 15
<210> 50
   <211> 25
   <212> ADN
   <213> Séquence artificielle
<220>
   <223> Description de la séquence artificielle: amorce
   PCR
<400> 50
   gttawkgyym ynctccgcrg ccctt 25
   dans laquelle W est A ou T, K est G ou T, Y est C ou T, M est A ou C, Y est C ou T, N est A, C, G ou T, R est A ou G,
<210> 51
   <211> 15
   <212> ADN
   <213> Séquence artificielle
<220>
   <223> Description de la séquence artificielle: amorce
   PCR
<400> 51
   tcctccgcag ccctt 15
<210> 52
   <211> 25
   <212> ADN
   <213> Séquence artificielle
<220>
   <223> Description de la séquence artificielle: amorce
   PCR
<400> 52
   dtwatgatth tyrttadatt acgct 25
   dans laquelle D est A, G ou T, W est A ou T, H est A, C ou T, Y est C ou T, R est A ou G,
<210> 53
   <211> 15
   <212> ADN
   <213> Séquence artificielle
<220>
   <223> Description de la séquence artificielle: amorce
   PCR
<400> 53
   tcgttagatt acgct 15
<210> 54
   <211> 24
   <212> ADN
   <213> Séquence artificielle
<220>
   <223> Description de la séquence artificielle: amorce
   PCR
<400> 54
   aayghatgaa yghycagnda ccat 24
   dans laquelle Y est C ou T, H est A, C ou T, N est A, C, G ou T, D est A, G ou T,
<210> 55
   <211> 15
   <212> ADN
   <213> Séquence artificielle
<220>
   <223> Description de la séquence artificielle: amorce
   PCR
<400> 55
   atgttctagg accat 15
<210> 56
   <211> 25
   <212> ADN
   <213> Séquence artificielle
<220>
   <223> Description de la séquence artificielle: amorce
   PCR
<400> 56
   cgggttctga tttcacgtga ggagt 25
<210> 57
   <211> 15
   <212> ADN
   <213> Séquence artificielle
<220>
   <223> Description de la séquence artificielle: amorce
   PCR
<400> 57
   tttcacgtga ggagt 15
<210> 58
   <211> 25
   <212> ADN
   <213> Séquence artificielle
<220>
   <223> Description de la séquence artificielle: amorce
   PCR
<400> 58
   atatgkmvnc ggcattaacc tatat 25
   dans laquelle K est G ou T, M est A ou C, V est A, C ou G, N est A, C, G ou T,
<210> 59
   <211> 15
   <212> ADN
   <213> Séquence artificielle
<220>
   <223> Description de la séquence artificielle: amorce
   PCR
<400> 59
   ggcattaacc tatat 15
<210> 60
   <211> 25
   <212> ADN
   <213> Séquence artificielle
<220>
   <223> Description de la séquence artificielle: amorce
   PCR
<400> 60
   gcgagyataa ccaaatsggt tacat 25
   dans laquelle Y est C ou T, S est C ou G,
<210> 61
   <211> 15
   <212> ADN
   <213> Séquence artificielle
<220>
   <223> Description de la séquence artificielle: amorce
   PCR
<400> 61
   ccaaatgggt tacat 15
<210> 62
   <211> 25
   <212> ADN
   <213> Séquence artificielle
<220>
   <223> Description de la séquence artificielle: amorce
   PCR
   <400> 62
   tacatacawa cymwccgcac aaata 25
   dans laquelle W est A ou T, Y est C ou T, M est A ou C,
<210> 63
   <211> 15
   <212> ADN
   <213> Séquence artificielle
<220>
   <223> Description de la séquence artificielle: amorce
   PCR
   <400> 63
   ctcaccgcac aaata 15
<210> 64
   <211> 25
   <212> ADN
   <213> Séquence artificielle
<220>
   <223> Description de la séquence artificielle: amorce
   PCR
<400> 64
   tgtaartawc aatataaata atata 25
   dans laquelle R est A ou G, W est A ou T,
<210> 65
   <211> 15
   <212> ADN
   <213> Séquence artificielle
<220>
   <223> Description de la séquence artificielle: amorce
   PCR
<400> 65
   aatataaata atata 15
<210> 66
   <211> 25
   <212> ADN
   <213> Séquence artificielle
<220>
   <223> Description de la séquence artificielle: amorce
   PCR
<400> 66
   tgaatgcttg yyggacatwa atacc 25
   dans laquelle Y est C ou T, W est A ou T,
<210> 67
   <211> 15
   <212> ADN
   <213> Séquence artificielle
<220>
   <223> Description de la séquence artificielle: amorce
   PCR
<400> 67
   ctggacataa atacc 15
<210> 68
   <211> 25
   <212> ADN
   <213> Séquence artificielle
<220>
   <223> Description de la séquence artificielle: amorce
   PCR
<400> 68
   ctataatakg rtgtggggcg tgttg 25
   dans laquelle K est G ou T, R est A ou G
<210> 69
   <211> 15
   <212> ADN
   <213> Séquence artificielle
<220>
   <223> Description de la séquence artificielle: amorce
   PCR
<400> 69
   ttgtggggcg tgttg 15
<210> 70
   <211> 25
   <212> ADN
   <213> Séquence artificielle
<220>
   <223> Description de la séquence artificielle: amorce
   PCR
<400> 70
   ccttcmcagt gmbkkygscr grgtv 25
   dans laquelle M est A ou C, B est C, G ou T, K est G ou T, Y est C ou T, S est C ou G, R est A ou G, V est A, C ou G,
<210> 71
   <211> 15
   <212> ADN
   <213> Séquence artificielle
<220>
   <223> Description de la séquence artificielle: amorce
   PCR
<400> 71
   gccgtcgcca gagta 15
<210> 72
   <211> 20
   <212> ADN
   <213> Séquence artificielle
<220>
   <223> Description de la séquence artificielle: amorce
   PCR
<400> 72
   acagtgccgt cgccagagta 20
<210> 73
   <211> 25
   <212> ADN
   <213> Séquence artificielle
<220>
   <223> Description de la séquence artificielle: amorce
   PCR
<400> 73
   rrwwrtrgwk grrgkgrrww ttnry 25
   dans laquelle R est A ou G, W est A ou T, K est G ou T, N est A, C, G ou T, Y est C ou T,
<210> 74
   <211> 15
   <212> ADN
   <213> Séquence artificielle
<220>
   <223> Description de la séquence artificielle: amorce
   PCR
<400> 74
   gaagtgaaaa ttagc 15
<210> 75
   <211> 20
   <212> ADN
   <213> Séquence artificielle
<220>
   <223> Description de la séquence artificielle: amorce
   PCR
<400> 75
   tagaggaagt gaaaattagc 20
<210> 76
   <211> 231
   <212> ADN
   <213> Séquence artificielle
<220>
   <223> Description de la séquence artificielle: fragment d'ADN amplifié à partir du génome d'oiseaux
   <400> 76 
mcagtgmskk ygscrgrgtv cyaytcaaky kragcctgga ytacwcctgc gttrcgysct 60
atyctakwyy tchvgkrtym ctcratgaka cggttkgcgt rtatkkggwa tcayyttgac 120
actgatgcac tttggaycrc attyrgttaw kgytctyccr cmsyycynkw wartrgkgyt 180
atwtagtgaa tgctyghygg acatayytta wsynaawwyy cmcyycmwcy a 231
 dans laquelle M est A ou C, S est C ou G, K est G ou T, Y est C ou T, R est A ou G, V est A, C ou G, W est A ou T, N est A, C, T ou G,
<210> 77
   <211> 331
   <212> ADN
   <213> Séquence artificielle
<220>
   <223> Description de la séquence artificielle: fragment d'ADN amplifié à partir du génome d'oiseaux
   <400> 77 
ymyykrctga tgcacttkgd ycdcattyrg ttrwkgyymy ccrchvhycy abnbwhwvrt 60
gytryynrgt gaatgctygh hggacatady wywawmmawh wmhywyyhws yrywwyyyhy 120
rsrmhhhmra aydmaaccar hrdwyttymd chrwthdyrt mwwhyhvhmh hnhwayybch 180
nnyhdmdwch dwmmrwwhdm ayddhdyvdh hyawhttatt akagraacwc cagyacyarv 240
vvhdvwhnha mhynrrhrwh myyaywahyy hvhyntynmn amhhnbnvyh yrdhyhryya 300
rybrmhyvhh hyryycdcmt wsywymmhnh h 331
 dans laquelle Y est C ou T, M est A ou C, N est A, C, G ou T, K est G ou T, B est C, G ou T, R est A ou G, W est A ou T, S est C ou G, D est A, G ou T, V est A, C ou G, H est A, C ou T,
<210> 78
   <211> 328
   <212> ADN
   <213> Séquence artificielle
<220>
   <223> Description de la séquence artificielle: fragment d'ADN amplifié à partir du génome d'oiseaux
   <400> 78 
ymyykryact gatgcacttk gdycdcatty rgttrwkgyy myntccrchv hycyabnbwh 60
wvrtgytryy nrgtgaatgc tyghhggaca tamhhhmnra aydmaaccay dmaachrhyh 120
 
hvhmhhhnhn hynhhnnyrh hwayybchnn yhhwawymys yywhhynwbv hwhnbybdmd 180
mdwchdwmnr wwhdmayddh dyvdhhyawm httattakag raacwccagy acyarvvvhd 240
vwhnhamhyn rrhrwhhmyy aywahyyhvh yntynmnamh hnbnvyhyrd hyhryyaryb 300
rmhyvhhhyr yycdcmtwsy wymmhnhh 328
 dans laquelle Y est C ou T, M est A ou C, N est A, C, G ou T, K est G ou T, B est C, G ou T, R est A ou G, W est A ou T, S est C ou G, D est A, G ou T, V est A, C ou G, H est A, C ou T,
<210> 79
   <211> 288
   <212> ADN
   <213> Séquence artificielle
<220>
   <223> Description de la séquence artificielle: fragment d'ADN amplifié à partir du génome d'oiseaux
   <400> 79 
ymyykryact gatgcacttk gdycdcatty rgttrwkgyy myntccrchv hycyabnbwh 60
wvrtgytryy nrgtgaatgc tyghhggaca tamhhhmnra aydmaachrh yhhvhmhhhn 120
hnhynhhnny hhhwaywbvh whwchdwmmn rwwhdmaydd hdyvdhhyaw mhttattaka 180
graacwccag yacyarvvvh dvwhnhamhy nrrhrwhhmy yaywahyyhv hyntynmnmh 240
hnbnvyhyrd hyhryyaryb rmhyvhhhyr yycdcmtwsy wymmhnhh 288
 dans laquelle Y est C ou T, M est A ou C, N est A, C, G ou T, K est G ou T, B est C, G ou T, R est A ou G, W est A ou T, S est C ou G, D est A, G ou T, V est A, C ou G, H est A, C ou T,

## Revendications

1. Oligonucléotides **caractérisés en ce qu'**ils sont choisis parmi ceux :
(1) - présentant une identité de séquence d'au moins 80%, préférentiellement 90% et avantageusement 95% avec un oligonucléotide constitué d'une séquence de 15 à 25 nucléotides, notamment de 20 à 25 nucléotides, comprise dans la séquence SEQ ID N°32 suivante :
ACG TGA CYA GCY TCA GGC CCA TAC G
dans laquelle Y est C ou T,
- ou comprenant la séquence SEQ ID N°33 suivante : CTT CAG GCC CAT ACG
- ou constitués par la séquence SEQ ID N°1 suivante :
ACT AGC TTC AGG CCC ATA CG
ou ceux,
(2) - présentant une identité de séquence d'au moins 80%, préférentiellement 90% et avantageusement 95% avec un oligonucléotide constitué d'une séquence de 15 à 25 nucléotides, notamment de 20 à 25 nucléotides, comprise dans la séquence SEQ ID N°34 suivante :
CCC TAA ACC CCT CGC CCT CCT CAC A
- ou comprenant la séquence SEQ ID N°35 suivante :
CTC GCC CTC CTC ACA
- ou constitués par la séquence SEQ ID N°2 suivante :
AAC CCC TCG CCC TCC TCA CA
ou ceux,
(3) - présentant une identité de séquence d'au moins 80%, préférentiellement 90% et avantageusement 95% avec un oligonucléotide constitué d'une séquence de 15 à 25 nucléotides, notamment de 20 à 25 nucléotides, comprise dans la séquence SEQ ID N°36 suivante :
GGG CCA TCM ATT GGG TTC ACT CAC C
dans laquelle M est A ou C,
- ou comprenant la séquence SEQ ID N°37 suivante :
TTG GGT TCA CTC ACC
- ou constitués par la séquence SEQ ID N°3 suivante :
ATC AAT TGG GTT CAC TCA CC
ou ceux,
(4) - présentant une identité de séquence d'au moins 80%, préférentiellement 90% et avantageusement 95% avec un oligonucléotide constitué d'une séquence de 15 à 25 nucléotides, notamment de 20 à 25 nucléotides, comprise dans la séquence SEQ ID N°38 suivante :
GGT RRA VST MNT CCA CAG ATG CCA C
dans laquelle R est A ou G, V est A, C ou G, N est A, C, G ou T, S est C ou G, M est A ou C,
- ou comprenant la séquence SEQ ID N°39 suivante : TTC CAC AGA TGC CAC
- ou constitués par la séquence SEQ ID N°4 suivante :
AAG TAT TCC ACA GAT GCC AC
ou ceux,
(5) - présentant une identité de séquence d'au moins 80%, préférentiellement 90% et avantageusement 95% avec un oligonucléotide constitué d'une séquence de 15 à 25 nucléotides, notamment de 20 à 25 nucléotides, comprise dans la séquence SEQ ID N°40 suivante :
CMG GAA RAR ADA ARW RGA ACC AGA G
dans laquelle R est A ou G, D est A, G ou T, M est A ou C, W est A ou T,
- ou comprenant la séquence SEQ ID N°4 suivante :
A TAA AAA GAA CCA GAG
- ou constitués par la séquence SEQ ID N°5 suivante :
AAA AAT AAA AGG AAC CAG AG
ou ceux,
(6) - présentant une identité de séquence d'au moins 80%, préférentiellement 90% et avantageusement 95% avec un oligonucléotide constitué d'une séquence de 15 à 25 nucléotides, notamment de 20 à 25 nucléotides, comprise dans la séquence SEQ ID N°42 suivante :
WVK CAY MYY KRY ACT GAT GCA CTT T
dans laquelle Y est C ou T, K est G ou T, R est A ou G, M est A ou C, W est A ou T, V est A, C ou G, M est A ou C,
- ou comprenant la séquence SEQ ID N°43 suivante :
ACA CTG ATG CAC TTT
- ou constitués par la séquence SEQ ID N°11 suivante :
CCT TGA CAC TGA TGC ACT TT
ou ceux,
(7) - présentant une identité de séquence d'au moins 80%, préférentiellement 90% et avantageusement 95% avec un oligonucléotide constitué d'une séquence de 15 à 25 nucléotides, notamment de 20 à 25 nucléotides, comprise dans la séquence SEQ ID N°44 suivante :
GCY TTD DND KKR WRS WAT GTG GAC G
dans laquelle Y est C ou T, D est A, G ou T, B est C, G ou T, K est G ou T, W est A ou T, N est A, C, G ou T, R est A ou G, et S est C ou G,
- ou comprenant la séquence SEQ ID N°45 suivante :
TAA GCT ATG TGG'ACG
- ou constitués par la séquence SEQ ID N°12 suivante :
TGT GGT AAG CTA TGT GGA CG
ou ceux,
(8) - présentant une identité de séquence d'au moins 80%, préférentiellement 90% et avantageusement 95% avec un oligonucléotide constitué d'une séquence de 15 à 25 nucléotides, notamment de 20 à 25 nucléotides, comprise dans la séquence SEQ ID N°46 suivante :
MTN MHM BDN HMV HVN NCA TAC CCT A
dans laquelle Y est C ou T, M est A ou C, H est A, C ou T, B est C, G ou T, D est A, G ou T, N est A, C, G ou T, W est A ou T, V est A, C ou G,
- ou comprenant la séquence SEQ ID N°47 suivante :
AAC GGA CAT ACC CTA
- ou constitués par la séquence SEQ ID N°14 suivante :
CAT GTC AAC GGA CAT ACC CTA
ou ceux,
(9) - présentant une identité de séquence d'au moins 80%, préférentiellement 90% et avantageusement 95% avec un oligonucléotide constitué d'une séquence de 15 à 25 nucléotides, notamment de 20 à 25 nucléotides, comprise dans la séquence SEQ ID N°48 suivante :
CAT RAG NWA WKG KYG GGT GGA GAG G
dans laquelle R est A ou G, N est A, C, G ou T, W est A ou T, K est G ou T, Y est CouT,
- ou comprenant la séquence SEQ ID N°49 suivante :
GGG TGG GTG GAG AGG
- ou constitués par la séquence SEQ ID N°15 suivante :
TAA TGG GTG GGT GGA GAG G
ou ceux,
(10) - présentant une identité de séquence d'au moins 80%, préférentiellement 90% et avantageusement 95% avec un oligonucléotide constitué d'une séquence de 15 à 25 nucléotides, notamment de 20 à 25 nucléotides, comprise dans la séquence SEQ ID N°50 suivante :
GTT AWK GYY MYN CTC CGC RGC CCT T
dans laquelle W est A ou T, K est G ou T, Y est C ou T, M est A ou C, Y est C ou T, N est A, C, G ou T, R est A ou G,
- ou comprenant la séquence SEQ ID N°51 suivante :
TCC TCC GCA GCC CTT
- ou constitués par la séquence SEQ ID N°16 suivante :
ATG CTC TCC TCC GCA GCC CTT
ou ceux,
(11) - présentant une identité de séquence d'au moins 80%, préférentiellement 90% et avantageusement 95% avec un oligonucléotide constitué d'une séquence de 15 à 25 nucléotides, notamment de 20 à 25 nucléotides, comprise dans la séquence SEQ ID N°52 suivante :
DTW ATG ATT HTY RTT ADA TTA CGC T
dans laquelle D est A, G ou T, W est A ou T, H est A, C ou T, Y est C ou T, R est A ou G,
- ou comprenant la séquence SEQ ID N°53 suivante :
TCG TTA GAT TAC GCT
- ou constitués par la séquence SEQ ID N°17 suivante :
GAT TGT CGT TAG ATT ACG CT
ou ceux,
(12) - présentant une identité de séquence d'au moins 80%, préférentiellement 90% et avantageusement 95% avec un oligonucléotide constitué d'une séquence de 15 à 25 nucléotides, notamment de 20 à 25 nucléotides, comprise dans la séquence SEQ ID N°54 suivante :
AAY GHA TGA AYG HYC AGN DAC CAT
dans laquelle Y est C ou T, H est A, C ou T, N est A, C, G ou T, D est A, G ou T,
- ou comprenant la séquence SEQ ID N°55 suivante :
ATG TTC TAG GAC CAT
- ou constitués par la séquence SEQ ID N°18 suivante :
ATG AAT GTT CTA GGA CCA T
ou ceux,
(13) - présentant une identité de séquence d'au moins 80%, préférentiellement 90% et avantageusement 95% avec un oligonucléotide constitué d'une séquence de 15 à 25 nucléotides, notamment de 20 à 25 nucléotides, comprise dans la séquence SEQ ID N°56 suivante :
CGG GTT CTG ATT TCA CGT GAG GAG T
- ou comprenant la séquence SEQ ID N°57 suivante :
TTT CAC GTG AGG AGT
- ou constitués par la séquence SEQ ID N°19 suivante :
TCT GAT TTC ACG TGA GGA GT.
(14) - présentant une identité de séquence d'au moins 80%, préférentiellement 90% et avantageusement 95% avec un oligonucléotide constitué d'une séquence de 15 à 25 nucléotides, notamment de 20 à 25 nucléotides, comprise dans la séquence SEQ ID N°58 suivante :
ATA TGK MVN CGG CAT TAA CCT ATA T
dans laquelle K est G ou T, M est A ou C, V est A, C ou G, N est A, C, G ou T,
- ou comprenant la séquence SEQ ID N°59 suivante :
GGC ATT AAC CTA TAT
- ou constitués par la séquence SEQ ID N°23 suivante :
ATA CGG GCA TTA ACC TAT AT
ou ceux,
(15) - présentant une identité de séquence d'au moins 80%, préférentiellement 90% et avantageusement 95% avec un oligonucléotide constitué d'une séquence de 15 à 25 nucléotides, notamment de 20 à 25 nucléotides, comprise dans la séquence SEQ ID N°60 suivante :
GCG AGY ATA ACC AAA TSG GTT ACA T
dans laquelle Y est C ou T, S est C ou G,
- ou comprenant la séquence SEQ ID N°61 suivante :
CCA AAT GGG TTA CAT
- ou constitués par la séquence SEQ ID N°24 suivante :
CAT AAC CAA ATG GGT TAC AT
ou ceux,
(16) - présentant une identité de séquence d'au moins 80%, préférentiellement 90% et avantageusement 95% avec un oligonucléotide constitué d'une séquence de 15 à 25 nucléotides, notamment de 20 à 25 nucléotides, comprise dans la séquence SEQ ID N°62 suivante :
TAC ATA CAW ACY MWC CGC ACA AAT A
dans laquelle W est A ou T, Y est C ou T, M est A ou C,
- ou comprenant la séquence SEQ ID N°63 suivante :
CTC ACC GCA CAA ATA
- ou constitués par la séquence SEQ ID N°25 suivante :
ACA AAC TCA CCG CAC AAA TA
ou ceux,
(17) - présentant une identité de séquence d'au moins 80%, préférentiellement 90% et avantageusement 95% avec un oligonucléotide constitué d'une séquence de 15 à 25 nucléotides, notamment de 20 à 25 nucléotides, comprise dans la séquence SEQ ID N°64 suivante :
TGT AAR TAW CAA TAT AAA TAA TAT A
dans laquelle R est A ou G, W est A ou T,
- ou comprenant la séquence SEQ ID N°65 suivante :
AAT ATA AAT AAT ATA
- ou constitués par la séquence SEQ ID N°26 suivante :
TTA ACA ATA TAA ATA ATA TA
ou ceux,
(18) - présentant une identité de séquence d'au moins 80%, préférentiellement 90% et avantageusement 95%, avec un oligonucléotide constitué d'une séquence de 15 à 25 nucléotides, notamment de 20 à 25 nucléotides, comprise dans la séquence SEQ ID N° 70 suivante :
CCT TCM CAG TGM BKK YGS CRG RGT V
dans laquelle M est A ou C, B est C, G ou T, K est G ou T, Y est C ou T, S est C ou G, R est A ou G, V est A, C ou G,
- ou comprenant la séquence SED ID N°71 suivante :
GCC GTC GCC AGA GTA
- ou constitués par la séquence SED ID N°72 suivante :
ACA GTG CCG TCG CCA GAG TA
ou ceux,
(19) - présentant une identité de séquence d'au moins 80%, préférentiellement 90% et avantageusement 95%, avec un oligonucléotide constitué d'une séquence de 15 à 25 nucléotides, notamment de 20 à 25 nucléotides, comprise dans la séquence SEQ ID N° 73 suivante :
RRW WRT RGW KGR RGK GRR WWT TNR Y
dans laquelle R est A ou G, W est A ou T, K est G ou T, N est A, C, G ou T, Y est C ou T,
- ou comprenant la séquence SED ID N°74 suivante
GAA GTG AAA ATT AGC
- ou constitués par la séquence SED ID N°75 suivante
TAG AGG AAG TGA AAA TTA GC
ou ceux,
(20) - présentant une identité de séquence d'au moins 80%, préférentiellement 90% et avantageusement 95% avec un oligonucléotide constitué d'une séquence de 15 à 25 nucléotides, notamment de 20 à 25 nucléotides, comprise dans la séquence SEQ ID N°66 suivante :
TGA ATG CTT GYY GGA CAT WAA TAC C
dans laquelle Y est C ou T, W est A ou T
- ou comprenant la séquence SEQ ID N°67 suivante :
CTG GAC ATA AAT ACC
- ou constitués par la séquence SEQ ID N°27 suivante :
GCT TGC TGG ACA TAA ATA CC
ou ceux,
(21) - présentant une identité de séquence d'au moins 80%, préférentiellement 90% et avantageusement 95% avec un oligonucléotide constitué d'une séquence de 15 à 25 nucléotides, notamment de 20 à 25 nucléotides, comprise dans la séquence SEQ ID N°68 suivante :
CTA TAA TAK GRT GTG GGG CGT GTT G
dans laquelle K représente G ou T, R est A ou G,
- ou comprenant la séquence SEQ ID N°69 suivante :
TTG TGG GGC GTG TTG
- ou constitués par la séquence SEQ ID N°28 suivante :
ATA AGT TGT GGG GCG TGT TG

2. Couples d'amorces **caractérisés en ce qu'**ils sont constitués :
- par l'un quelconque des oligonucléotides SEQ ID N°1, SEQ ID N°32, SEQ ID N°33, SEQ ID N°2, SEQ ID N°34, SEQ ID N°35, SEQ ID N°3, SEQ ID N°36, SEQ ID N°37 et l'un quelconque des oligonucléotides SEQ ID N°4, SEQ ID N°38, SEQ ID N°39, SEQ ID N°5, SEQ ID N°40, SEQ ID N°41 selon la revendication 1 ou,
- par l'un quelconque des oligonucléotides SEQ ID N°11, SEQ ID N°42, SEQ ID N°43 et l'un quelconque des oligonucléotides SEQ ID N°12, SEQ ID N°44, SEQ ID N°45 selon la revendication 1 ou,
- par l'un quelconque des oligonucléotides SEQ ID N°14, SEQ ID N°46, SEQ ID N°47, et l'un quelconque des oligonucléotides SEQ ID N°15, SEQ ID N°48, SEQ ID N°49 selon la revendication 1 ou,
- par l'un quelconque des oligonucléotides SEQ ID N°16, SEQ ID N°50, SEQ ID N°51, et l'un quelconque des oligonucléotides SEQ ID N°17, SEQ ID N°52, SEQ ID N°53 selon la revendication 1 ou,
- par l'un quelconque des oligonucléotides SEQ ID N°18, SEQ ID N°54, SEQ ID N°55, et l'un quelconque des oligonucléotides SEQ ID N°19, SEQ ID N°56, SEQ ID N°57 selon la revendication 1,
- par l'un quelconque des oligonucléotides SEQ ID N°23, SEQ ID N°58, SEQ ID N°59, et l'un quelconque des oligonucléotides SEQ ID N°24, SEQ ID N°60, SEQ ID N°61 selon la revendication 1 ou,
- par l'un quelconque des oligonucléotides SEQ ID N°25, SEQ ID N°62, SEQ ID N°63, et l'un quelconque des oligonucléotides SEQ ID N°26, SEQ ID N°64, SEQ ID N°65 selon la revendication 1 ou,
- par l'un quelconque des oligonucléotides SEQ ID N°70, SEQ ID N°71, SEQ ID N°72, et l'un quelconque des oligonucléotides SEQ ID N°73, SEQ ID N°74, SEQ ID N°75 selon la revendication 1 ou,
- par l'un quelconque des oligonucléotides SEQ ID N°27, SEQ ID N°66, SEQ ID N°67, et l'un quelconque des oligonucléotides SEQ ID N°28, SEQ ID N°68, SEQ ID N°69 selon la revendication 1,
et avantageusement constitués par le couple d'oligonucléotides choisis parmi les couples suivants :
- SEQ ID N° 1 et SEQ ID N° 5,
- SEQ ID N° 2 et SEQ ID N° 5,
- SEQ ID N° 1 et SEQ ID N° 4,
- SEQ ID N° 2 et SEQ ID N° 4,
- SEQ ID N° 3 et SEQ ID N° 5,
- SEQ ID N° 11 et SEQ ID N° 12,
- SEQ ID N° 14 et SEQ ID N° 15,
- SEQ ID N° 16 et SEQ ID N° 17,
- SEQ ID N° 18 et SEQ ID N° 19,
- SEQ ID N°23 et SEQ ID N°24,
- SEQ ID N°25 et SEQ ID N°26,
- SEQ ID N°72 et SEQ ID N°75,
- SEQ ID N°27 et SEQ ID N°28.

3. Procédé de détection de la présence de matières biologiques provenant d'oiseaux, notamment d'ansériformes, de galliformes ou de struthioniformes dans un échantillon de matière organique, **caractérisé en ce que** l'on détermine la présence d'ADN provenant d'oiseaux dans ladite matière organique par amplification d'une séquence d'ADN spécifique du génome des oiseaux et contenue dans l'ADN extrait dudit échantillon, à savoir une séquence présente dans les génomes des oiseaux mais absente dans les génomes des autres genres animaliers, et notamment des autres espèces animales,
ladite séquence étant une séquence d'ADN mitochondrial spécifique du génome des oiseaux amplifiée à l'aide d'oligonucléotides choisis parmi les oligonucléotides tels que définis dans la revendication 1.

4. Procédé selon la revendication 3, comprenant également l'identification du genre, notamment de l'espèce d'oiseau, notamment d'ansériforme, de galliforme ou de struthioniforme présente dans ledit échantillon, **caractérisé en ce qu'**on l'on compare la séquence d'ADN spécifique du génome des oiseaux ainsi amplifiée telle que définie dans la revendication 3 avec d'autres séquences d'ADN du génome des oiseaux, ladite séquence d'ADN spécifique du génome des oiseaux ainsi amplifiée présentant au moins 50% d'identité, notamment 60% d'identité avec les autres susdites séquences d'ADN du génome des oiseaux.

5. Procédé selon la revendication 3 ou la revendication 4, **caractérisé en ce qu'**il permet de détecter et/ou d'identifier la présence d'ansériformes, notamment d'anatidés choisis dans le groupe constitué par les canards tels que le canard pilet *(Anas acuta*), le canard mandarin (*Aix galericulata*), le canard souchet *(Anas clypeata*), la sarcelle d'été (*Anas querquedula*), l'eider à duvet (*Somateria mollissima*), les oies telles que l'oie naine *(Anser erythropus*), l'oie des neiges *(Anser caerulescens*), la bernache du Canada (*Branta canadensis*), et les cygnes tels que le cygne sauvage *(Cygnus cygnus*), le cygne noir (*Cygnus atratus*), le cygne commun *(Cygnus color*) et notamment les espèces domestiques de canards telles que le canard colvert *(Anas platyrhynchos*), le canard de barbarie (*Cairina moschata*) ou l'espèce domestique d'oie telle que l'oie commune *(Anser anser).*

6. Procédé selon l'une quelconque des revendications 3 à 5, **caractérisé en ce que** l'amplification de ladite séquence d'ADN spécifique du génome des oiseaux, notamment d'ansériformes, de galliformes ou de struthioniformes est effectuée par la méthode d'amplification en chaîne par polymérase (PCR), comprenant une répétition du cycle des étapes suivantes :
- chauffage de l'ADN extrait de l'échantillon de matière organique, de façon à séparer l'ADN en deux brins monocaténaires,
- hybridation d'amorces oligonucléotidiques selon les revendications 1 ou 2 aux brins d'ADN monocaténaires à une température adéquate, et
- élongation des amorces oligonucléotidiques par une polymérase à une température adéquate, pour obtenir une séquence d'ADN ou un fragment d'ADN amplifié(e) spécifique du génome des oiseaux, notamment d'ansériformes, de galliformes ou de struthioniformes.

7. Procédé selon l'une des revendications 3 à 6, **caractérisé en ce que** le fragment d'ADN amplifié obtenu est identifié :
- par séquençage dudit fragment amplifié,
- par électrophorèse sur gel des fragments obtenus après digestion à l'aide d'enzymes de restriction dudit fragment amplifié,
- directement, par simple visualisation de la présence dudit fragment amplifié par électrophorèse sur gel.

8. Procédé selon l'une quelconque des revendications 3 à 7, **caractérisé en ce que** l'ADN extrait de l'échantillon de matière organique est :
- de l'ADN non dégradé provenant notamment d'un échantillon frais ou,
- de l'ADN dégradé, provenant notamment d'un échantillon transformé, notamment cuit, lyophilisé, séché, saumuré, appertisé ou pasteurisé.

9. Fragment d'ADN tel qu'amplifié à l'issue du procédé selon l'une des revendications 3 à 8, **caractérisé en ce qu'**il présente une identité de séquence d'au moins 80%, préférentiellement 90% et avantageusement 95% avec :
- la SEQ ID N°6 suivante : 
ACYAGCTTCA GGCCCATACG TTCCCCCTAA ACCCCTCGCC CTCCTCACAT TTTTGCGCCT
CTGGTTCCTC GGTCAGGGCC ATCMATTGGG TTCACTCACC YCYMYTYGCC YTTCAAAGTG
GCATCTGTGG ANKACBTYCA CCWYYYCRRT GCGTWATCGC GGCATBYTYM ASYWTTTWSM
CGCCTYTGGT TCYMYTTHTY TYT
 dans laquelle Y est C ou T, M est A ou C, K est G ou T, B est C, G ou T, R est A ou G, W est A ou T, S est C ou G, H est A, C ou T, N est A, C, G ou T,
ladite séquence SEQ ID N° 6 comprenant 203 paires de bases,
- ou la SEQ ID N°7 suivante : 
ACYAGCTTCA GGCCCATACG TTCCCCCTAA ACCCCTCGCC CTCCTCACAT TTTTGCGCCT
CTGGTTCCTC GGTCAGGGCC ATCMATTGGG TTCACTCACC YCYMYTYGCC YTTCAAAGTG
GCATCTGTGG ANKACBT
 dans laquelle Y est C ou T, M est A ou C, N est A, C, G ou T, K est G ou T, B est C, G ou T,
ladite séquence SEQ ID N°7 comprenant 137 paires de bases,
- ou la SEQ ID N°8 suivante : 
AACCCCTCGC CCTCCTCACA TTTTTGCGCC TCTGGTTCCT CGGTCAGGGC CATCMATTGG
GTTCACTCAC CYCYMYTYGC CYTTCAAAGT GGCATCTGTG GANKACBTYC ACCWYYYCRR
TGCGTWATCG CGGCATBYTY MASYWTTTWS MCGCCTYTGG TTCYMYTTHT YTYT
 dans laquelle Y est C ou T, M est A ou C, N est A, C, G ou T, K est G ou T, B est C, G ou T, R est A ou G, W est A ou T, S est C ou G, H est A, C ou T,
ladite séquence SEQ ID N°8 comprenant 174 paires de bases,
- ou la SEQ ID N°9 suivante : 
AACCCCTCGC CCTCCTCACA TTTTTGCGCC TCTGGTTCCT CGGTCAGGGC CATCMATTGG
GTTCACTCAC CYCYMYTYGC CYTTCAAAGT GGCATCTGTG GANKACBT
 dans laquelle Y est C ou T, M est A ou C, N est A, C, G ou T, K est G ou T, B est C, G ou T,
ladite séquence SEQ ID N°9 comprenant 108 paires de bases,
- ou la SEQ ID N°10 suivante : 
ATCMATTGGG TTCACTCACC YCYMYTYGCC YTTCAAAGTG GCATCTGTGG ANKACBTYCA
CCWYYYCRRT GCGTWATCGC GGCATBYTYM ASYWTTTWSM CGCCTYTGGT TCYMYTTHTY
TYT
 dans laquelle Y est C ou T, M est A ou C, N est A, C, G ou T, K est G ou T, B est C, G ou T, R est A ou G, W est A ou T, S est C ou G, H est A, C ou T,
ladite séquence SEQ ID N° 10 comprenant 123 paires de bases,
- ou la SEQ ID N°13 suivante : 
YMYYKRYACT GATGCACTTK GDYCDCATTY RGTTRWKGYY MYNTCCRCHV HYCYABNBWH
WVRTGYTRYY NRGTGAATGC TYGHHGGACA TADYWYWAWM MAWHWWMHYW YYHWSYRYWW
YYYHYRSRMH HHMNRAAYDM AACCARHRDW YTTYMDCHRW THWDHWWHMM WHYWYVTWDY
RTMWWHYHHV HMHHHHMHWM HHBHAWWHHH HNHNHYNHWA YYBCHNNYHH HWAWYNYSYY
WHHYNWBVHW HHNBYBDMDM DWCHDWMMNR WWHDMAYDDH DYVDHHYAWM HTTATTAKAG
RAACWCCAGY ACYARVVVHD VWHNHAMHYN RRHRWHHMYY AYWAHYYHVH YNTYNMNAMH
HNBNVYHYRD HYHRYYARYB RMHYVHHHYR YYCDCMTWSY WYMMHNHH
 dans laquelle Y est C ou T, M est A ou C, N est A, C, G ou T, K est G ou T, B est C, G ou T, R est A ou G, W est A ou T, S est C ou G, D est A, G ou T, V est A, C ou G, H est A, C ou T,
ladite séquence SEQ ID N°13 comprenant 408 paires de bases,
- ou la séquence SEQ ID NO : 77 suivante : YMYYKRCTGA  TGCACTTKGD  YCDCATTYRG  TTRWKGYYMY  CCRCHVHYCY  ABNBWHWVRT 
GYTRYYNRGT GAATGCTYGH HGGACATADY WYWAWMMAWH WMHYWYYHWS YRYWWYYYHY
RSRMHHHMRA AYDMAACCAR HRDWYTTYMD CHRWTHDYRT MWWHYHVHMH HNHWAYYBCH
NNYHDMDWCH DWMMRWWHDM AYDDHDYVDH HYAWHTTATT AKAGRAACWC CAGYACYARV
VVHDVWHNHA MHYNRRHRWH MYYAYWAHYY HVHYNTYNMN AMHHNBNVYH YRDHYHRYYA
RYBRMHYVHH HYRYYCDCMT WSYWYMMHNH H
 dans lequel Y est C ou T, M est A ou C, N est A, C, G ou T, K est G ou T, B est C, G ou T, R est A ou G, W est A ou T, S est C ou G, D est A, G ou T, V est A, C ou G, H est A, C ou T,
ladite séquence SEQ ID N°77 comprenant 331 paires de bases,
- ou la séquence SEQ ID NO : 78 suivante : 
YMYYKRYACT GATGCACTTK GDYCDCATTY RGTTRWKGYY MYNTCCRCHV HYCYABNBWH
WVRTGYTRYY NRGTGAATGC TYGHHGGACA TAMHHHMNRA AYDMAACCAY DMAACHRHYH
HVHMHHHNHN HYNHHNNYRH HWAYYBCHNN YHHWAWYMYS YYWHHYNWBV HWHNBYBDMD
MDWCHDWMNR WWHDMAYDDH DYVDHHYAWM HTTATTAKAG RAACWCCAGY ACYARVVVHD
VWHNHAMHYN RRHRWHHMYY AYWAHYYHVH YNTYNMNAMH HNBNVYHYRD HYHRYYARYB
RMHYVHHHYR YYCDCMTWSY WYMMHNHH
 dans lequel Y est C ou T, M est A ou C, N est A, C, G ou T, K est G ou T, B est C, G ou T, R est A ou G, W est A ou T, S est C ou G, D est A, G ou T, V est A, C ou G, H est A, C ou T,
ladite séquence SEQ ID N°78 comprenant 328 paires de bases,
- ou la séquence SEQ ID NO : 79 suivante : 
YMYYKRYACT GATGCACTTK GDYCDCATTY RGTTRWKGYY MYNTCCRCHV HYCYABNBWH
WVRTGYTRYY NRGTGAATGC TYGHHGGACA TAMHHHMNRA AYDMAACHRH YHHVHMHHHN
HNHYNHHNNY HHHWAYWBVH WHWCHDWMMN RWWHDMAYDD HDYVDHHYAW MHTTATTAKA
GRAACWCCAG YACYARVVVH DVWHNHAMHY NRRHRWHHMY YAYWAHYYHV HYNTYNMNMH
HNBNVYHYRD HYHRYYARYB RMHYVHHHYR YYCDCMTWSY WYMMHNHH
 dans lequel Y est C ou T, M est A ou C, N est A, C, G ou T, K est G ou T, B est C, G ou T, R est A ou G, W est A ou T, S est C ou G, D est A, G ou T, V est A, C ou G, H est A, C ou T,
ladite séquence SEQ ID N°79 comprenant 288 paires de bases,
- ou la SEQ ID N°20 suivante : 
HWBDNHMRMG RNCATHHHHN NYHVHBDNAH NNNHCHHCCA VVRMHHHNNV RTGMAYGHYC
YHRNDVHHHH NHVNHVVMHV VMHVNVHNHN NHHHNHVHVV RWNMACMARR RCMMHDHNNR
VTGAATGCTH NMHRRACAWR DMNYNDVHAH HMHTCYDDNB YYBHHTCCAC CCRMMCATWH
 dans laquelle Y est C ou T, M est A ou C, N est A, C, G ou T, B est C, G ou T, R est A ou G, W est A ou T, D est A, G ou T, V est A, C ou G, H est A, C ou T,
ladite séquence SEQ ID N°20 comprenant 180 paires de bases,
- ou la SEQ ID N°21 suivante : 
WKGYYMYNTC CRCHVHYCYA BNBWHWVRTG YTRYYNRGTG AATGCTYGHH GGACATAMHH
HMNRAAYDMA ACHRHYHHVH MHHHNHNHNH HNNYHHHWAY WBVHWHWCHD WMMNRWWHDM
AYDDHDYVDH HYAWMHTTAT TAKAGRAACW CCAGYACYAR VVVHDVWHNH AMHYNRRHRW
HHMYYAYWAH Y
 dans laquelle Y est C ou T, M est A ou C, N est A, C, G ou T, B est C, G ou T, R est A ou G, W est A ou T, D est A, G ou T, V est A, C ou G, H est A, C ou T, K est GouT,
ladite séquence SEQ ID N°21 comprenant 191 paires de bases,
- ou la SEQ ID N°22 suivante : 
RTGAAYGHYC YHRNDVHHNH MVHNHNVMHV VNHNHHNHNH VVVSWNMARD SCMNHNNTTM
VTGAATGCTH NMHRRACAWD MNYNDVHAHH MHTCYNDNBH YBHHTCCACC CRMMCATWHM
TYATGRAVYY NCGTAYCARR TGGATTTATT RRYCGTACWC CTCACGTGAA ATCAGC
 dans laquelle Y est C ou T, M est A ou C, N est A, C, G ou T, B est C, G ou T, R est A ou G, W est A ou T, D est A, G ou T, V est A, C ou G, H est A, C ou T, S est CouG,
ladite séquence SEQ ID N°22 comprenant 176 paires de bases,
- ou la SEQ ID N°29 suivante : 
KMAWVNGGHM WTVACHYATA TTCCMCWTYY CKYMCHAHVB MCHYTVDATG MAYSMTSHHRK
RCMHVCTMHN TTHCVYDHMC CATAGABAGT TCCWAACHAC WATCRRKVMM CMTAACTATG
AATGGTTRCVG HACATAAATC TCACTCTCAT GTTCTCCCCC CAACAAGTCA CCTAACTATG
AATGGTTACAG GACATACATT TDACTAYCAT GTTCTAACCS ATTTGGTT
 dans laquelle Y est C ou T, M est A ou C, N est A, C, G ou T, B est C, G ou T, R est A ou G, W est A ou T, D est A, G ou T, V est A, C ou G, H est A, C ou T, S est C ou G, K est G ou T,
ladite séquence SEQ ID N°29 comprenant 231 paires de bases,
- ou la SEQ ID N°30 suivante : 
TACAWACYMW CGYAWWRRWD CCCTYAMAYY AYAHAAMCGY YTATCGYWTA DTATATATAC
ATTRYWRYDY AYYMYAYMAT TATTAKAGAA ACYCCMCTAC CRAAACMAWC AWHARARRCW
WAHADHWAHA TKCMRCDYAW VBYHCYTCAS AWRCMWWBRT KRTWTATATT GTTAA
 dans laquelle Y est C ou T, M est A ou C, B est C, G ou T, R est A ou G, W est A ou T, D est A, G ou T, V est A, C ou G, H est A, C ou T, S est C ou G, K est G ou T,
ladite séquence SEQ ID N°30 comprenant 175 paires de bases,
- ou la SEQ ID N°76 suivante : 
MCAGTGMSKK YGSCRGRGTV CYAYTCAAKY KRAGCCTGGA YTACWCCTGC GTTRCGYSCT
ATYCTAKWYY TCHVGKRTYM CTCRATGAKA CGGTTKGCGT RTATKKGGWA TCAYYTTGAC
ACTGATGCAC TTTGGAYCRC ATTYRGTTAW KGYTCTYCCR CMSYYCYNKW WARTRGKGYT
ATWTAGTGAA TGCTYGHYGG ACATAYYTTA WSYNAAWWYY CMCYYCMWCY A
 dans laquelle M est A ou C, S est C ou G, K est G ou T, Y est C ou T, R est A ou G, V est A, C ou G, W est A ou T, N est A, C, T ou G,
ladite séquence SEQ ID N°76 comprenant 231 paires de bases,
- ou la SEQ ID N°31 suivante : 
TGAATGCTTG YYGGACATWW TWYMAYYWAT TWTCACTTCC WCTGAYTTTY YTMACAMMAC
YAGGAYWMAW YTYYTWYDTY DKTWYWAAYW YWTKWTTTVW TYAYWTYAAW MACATYTTTT
AMYTAWAYTA MCYWTACAWA CYMWCMGYAY WRRWTDTYAM AYYAAHAAMC GYYCGYWTAD
TATATATACA TARYWRYTGY RYYTMYAYCM ATTATT
 dans laquelle Y est C ou T, M est A ou C, R est A ou G, W est A ou T, D est A, G ou T, V est A, C ou G, H est A, C ou T,
ladite séquence SEQ ID N°31 comprenant 216 paires de bases.

10. Utilisation de séquences nucléotidiques choisies parmi les oligonucléotides tels que définis dans l'une quelconque des revendications 1 ou 2, ou de fragments d'ADN selon la revendication 9, pour la mise en oeuvre d'une méthode de détection de la présence de matières biologiques provenant d'oiseaux, notamment d'ansériformes, de galliformes ou de struthioniformes, et/ou d'identification de l'espèce d'oiseaux, notamment d'ansériformes, de galliformes ou de struthioniformes présente, dans un échantillon de matière organique susceptible de contenir de telles matières biologiques.

11. Utilisation selon la revendication 10, pour la mise en oeuvre d'une méthode de détection de la présence de matières biologiques provenant d'oiseaux, notamment d'ansériformes, de galliformes ou de struthioniformes, et/ou d'identification de l'espèce, notamment d'ansériformes, de galliformes ou de struthioniformes, dans des produits frais ou bien transformés tels que les produits agro-alimentaires, notamment le foie gras, les pâtés, les viandes, les graisses; les plats cuisinés, les oeufs, les produits manufacturés tels que ceux à base de plumes.

## Claims

1. Oligonucleotides **characterized in that** they are chosen from those :
(1) - presenting a sequence identity of at least 80%, preferably 90% and advantageously 95% with an oligonucleotide constituted by a sequence of approximately 15 to 25 nucleotides, in particular 20 to 25 nucleotides, contained in the following sequence SEQ ID N°32:
ACG TGA CYA GCY TCA GGC CCA TAC G
in which Y is C or T,
- or comprising the following sequence SEQ ID N°33:
CTT CAG GCC CAT ACG
- or constituted by the following sequence SEQ ID N°1:
ACT AGC TTC AGG CCC ATA CG
or those,
(2) - presenting a sequence identity of at least 80%, preferably 90% and advantageously 95% with an oligonucleotide constituted by a sequence of approximately 15 to 25 nucleotides, in particular 20 to 25 nucleotides, contained in the following sequence SEQ ID N°34:
CCC TAA ACC CCT CGC CCT CCT CAC A
- or comprising the following sequence SEQ ID N°35:
CTC GCC CTC CTC ACA
- or constituted by the following sequence SEQ ID N°2:
AAC CCC TCG CCC TCC TCA CA
or those,
(3) - presenting a sequence identity of at least 80%, preferably 90% and advantageously 95% with an oligonucleotide constituted by a sequence of approximately 15 to 25 nucleotides, in particular 20 to 25 nucleotides, contained in the following sequence SEQ ID N°36:
GGG CCA TCM ATT GGG TTC ACT CAC C
in which M is A or C,
- or comprising the following sequence SEQ ID N°37:
TTG GGT TCA CTC ACC
- or constituted by the following sequence SEQ ID N°3:
ATC AAT TGG GTT CAC TCA CC
or those,
(4) - presenting a sequence identity of at least 80%, preferably 90% and advantageously 95% with an oligonucleotide constituted by a sequence of approximately 15 to 25 nucleotides, in particular 20 to 25 nucleotides, contained in the following sequence SEQ ID N°38:
GGT RRA VST MNT CCA CAG ATG CCA C
in which R is A or G, V is A, C or G, N is A, C, G or T, S is C or G, M is A or C,
- or comprising the following sequence SEQ ID N°39:
TTC CAC AGA TGC CAC
- or constituted by the following sequence SEQ ID N°4:
AAG TAT TCC ACA GAT GCC AC
or those,
(5) - presenting a sequence identity of at least 80%, preferably 90% and advantageously 95% with an oligonucleotide constituted by a sequence of approximately 15 to 25 nucleotides, in particular 20 to 25 nucleotides, contained in the following sequence SEQ ID N°40:
CMG GAA RAR ADA ARW RGA ACC AGA G
in which R is A or G, D is A, G or T, M is A or C, W is A or T,
- or comprising the following sequence SEQ ID N°41:
A TAA AAA GAA CCA GAG
- or constituted by the following sequence SEQ ID N°5:
AAA AAT AAA AGG AAC CAG AG
or those,
(6) - presenting a sequence identity of at least 80%, preferably 90% and advantageously 95% with an oligonucleotide constituted by a sequence of approximately 15 to 25 nucleotides, in particular 20 to 25 nucleotides, contained in the following sequence SEQ ID N°42:
WVK CAY MYY KRY ACT GAT GCA CTT T
in which Y is C or T, K is G or T, R is A or G, M is A or C, W is A or T, V is A, C or G, M is A or C,
- or comprising the following sequence SEQ ID N°43:
ACA CTG ATG CAC TTT
- or constituted by the following sequence SEQ ID N° 11:
CCT TGA CAC TGA TGC ACT TT
or those,
(7) - presenting a sequence identity of at least 80%, preferably 90% and advantageously 95% with an oligonucleotide constituted by a sequence of approximately 15 to 25 nucleotides, in particular 20 to 25 nucleotides, contained in the following sequence SEQ ID N°44:
GCY TTD DND KKR WRS WAT GTG GAC G
in which Y is C or T, D is A, G or T, B is C, G or T, K is G or T, W is A or T, N is A, C, G or T, R is A or G, and S is C or G,
- or comprising the following sequence SEQ ID N°45:
TAA GCT ATG TGG ACG
- or constituted by the following sequence SEQ ID N°12:
TGT GGT AAG CTA TGT GGA CG
or those,
(8) - presenting a sequence identity of at least 80%, preferably 90% and advantageously 95% with an oligonucleotide constituted by a sequence of approximately 15 to 25 nucleotides, in particular 20 to 25 nucleotides, contained in the following sequence SEQ ID N°46:
MTN MHM BDN HMV HVN NCA TAC CCT A
in which Y is C or T, M is A or C, H is A, C or T, B is C, G or T, D is A, G or T, N is A, C, G or T, W is A or T, V is A, C or G,
- or comprising the following sequence SEQ ID N°47:
AAC GGA CAT ACC CTA
- or constituted by the following sequence SEQ ID N°14:
CAT GTC AAC GGA CAT ACC CTA
or those,
(9) - presenting a sequence identity of at least 80%, preferably 90% and advantageously 95% with an oligonucleotide constituted by a sequence of approximately 15 to 25 nucleotides, in particular 20 to 25 nucleotides, contained in the following sequence SEQ ID N°48:
CAT RAG NWA WKG KYG GGT GGA GAG G
in which R is A or G, N is A, C, G or T, W is A or T, K is G or T, Y is C or T,
- or comprising the following sequence SEQ ID N°49:
GGG TGG GTG GAG AGG
- or constituted by the following sequence SEQ ID N°15:
TAA TGG GTG GGT GGA GAG G
or those,
(10) - presenting a sequence identity of at least 80%, preferably 90% and advantageously 95% with an oligonucleotide constituted by a sequence of approximately 15 to 25 nucleotides, in particular 20 to 25 nucleotides, contained in the following sequence SEQ ID N°50:
GTT AWK GYY MYN CTC CGC RGC CCT T
in which W is A or T, K is G or T, Y is C or T, M is A or C, Y is C or T, N is A, C, G or T, R is A or G,
- or comprising the following sequence SEQ ID N°51:
TCC TCC GCA GCC CTT
- or constituted by the following sequence SEQ ID N°16:
ATG CTC TCC TCC GCA GCC CTT
or those,
(11) - presenting a sequence identity of at least 80%, preferably 90% and advantageously 95% with an oligonucleotide constituted by a sequence of approximately 15 to 25 nucleotides, in particular 20 to 25 nucleotides, contained in the following sequence SEQ ID N°52:
DTW ATG ATT HTY RTT ADA TTA CGC T
in which D is A, G or T, W is A or T, H is A, C or T, Y is C or T, R is A or G,
- or comprising the following sequence SEQ ID N°53:
TCG TTA GAT TAC GCT
- or constituted by the following sequence SEQ ID N°17:
GAT TGT CGT TAG ATT ACG CT
or those,
(12) - presenting a sequence identity of at least 80%, preferably 90% and advantageously 95% with an oligonucleotide constituted by a sequence of approximately 15 to 25 nucleotides, in particular 20 to 25 nucleotides, contained in the following sequence SEQ ID N°54:
AAY GHA TGA AYG HYC AGN DAC CAT
in which Y is C or T, H is A, C or T, N is A, C, G or T, D is A, G or T,
- or comprising the following sequence SEQ ID N°55:
ATG TTC TAG GAC CAT
- or constituted by the following sequence SEQ ID N°18:
ATG AAT GTT CTA GGA CCA T
or those,
(13) - presenting a sequence identity of at least 80%, preferably 90% and advantageously 95% with an oligonucleotide constituted by a sequence of approximately 15 to 25 nucleotides, in particular 20 to 25 nucleotides, contained in the following sequence SEQ ID N°56:
CGG GTT CTG ATT TCA CGT GAG GAG T
- or comprising the following sequence SEQ ID N°57:
TTT CAC GTG AGG AGT
- or constituted by the following sequence SEQ ID N°19:
TCT GAT TTC ACG TGA GGA GT
(14) - presenting a sequence identity of at least 80%, preferably 90% and advantageously 95% with an oligonucleotide constituted by a sequence of approximately 15 to 25 nucleotides, in particular 20 to 25 nucleotides, contained in the following sequence SEQ ID N°58:
ATA TGK MVN CGG CAT TAA CCT ATA T
in which K is G or T, M is A or C, V is A, C or G, N is A, C, G or T,
- or comprising the following sequence SEQ ID N°59:
GGC ATT AAC CTA TAT
- or constituted by the following sequence SEQ ID N°23:
ATA CGG GCA TTA ACC TAT AT
or those,
(15) - presenting a sequence identity of at least 80%, preferably 90% and advantageously 95% with an oligonucleotide constituted by a sequence of approximately 15 to 25 nucleotides, in particular 20 to 25 nucleotides, contained in the following sequence SEQ ID N°60:
GCG AGY ATA ACC AAA TSG GTT ACA T
in which Y is C or T, S is C or G,
- or comprising the following sequence SEQ ID N°61:
CCA AAT GGG TTA CAT
- or constituted by the following sequence SEQ ID N°24:
CAT AAC CAA ATG GGT TAC AT
or those,
(16) - presenting a sequence identity of at least 80%, preferably 90% and advantageously 95% with an oligonucleotide constituted by a sequence of approximately 15 to 25 nucleotides, in particular 20 to 25 nucleotides, contained in the following sequence SEQ ID N°62:
TAC ATA CAW ACY MWC CGC ACA AAT A
in which W is A or T, Y is C or T, M is A or C,
- or comprising the following sequence SEQ ID N°63:
CTC ACC GCA CAA ATA
- or constituted by the following sequence SEQ ID N°25:
ACA AAC TCA CCG CAC AAA TA
or those,
(17) - presenting a sequence identity of at least 80%, preferably 90% and advantageously 95% with an oligonucleotide constituted by a sequence of approximately 15 to 25 nucleotides, in particular 20 to 25 nucleotides, contained in the following sequence SEQ ID N°64:
TGT AAR TAW CAA TAT AAA TAA TAT A
in which R is A or G, W is A or T,
- or comprising the following sequence SEQ ID N°65:
AAT ATA AAT AAT ATA
- or constituted by the following sequence SEQ ID N°26:
TTA ACA ATA TAA ATA ATA TA
or those,
(18) - presenting a sequence identity of at least 80 %, preferably 90 % and advantageously 95 %, with an oligonucleotide constituted by a sequence of approximately 15 to 25 nucleotides, in particular 20 to 25 nucleotides, contained in the following sequence SEQ ID N° 70:
CCT TCM CAG TGM BKK YGS CRG RGT V
in which M is A or C, B is C, G or T, K is G or T, Y is C or T, S is C or G, R is A or G, V is A, C or G,
- or comprising the following sequence SEQ ID N°71:
GCC GTC GCC AGA GTA
- or constituted by the following sequence SEQ ID N°72:
ACA GTG CCG TCG CCA GAG TA
or those,
(19) - presenting a sequence identity of at least 80 %, preferably 90 % and advantageously 95 %, with an oligonucleotide constituted by a sequence of approximately 15 to 25 nucleotides, in particular 20 to 25 nucleotides, contained in the following sequence SEQ ID N° 73:
RRW WRT RGW KGR RGK GRR WWT TNR Y
in which R is A or G, W is A or T, K is G or T, N is A, C, G or T, Y is C or T,
- or comprising the following sequence SEQ ID N°74:
GAA GTG AAA ATT AGC
- or constituted by the following sequence SEQ ID N°75:
TAG AGG AAG TGA AAA TTA GC
or those,
(20) - presenting a sequence identity of at least 80%, preferably 90% and advantageously 95% with an oligonucleotide constituted by a sequence of approximately 15 to 25 nucleotides, in particular 20 to 25 nucleotides, contained in the following sequence SEQ ID N°66:
TGA ATG CTT GYY GGA CAT WAA TAC C
in which Y is C or T, W is A or T,
- or comprising the following sequence SEQ ID N°67:
CTG GAC ATA AAT ACC
- or constituted by the following sequence SEQ ID N°27:
GCT TGC TGG ACA TAA ATA CC
or those,
(21) - presenting a sequence identity of at least 80%, preferably 90% and advantageously 95% with an oligonucleotide constituted by a sequence of approximately 15 to 25 nucleotides, in particular 20 to 25 nucleotides, contained in the following sequence SEQ ID N°68:
CTA TAA TAK GRT GTG GGG CGT GTT G
in which K is G or T, R is A or G
- or comprising the following sequence SEQ ID N°69:
TTG TGG GGC GTG TTG
- or constituted by the following sequence SEQ ID N°28:
ATA AGT TGT GGG GCG TGT TG

2. Primer pairs **characterized in that** they are constituted:
- by any one of the oligonucleotides SEQ ID N°1, SEQ ID N°32, SEQ ID N°33, SEQ ID N°2, SEQ ID N°34, SEQ ID N°35, SEQ ID N°3, SEQ ID N°36, SEQ ID N°37 and any one of the oligonucleotides SEQ ID N°4, SEQ ID N°38, SEQ ID N°39, SEQ ID N°5, SEQ ID N°40, SEQ ID N°41 according to claim 1 or,
- by any one of the oligonucleotides SEQ ID N°11, SEQ ID N°42, SEQ ID N°43 and any one of the oligonucleotides SEQ ID N°12, SEQ ID N°44, SEQ ID N°45 according to claim 1 or,
- by any one of the oligonucleotides SEQ ID N°14, SEQ ID N°46, SEQ ID N°47, and any one of the oligonucleotides SEQ ID N°15, SEQ ID N°48, SEQ ID N°49 according to claim 1 or,
- by any one of the oligonucleotides SEQ ID N°16, SEQ ID N°50, SEQ ID N°51, and any one of the oligonucleotides SEQ ID N°17, SEQ ID N°52, SEQ ID N°53 according to claim 1 or,
- by any one of the oligonucleotides SEQ ID N°18, SEQ ID N°54, SEQ ID N°55, and any one of the oligonucleotides SEQ ID N°19, SEQ ID N°56, SEQ ID N°57 according to claim 1,
- by any one of the oligonucleotides SEQ ID N°23, SEQ ID N°58, SEQ ID N°59, and any one of the oligonucleotides SEQ ID N°24, SEQ ID N°60, SEQ ID N°61 according to claim 1 or,
- by any one of the oligonucleotides SEQ ID N°25, SEQ ID N°62, SEQ ID N°63, and any one of the oligonucleotides SEQ ID N°26, SEQ ID N°64, SEQ ID N°65 according to claim 1 or,
- by any one of the oligonucleotides SEQ ID N°70, SEQ ID N°71, SEQ ID N°72, and any one of the oligonucleotides SEQ ID N°73, SEQ ID N°74, SEQ ID N°75 according to claim 1,
- by any one of the oligonucleotides SEQ ID N°27, SEQ ID N°66, SEQ ID N°67, and any one of the oligonucleotides SEQ ID N°28, SEQ ID N°68, SEQ ID N°69 according to claim 1,
and advantageously constituted by the pair of oligonucleotides chosen from the following pairs:
- SEQ ID N° 1 and SEQ ID N° 5,
- SEQ ID N° 2 and SEQ ID N° 5,
- SEQ ID N° 1 and SEQ ID N° 4,
- SEQ ID N° 2 and SEQ ID N° 4,
- SEQ ID N° 3 and SEQ ID N° 5,
- SEQ ID N° 11 and SEQ ID N° 12,
- SEQ ID N° 14 and SEQ ID N° 15,
- SEQ ID N° 16 and SEQ ID N° 17,
- SEQ ID N° 18 and SEQ ID N° 19,
- SEQ ID N°23 and SEQ ID N°24,
- SEQ ID N°25 and SEQ ID N°26,
- SEQ ID N°72 and SEQ ID N°75,
- SEQ ID N°27 and SEQ ID N°28.

3. Method of detecting the presence of biological materials derived from birds, in particular from Anseriformes, Galliformes or Struthioniformes, in a sample of organic material, **characterized in that** the presence of DNA derived from birds in the said organic material is determined by amplification of a DNA sequence specific to the genome of birds, and contained in the DNA extracted from the said sample, namely a sequence present in the genomes of birds, but absent from the genomes of other animal genera, and in particular of other animal species,
said sequence being a mitochondrial DNA sequence that is specific of the genomes of birds, said sequence being amplified with oligonucleotides chosen from the oligonucleotides as defined in claim 1.

4. Method according to claim 3, comprising also the identification of the genus, in particular the species of bird, in particular Anseriforme, Galliforme or Struthioniforme, present in the said sample, **characterized in that** the DNA sequence specific to the genome of birds thus amplified as defined in claim 3, is compared with other DNA sequences of the genome of birds, the said DNA sequence specific to the genome of birds, thus amplified, presenting at least 50% identity, in particular 60% identity with the other aforementioned DNA sequences of the genome of birds.

5. Method according to claim 3 or claim 4, **characterized in that** it permits the detection and/or identification of the presence of Anseriformes, in particular of Anatidae chosen from the group constituted by the ducks such as the pintail *(Anas acuta*), mandarin duck (*Aix galericulata*), shoveler *(Anas clypeata*), garganey *(Anas querquedula*), eider *(Somateria mollissima*), geese such as the lesser white-fronted goose *(Anser erythropus*), snow goose *(Anser caerulescens*), Canada goose *(Branta canadensis*), and swans such as the whooper swan *(Cygnus cygnus*), black swan *(Cygnus atratus*), mute swan *(Cygnus olor)* and in particular the domestic species of ducks such as the mallard *(Anas platyrhynchos*), Muscovy duck *(Cairina moschata*) or the domestic species of goose such as the greylag goose *(Anser anser)*.

6. Method according to any one of claims 3 to 5, **characterized in that** the amplification of the said DNA sequence specific to the genome of birds, in particular of Anseriformes, Galliformes or Struthioniformes, is carried out by the polymerase chain (PCR) amplification method, comprising a repetition of the cycle of the following steps:
- heating of the DNA extracted from the sample of organic material, so as to separate the DNA into two single-stranded strands,
- hybridization of oligonucleotide primers according to claims 1 or 2 to the single-stranded DNA strands at an adequate temperature and,
- elongation of said oligonucleotide primers by a polymerase at an adequate temperature, in order to obtain an amplified DNA sequence or DNA fragment specific to the genome of birds, in particular of Anseriformes, Galliformes or Struthioniformes.

7. Method according to one of claims 3 to 6, **characterized in that** the amplified DNA fragment obtained is identified:
- by sequencing of the said amplified fragment,
- by gel electrophoresis of the fragments obtained after digestion using restriction enzymes of the said amplified fragment,
- directly, by simple visualization of the presence of the said amplified fragment by gel electrophoresis.

8. Method according to any one of claims 3 to 7, **characterized in that** the DNA extracted from the sample of organic material is:
- non-degraded DNA derived in particular from a fresh sample or,
- degraded DNA, derived in particular from a processed, in particular cooked, freeze-dried, dried, pickled, appertized or pasteurized, sample.

9. DNA fragment as amplified at the end of the method according to one of claims 3 to 8, **characterized in that** it presents a sequence identity of at least 80%, preferably 90% and advantageously 95% with:
- the following SEQ ID N°6: 
ACYAGCTTCA GGCCCATACG TTCCCCCTAA ACCCCTCGCC CTCCTCACAT TTTTGCGCCT
CTGGTTCCTC GGTCAGGGCC ATCMATTGGG TTCACTCACC YCYMYTYGCC YTTCAAAGTG
GCATCTGTGG ANKACBTYCA CCWYYYCRRT GCGTWATCGC GGCATBYTYM ASYWTTTWSM
CGCCTYTGGT TCYMYTTHTY TYT
 in which Y is C or T, M is A or C, K is G or T, B is C, G or T, R is A or G, W is A or T, S is C or G, H is A, C or T, N is A, C, G or T,
the said sequence SEQ ID N° 6 comprising 203 base pairs,
- or the following SEQ ID N°7: 
ACYAGCTTCA GGCCCATACG TTCCCCCTAA ACCCCTCGCC CTCCTCACAT TTTTGCGCCT
CTGGTTCCTC GGTCAGGGCC ATCMATTGGG TTCACTCACC YCYMYTYGCC YTTCAAAGTG
GCATCTGTGG ANKACBT
 in which Y is C or T, M is A or C, N is A, C, G or T, K is G or T, B is C, G or T,
the said sequence SEQ ID N°7 comprising 137 base pairs,
- or the following SEQ ID N°8: AACCCCTCGC   CCTCCTCACA   TTTTTGCGCC   TCTGGTTCCT   CGGTCAGGGC   CATCMATTGG 
GTTCACTCAC CYCYMYTYGC CYTTCAAAGT GGCATCTGTG GANKACBTYC ACCWYYYCRR
TGCGTWATCG CGGCATBYTY MASYWTTTWS MCGCCTYTGG TTCYMYTTHT YTYT
 in which Y is C or T, M is A or C, N is A, C, G or T, K is G or T, B is C, G or T, R is A or G, W is A or T, S is C or G, H is A, C or T,
the said sequence SEQ ID N°8 comprising 174 base pairs,
- or the following SEQ ID N°9: 
AACCCCTCGC CCTCCTCACA TTTTTGCGCC TCTGGTTCCT CGGTCAGGGC CATCMATTGG
GTTCACTCAC CYCYMYTYGC CYTTCAAAGT GGCATCTGTG GANKACBT
 in which Y is C or T, M is A or C, N is A, C, G or T, K is G or T, B is C, G or T,
the said sequence SEQ ID N°9 comprising 108 base pairs,
- or the following SEQ ID N°10: 
ATCMATTGGG TTCACTCACC YCYMYTYGCC YTTCAAAGTG GCATCTGTGG ANKACBTYCA
CCWYYYCRRT GCGTWATCGC GGCATBYTYM ASYWTTTWSM CGCCTYTGGT TCYMYTTHTY
TYT
 in which Y is C or T, M is A or C, N is A, C, G or T, K is G or T, B is C, G or T, R is A or G, W is A or T, S is C or G, H is A, C or T,
the said sequence SEQ ID N°10 comprising 123 base pairs,
- or the following SEQ ID N°13: 
YMYYKRYACT GATGCACTTK GDYCDCATTY RGTTRWKGYY MYNTCCRCHV HYCYABNBWH
WVRTGYTRYY NRGTGAATGC TYGHHGGACA TADYWYWAWM MAWHWWMHYW YYHWSYRYWW
YYYHYRSRMH HHMNRAAYDM AACCARHRDW YTTYMDCHRW THWDHWWHMM WHYWYVTWDY
RTMWWHYHHV HMHHHHMHWM HHBHAWWHHH HNHNHYNHWA YYBCHNNYHH HWAWYNYSYY
WHHYNWBVHW HHNBYBDMDM DWCHDWMMNR WWHDMAYDDH DYVDHHYAWM HTTATTAKAG
RAACWCCAGY ACYARVVVHD VWHNHAMHYN RRHRWHHMYY AYWAHYYHVH YNTYNMNAMH
HNBNVYHYRD HYHRYYARYB RMHYVHHHYR YYCDCMTWSY WYMMHNHH
 in which Y is C or T, M is A or C, N is A, C, G or T, K is G or T, B is C, G or T, R is A or G, W is A or T, S is C or G, D is A, G or T, V is A, C or G, H is A, C or T,
the said sequence SEQ ID N°13 comprising 408 base pairs,
- or the following SEQ ID N°77: 
YMYYKRCTGA TGCACTTKGD YCDCATTYRG TTRWKGYYMY CCRCHVHYCY ABNBWHWVRT
GYTRYYNRGT GAATGCTYGH HGGACATADY WYWAWMMAWH WMHYWYYHWS YRYWWYYYHY
RSRMHHHMRA AYDMAACCAR HRDWYTTYMD CHRWTHDYRT MWWHYHVHMH HNHWAYYBCH
NNYHDMDWCH DWMMRWWHDM AYDDHDYVDH HYAWHTTATT AKAGRAACWC CAGYACYARV
VVHDVWHNHA MHYNRRHRWH MYYAYWAHYY HVHYNTYNMN AMHHNBNVYH YRDHYHRYYA
 RYBRMHYVHH HYRYYCDCMT WSYWYMMHNH H in which Y is C or T, M is A or C, N is A, C, G or T, K is G or T, B is C, G or T, R is A or G, W is A or T, S is C or G, D is A, G or T, V is A, C or G, H is A, C or T,
the said sequence SEQ ID N°77 comprising 331 base pairs,
- or the following SEQ ID N°78: 
YMYYKRYACT GATGCACTTK GDYCDCATTY RGTTRWKGYY MYNTCCRCHV HYCYABNBWH
WVRTGYTRYY NRGTGAATGC TYGHHGGACA TAMHHHMNRA AYDMAACCAY DMAACHRHYH
HVHMHHHNHN HYNHHNNYRH HWAYYBCHNN YHHWAWYMYS YYWHHYNWBV HWHNBYBDMD
MDWCHDWMNR WWHDMAYDDH DYVDHHYAWM HTTATTAKAG RAACWCCAGY ACYARVVVHD
VWHNHAMHYN RRHRWHHMYY AYWAHYYHVH YNTYNMNAMH HNBNVYHYRD HYHRYYARYB
RMHYVHHHYR YYCDCMTWSY WYMMHNHH
 in which Y is C or T, M is A or C, N is A, C, G or T, K is G or T, B is C, G or T, R is A or G, W is A or T, S is C or G, D is A, G or T, V is A, C or G, H is A, C or T,
the said sequence SEQ ID N°78 comprising 328 base pairs,
- or the following SEQ ID N°79: 
YMYYKRYACT GATGCACTTK GDYCDCATTY RGTTRWKGYY MYNTCCRCHV HYCYABNBWH
WVRTGYTRYY NRGTGAATGC TYGHHGGACA TAMHHHMNRA AYDMAACHRH YHHVHMHHHN
HNHYNHHNNY HHHWAYWBVH WHWCHDWMMN RWWHDMAYDD HDYVDHHYAW MHTTATTAKA
GRAACWCCAG YACYARVVVH DVWHNHAMHY NRRHRWHHMY YAYWAHYYHV HYNTYNMNMH
HNBNVYHYRD HYHRYYARYB RMHYVHHHYR YYCDCMTWSY WYMMHNHH
 in which Y is C or T, M is A or C, N is A, C, G or T, K is G or T, B is C, G or T, R is A or G, W is A or T, S is C or G, D is A, G or T, V is A, C or G, H is A, C or T,
the said sequence SEQ ID N°79 comprising 288 base pairs,
- or the following SEQ ID N°20: 
HWBDNHMRMG RNCATHHHHN NYHVHBDNAH NNNHCHHCCA VVRMHHHNNV RTGMAYGHYC
YHRNDVHHHH NHVNHVVMHV VMHVNVHNHN NHHHNHVHVV RWNMACMARR RCMMHDHNNR
VTGAATGCTH NMHRRACAWR DMNYNDVHAH HMHTCYDDNB YYBHHTCCAC CCRMMCATWH
 in which Y is C or T, M is A or C, N is A, C, G or T, B is C, G or T, R is A or G, W is A or T, D is A, G or T, V is A, C or G, H is A, C or T,
the said sequence SEQ ID N°20 comprising 180 base pairs,
- or the following SEQ ID N°21: 
WKGYYMYNTC CRCHVHYCYA BNBWHWVRTG YTRYYNRGTG AATGCTYGHH GGACATAMHH
HMNRAAYDMA ACHRHYHHVH MHHHNHNHNH HNNYHHHWAY WBVHWHWCHD WMMNRWWHDM
 
AYDDHDYVDH HYAWMHTTAT TAKAGRAACW CCAGYACYAR VVVHDVWHNH AMHYNRRHRW
HHMYYAYWAH Y
 in which Y is C or T, M is A or C, N is A, C, G or T, B is C, G or T, R is A or G, W is A or T, D is A, G or T, V is A, C or G, H is A, C or T, K is G or T,
the said sequence SEQ ID N°21 comprising 191 base pairs,
- or the following SEQ ID N°22: 
RTGAAYGHYC YHRNDVHHNH MVHNHNVMHV VNHNHHNHNH VVVSWNMARD SCMNHNNTTM
VTGAATGCTH NMHRRACAWD MNYNDVHAHH MHTCYNDNBH YBHHTCCACC CRMMCATWHM
TYATGRAVYY NCGTAYCARR TGGATTTATT RRYCGTACWC CTCACGTGAA ATCAGC
 in which Y is C or T, M is A or C, N is A, C, G or T, B is C, G or T, R is A or G, W is A or T, D is A, G or T, V is A, C or G, H is A, C or T, S is C or G,
the said sequence SEQ ID N°22 comprising 176 base pairs.
- the following SEQ ID N°29: 
KMAWVNGGHM WTVACHYATA TTCCMCWTYY CKYMCHAHVB MCHYTVDATG MAYSMTSHHRK
RCMHVCTMHN TTHCVYDHMC CATAGABAGT TCCWAACHAC WATCRRKVMM CMTAACTATG
AATGGTTRCVG HACATAAATC TCACTCTCAT GTTCTCCCCC CAACAAGTC CCTAACTATG
AATGGTTACAG GACATACATT TDACTAYCAT GTTCTAACCS ATTTGGTT
 in which Y is C or T, M is A or C, N is A, C, G or T, B is C, G or T, R is A or G, W is A or T, D is A, G or T, V is A, C or G, H is A, C or T, S is C or G, K is G or T,
the said sequence SEQ ID N°29 comprising 231 base pairs,
- or the following SEQ ID N°30: 
TACAWACYMW CGYAWWRRWD CCCTYAMAYY AYAHAAMCGY YTATCGYWTA DTATATATAC
ATTRYWRYDY AYYMYAYMAT TATTAKAGAA ACYCCMCTAC CRAAACMAWC AWHARARRCW
WAHADHWAHA TKCMRCDYAW VBYHCYTCAS AWRCMWWBRT KRTWTATATT GTTAA
 in which Y is C or T, M is A or C, B is C, G or T, R is A or G, W is A or T, D is A, G or T, V is A, C or G, H is A, C or T, S is C or G, K is G or T,
the said sequence SEQ ID N°30 comprising 175 base pairs,
- or the following SEQ ID N°76: 
MCAGTGMSKK YGSCRGRGTV CYAYTCAAKY KRAGCCTGGA YTACWCCTGC GTTRCGYSCT
ATYCTAKWYY TCHVGKRTYM CTCRATGAKA CGGTTKGCGT RTATKKGGWA TCAYYTTGAC
ACTGATGCAC TTTGGAYCRC ATTYRGTTAW KGYTCTYCCR CMSYYCYNKW WARTRGKGYT
ATWTAGTGAA TGCTYGHYGG ACATAYYTTA WSYNAAWWYY CMCYYCMWCY A
 in which M is A or C, S is C or G, K is G or T, Y is C or T, R is A or G, V is A, C or G, W is A or T, N is A, C, T or G,
the said sequence SEQ ID N°76 comprising 231 base pairs,
- or the following SEQ ID N°31: 
TGAATGCTTG YYGGACATWW TWYMAYYWAT TWTCACTTCC WCTGAYTTTY YTMACAMMAC
YAGGAYWMAW YTYYTWYDTY DKTWYWAAYW YWTKWTTTVW TYAYWTYAAW MACATYTTTT
AMYTAWAYTA MCYWTACAWA CYMWCMGYAY WRRWTDTYAM AYYAAHAAMC GYYCGYWTAD
TATATATACA TARYWRYTGY RYYTMYAYCM ATTATT
 in which Y is C or T, M is A or C, R is A or G, W is A or T, D is A, G or T, V is A, C or G, H is A, C or T,
the said sequence SEQ ID N°31 comprising 216 base pairs.

10. Use of nucleotide sequences chosen from the oligonucleotide primers according to any one of claims 1 or 2, or DNA fragments according to claim 9, for implementing a method of detecting the presence of biological materials derived from birds, in particular Anseriformes, Galliformes or Struthioniformes, and/or of identifying the species of birds, in particular Anseriformes, Galliformes or Struthioniformes, that is present in a sample of organic material capable of containing such biological materials.

11. Use according to claim 10, for implementing a method of detecting the presence of biological materials derived from birds, in particular Anseriformes, Galliformes or Struthioniformes, and/or of identifying the species, in particular Anseriformes, Galliformes or Struthioniformes, in fresh or processed products such as agro-food products, in particular foie gras, pâtés, meats, fats, cooked dishes, eggs, manufactured products such as those based on feathers.

## Patentansprüche

1. Oligonucleotide, **dadurch gekennzeichnet, dass** sie ausgewählt sind aus jenen,
(1) - die eine Sequenzidentität von mindestens 80 %, vorzugsweise 90 % und vorteilhafterweise 95 %, mit einem Oligonucleotid aufweisen, das aus einer Sequenz von 15 bis 25 Nucleotiden, insbesondere 20 bis 25 Nucleotiden gebildet ist, enthalten in der folgenden Sequenz SEQ ID NO 32:
ACG TGA CYA GCY TCA GGC CCA TAC G
worin Y gleich C oder T ist,
- oder die folgende Sequenz SEQ ID NO 33 umfassen:
CTT CAG GCC CAT ACG
- oder durch die folgende Sequenz SEQ ID NO 1 gebildet sind:
ACT AGC TTC AGG CCC ATA CG
oder jenen,
(2) - die eine Sequenzidentität von mindestens 80 %, vorzugsweise 90 % und vorteilhafterweise 95 %, mit einem Oligonucleotid aufweisen, das aus einer Sequenz von 15 bis 25 Nucleotiden, insbesondere 20 bis 25 Nucleotiden gebildet ist, enthalten in der folgenden Sequenz SEQ ID NO 34:
CCC TAA ACC CCT CGC CCT CCT CAC A
- oder die folgende Sequenz SEQ ID NO 35 umfassen:
CTC GCC CTC CTC ACA
- oder durch die folgende Sequenz SEQ ID NO 2 gebildet sind:
AAC CCC TCG CCC TCC TCA CA
oder jenen,
(3) - die eine Sequenzidentität von mindestens 80 %, vorzugsweise 90 % und vorteilhafterweise 95 %, mit einem Oligonucleotid aufweisen, das aus einer Sequenz von 15 bis 25 Nucleotiden, insbesondere 20 bis 25 Nucleotiden gebildet ist, enthalten in der folgenden Sequenz SEQ ID NO 36:
GGG CCA TCM ATT GGG TTC ACT CAC C
worin M gleich A oder C ist,
- oder die folgende Sequenz SEQ ID NO 37 umfassen:
TTG GGT TCA CTC ACC
- oder durch die folgende Sequenz SEQ ID NO 3 gebildet sind:
ATC AAT TGG GTT CAC TCA CC
oder jenen,
(4) - die eine Sequenzidentität von mindestens 80 %, vorzugsweise 90 % und vorteilhafterweise 95 %, mit einem Oligonucleotid aufweisen, das aus einer Sequenz von 15 bis 25 Nucleotiden, insbesondere 20 bis 25 Nucleotiden gebildet ist, enthalten in der folgenden Sequenz SEQ ID NO 38:
GGT RRA VST MNT CCA CAG ATG CCA C
worin R gleich A oder G ist, V gleich A, C oder G ist, N gleich A, C, G oder T ist,
S gleich C oder G ist, M gleich A oder C ist,
- oder die folgende Sequenz SEQ ID NO 39 umfassen:
TTC CAC AGA TGC CAC
- oder durch die folgende Sequenz SEQ ID NO 4 gebildet sind:
AAG TAT TCC ACA GAT GCC AC
oder jenen,
(5) - die eine Sequenzidentität von mindestens 80 %, vorzugsweise 90 % und vorteilhafterweise 95 %, mit einem Oligonucleotid aufweisen, das aus einer Sequenz von 15 bis 25 Nucleotiden, insbesondere 20 bis 25 Nucleotiden gebildet ist, enthalten in der folgenden Sequenz SEQ ID NO 40:
CMG GAA RAR ADA ARW RGA ACC AGA G
worin R gleich A oder G ist, D gleich A, G oder T ist, M gleich A oder C ist, W
gleich A oder T ist,
- oder die folgende Sequenz SEQ ID NO 41 umfassen:
A TAA AAA GAA CCA GAG
- oder durch die folgende Sequenz SEQ ID NO 5 gebildet sind:
AAA AAT AAA AGG AAC CAG AG
oder jenen,
(6) - die eine Sequenzidentität von mindestens 80 %, vorzugsweise 90 % und vorteilhafterweise 95 %, mit einem Oligonucleotid aufweisen, das aus einer Sequenz von 15 bis 25 Nucleotiden, insbesondere 20 bis 25 Nucleotiden gebildet ist, enthalten in der folgenden Sequenz SEQ ID NO 42:
WVK CAY MYY KRY ACT GAT GCA CTT T
worin Y gleich C oder T ist, K gleich G oder T ist, R gleich A oder G ist, M gleich A oder C ist, W gleich A oder T ist, V gleich A, C oder G ist, M gleich A oder C ist,
- oder die folgende Sequenz SEQ ID NO 43 umfassen:
ACA CTG ATG CAC TTT
- oder durch die folgende Sequenz SEQ ID NO 11 gebildet sind:
CCT TGA CAC TGA TGC ACT TT
oder jenen,
(7) - die eine Sequenzidentität von mindestens 80 %, vorzugsweise 90 % und vorteilhafterweise 95 %, mit einem Oligonucleotid aufweisen, das aus einer Sequenz von 15 bis 25 Nucleotiden, insbesondere 20 bis 25 Nucleotiden gebildet ist, enthalten in der folgenden Sequenz SEQ ID NO 44:
GCY TTD DND KKR WRS WAT GTG GAC G
worin Y gleich C oder T ist, D gleich A, G oder T ist, B gleich C, G oder T ist, K gleich G oder T ist, W gleich A oder T ist, N gleich A, C, G oder T ist, R gleich A oder G ist und S gleich C oder G ist,
- oder die folgende Sequenz SEQ ID NO 45 umfassen:
TAA GCT ATG TGG ACG
- oder durch die folgende Sequenz SEQ ID NO 12 gebildet sind:
TGT GGT AAG CTA TGT GGA CG
oder jenen,
(8) - die eine Sequenzidentität von mindestens 80 %, vorzugsweise 90 % und vorteilhafterweise 95 %, mit einem Oligonucleotid aufweisen, das aus einer Sequenz von 15 bis 25 Nucleotiden, insbesondere 20 bis 25 Nucleotiden gebildet ist, enthalten in der folgenden Sequenz SEQ ID NO 46:
MTN MHM BDN HMV HVN NCA TAC CCT A
worin Y gleich C oder T ist, M gleich A oder C ist, H gleich A, C oder T ist, B gleich C, G oder T ist, D gleich A, G oder T ist, N gleich A, C, G oder T ist, W gleich A oder T ist, V gleich A, C oder G ist,
- oder die folgende Sequenz SEQ ID NO 47 umfassen:
AAC GGA CAT ACC CTA
- oder durch die folgende Sequenz SEQ ID NO 14 gebildet sind:
CAT GTC AAC GGA CAT ACC CTA
oder jenen,
(9) - die eine Sequenzidentität von mindestens 80 %, vorzugsweise 90 % und vorteilhafterweise 95 %, mit einem Oligonucleotid aufweisen, das aus einer Sequenz von 15 bis 25 Nucleotiden, insbesondere 20 bis 25 Nucleotiden gebildet ist, enthalten in der folgenden Sequenz SEQ ID NO 48:
CAT RAG NWA WKG KYG GGT GGA GAG G
worin R gleich A oder G ist, N gleich A, C, G oder T ist, W gleich A oder T ist, K gleich G oder T ist, Y gleich C oder T ist,
- oder die folgende Sequenz SEQ ID NO 49 umfassen:
GGG TGG GTG GAG AGG
- oder durch die folgende Sequenz SEQ ID NO 15 gebildet sind:
TAA TGG GTG GGT GGA GAG G
oder jenen,
(10) - die eine Sequenzidentität von mindestens 80 %, vorzugsweise 90 % und vorteilhafterweise 95 %, mit einem Oligonucleotid aufweisen, das aus einer Sequenz von 15 bis 25 Nucleotiden, insbesondere 20 bis 25 Nucleotiden gebildet ist, enthalten in der folgenden Sequenz SEQ ID NO 50:
GTT AWK GYY MYN CTC CGC RGC CCT T
worin W gleich A oder T ist, K gleich G oder T ist, Y gleich C oder T ist, M gleich A oder C ist, Y gleich C oder T ist, N gleich A, C, G oder T ist, R gleich A oder G ist,
- oder die folgende Sequenz SEQ ID NO 51 umfassen:
TCC TCC GCA GCC CTT
- oder durch die folgende Sequenz SEQ ID NO 16 gebildet sind:
ATG CTC TCC TCC GCA GCC CTT
oder jenen,
(11) - die eine Sequenzidentität von mindestens 80 %, vorzugsweise 90 % und vorteilhafterweise 95 %, mit einem Oligonucleotid aufweisen, das aus einer Sequenz von 15 bis 25 Nucleotiden, insbesondere 20 bis 25 Nucleotiden gebildet ist, enthalten in der folgenden Sequenz SEQ ID NO 52:
DTW ATG ATT HTY RTT ADA TTA CGC T
worin D gleich A, G oder T ist, W gleich A oder T ist, H gleich A, C oder T ist, Y gleich C oder T ist, R gleich A oder G ist,
- oder die folgende Sequenz SEQ ID NO 53 umfassen:
TCG TTA GAT TAC GCT
- oder durch die folgende Sequenz SEQ ID NO 17 gebildet sind:
GAT TGT CGT TAG ATT ACG CT
oderjenen,
(12) - die eine Sequenzidentität von mindestens 80 %, vorzugsweise 90 % und vorteilhafterweise 95 %, mit einem Oligonucleotid aufweisen, das aus einer Sequenz von 15 bis 25 Nucleotiden, insbesondere 20 bis 25 Nucleotiden gebildet ist, enthalten in der folgenden Sequenz SEQ ID NO 54:
AAY GHA TGA AYG HYC AGN DAC CAT
worin Y gleich C oder T ist, H gleich A, C oder T ist, N gleich A, C, G oder T ist, D gleich A, G oder T ist,
- oder die folgende Sequenz SEQ ID NO 55 umfassen:
ATG TTC TAG GAC CAT
- oder durch die folgende Sequenz SEQ ID NO 18 gebildet sind:
ATG AAT GTT CTA GGA CCA T
oder jenen,
(13) - die eine Sequenzidentität von mindestens 80 %, vorzugsweise 90 % und vorteilhafterweise 95 %, mit einem Oligonucleotid aufweisen, das aus einer Sequenz von 15 bis 25 Nucleotiden, insbesondere 20 bis 25 Nucleotiden gebildet ist, enthalten in der folgenden Sequenz SEQ ID NO 56:
CGG GTT CTG ATT TCA CGT GAG GAG T
- oder die folgende Sequenz SEQ ID NO 57 umfassen:
TTT CAC GTG AGG AGT
- oder durch die folgende Sequenz SEQ ID NO 19 gebildet sind:
TCT GAT TTC ACG TGA GGA GT
(14) - die eine Sequenzidentität von mindestens 80 %, vorzugsweise 90 % und vorteilhafterweise 95 %, mit einem Oligonucleotid aufweisen, das aus einer Sequenz von 15 bis 25 Nucleotiden, insbesondere 20 bis 25 Nucleotiden gebildet ist, enthalten in der folgenden Sequenz SEQ ID NO 58:
ATA TGK MVN CGG CAT TAA CCT ATA T
worin K gleich G oder T ist, M gleich A oder C ist, V gleich A, C oder G ist, N gleich A, C, G oder T ist,
- oder die folgende Sequenz SEQ ID NO 59 umfassen:
GGC ATT AAC CTA TAT
- oder durch die folgende Sequenz SEQ ID NO 23 gebildet sind:
ATA CGG GCA TTA ACC TAT AT
oder jenen,
(15) - die eine Sequenzidentität von mindestens 80 %, vorzugsweise 90 % und vorteilhafterweise 95 %, mit einem Oligonucleotid aufweisen, das aus einer Sequenz von 15 bis 25 Nucleotiden, insbesondere 20 bis 25 Nucleotiden gebildet ist, enthalten in der folgenden Sequenz SEQ ID NO 60:
GCG AGY ATA ACC AAA TSG GTT ACA T
worin Y gleich C oder T ist, S gleich C oder G ist,
- oder die folgende Sequenz SEQ ID NO 61 umfassen:
CCA AAT GGG TTA CAT
- oder durch die folgende Sequenz SEQ ID NO 24 gebildet sind:
CAT AAC CAA ATG GGT TAC AT
oder jenen,
(16) - die eine Sequenzidentität von mindestens 80 %, vorzugsweise 90 % und vorteilhafterweise 95 %, mit einem Oligonucleotid aufweisen, das aus einer Sequenz von 15 bis 25 Nucleotiden, insbesondere 20 bis 25 Nucleotiden gebildet ist, enthalten in der folgenden Sequenz SEQ ID NO 62:
TAC ATA CAW ACY MWC CGC ACA AAT A
worin W gleich A oder T ist, Y gleich C oder T ist, M gleich A oder C ist,
- oder die folgende Sequenz SEQ ID NO 63 umfassen:
CTC ACC GCA CAA ATA
- oder durch die folgende Sequenz SEQ ID NO 25 gebildet sind:
ACA AAC TCA CCG CAC AAA TA
oder jenen,
(17) - die eine Sequenzidentität von mindestens 80 %, vorzugsweise 90 % und vorteilhafterweise 95 %, mit einem Oligonucleotid aufweisen, das aus einer Sequenz von 15 bis 25 Nucleotiden, insbesondere 20 bis 25 Nucleotiden gebildet ist, enthalten in der folgenden Sequenz SEQ ID NO 64:
TGT AAR TAW CAA TAT AAA TAA TAT A
worin R gleich A oder G ist, W gleich A oder T ist,
- oder die folgende Sequenz SEQ ID NO 65 umfassen:
AAT ATA AAT AAT ATA
- oder durch die folgende Sequenz SEQ ID NO 26 gebildet sind:
TTA ACA ATA TAA ATA ATA TA
oder jenen,
(18) - die eine Sequenzidentität von mindestens 80 %, vorzugsweise 90 % und vorteilhafterweise 95 %, mit einem Oligonucleotid aufweisen, das aus einer Sequenz von 15 bis 25 Nucleotiden, insbesondere 20 bis 25 Nucleotiden gebildet ist, enthalten in der folgenden Sequenz SEQ ID NO 70:
CCT TCM CAG TGM BKK YGS CRG RGT V
worin M gleich A oder C ist, B gleich C, G oder T ist, K gleich G oder T ist, Y gleich C oder T ist, S gleich C oder G ist, R gleich A oder G ist, V gleich A, C oder G ist,
- oder die folgende Sequenz SEQ ID NO 71 umfassen:
GCC GTC GCC AGA GTA
- oder durch die folgende Sequenz SEQ ID NO 72 gebildet sind:
ACA GTG CCG TCG CCA GAG TA
oder jenen,
(19) - die eine Sequenzidentität von mindestens 80 %, vorzugsweise 90 % und vorteilhafterweise 95 %, mit einem Oligonucleotid aufweisen, das aus einer Sequenz von 15 bis 25 Nucleotiden, insbesondere 20 bis 25 Nucleotiden gebildet ist, enthalten in der folgenden Sequenz SEQ ID NO 73:
RRW WRT RGW KGR RGK GRR WWT TNR Y
worin R gleich A oder G ist, W gleich A oder T ist, K gleich G oder T ist, N gleich A, C, G oder T ist, Y gleich C oder T ist,
- oder die folgende Sequenz SEQ ID NO 74 umfassen:
GAA GTG AAA ATT AGC
- oder durch die folgende Sequenz SEQ ID NO 75 gebildet sind:
TAG AGG AAG TGA AAA TTA GC
oder jenen,
(20) - die eine Sequenzidentität von mindestens 80 %, vorzugsweise 90 % und vorteilhafterweise 95 %, mit einem Oligonucleotid aufweisen, das aus einer Sequenz von 15 bis 25 Nucleotiden, insbesondere 20 bis 25 Nucleotiden gebildet ist, enthalten in
der folgenden Sequenz SEQ ID NO 66:
TGA ATG CTT GYY GGA CAT WAA TAC C
worin Y gleich C oder T ist, W gleich A oder T ist,
- oder die folgende Sequenz SEQ ID NO 67 umfassen:
CTG GAC ATA AAT ACC
- oder durch die folgende Sequenz SEQ ID NO 27 gebildet sind:
GCT TGC TGG ACA TAA ATA CC
oder jenen,
(21) - die eine Sequenzidentität von mindestens 80 %, vorzugsweise 90 % und vorteilhafterweise 95 %, mit einem Oligonucleotid aufweisen, das aus einer Sequenz von 15 bis 25 Nucleotiden, insbesondere 20 bis 25 Nucleotiden gebildet ist, enthalten in der folgenden Sequenz SEQ ID NO 68:
CTA TAA TAK GRT GTG GGG CGT GTT G
worin K gleich G oder T ist, R gleich A oder G ist,
- oder die folgende Sequenz SEQ ID NO 69 umfassen:
TTG TGG GGC GTG TTG
- oder durch die folgende Sequenz SEQ ID NO 28 gebildet sind:
ATA AGT TGT GGG GCG TGT TG

2. Primerpaare, **dadurch gekennzeichnet, dass** sie gebildet sind aus:
- irgendeinem der Oligonucleotide SEQ ID NO 1, SEQ ID NO 32, SEQ ID NO 33, SEQ ID NO 2, SEQ ID NO 34, SEQ ID NO 35, SEQ ID NO 3, SEQ ID NO 36, SEQ ID NO 37 und irgendeinem der Oligonucleotide SEQ ID NO 4, SEQ ID NO 38, SEQ ID NO 39, SEQ ID NO 5, SEQ ID NO 40, SEQ ID NO 41 nach Anspruch 1 oder
- irgendeinem der Oligonucleotide SEQ ID NO 11, SEQ ID NO 42, 15 SEQ ID NO 43 und irgendeinem der Oligonucleotide SEQ ID NO 12, SEQ ID NO 44, SEQ ID NO 45 nach Anspruch 1 oder
- irgendeinem der Oligonucleotide SEQ ID NO 14, SEQ ID NO 46, SEQ ID NO 47 und irgendeinem der Oligonucleotide SEQ ID NO 15, SEQ ID NO 48, SEQ ID NO 49 nach Anspruch 1 oder
- irgendeinem der Oligonucleotide SEQ ID NO 16, SEQ ID NO 50, SEQ ID NO 51 und irgendeinem der Oligonucleotide SEQ ID NO 17, SEQ ID NO 52, SEQ ID NO 53 nach Anspruch 1 oder
- irgendeinem der Oligonucleotide SEQ ID NO 18, SEQ ID NO 54, SEQ ID NO 55 und irgendeinem der Oligonucleotide SEQ ID NO 19, SEQ ID NO 56, SEQ ID NO 57 nach Anspruch 1 oder
- irgendeinem der Oligonucleotide SEQ ID NO 23, SEQ ID NO 58, SEQ ID NO 59 und irgendeinem der Oligonucleotide SEQ ID NO 24, SEQ ID NO 60, SEQ ID NO 61 nach Anspruch 1 oder
- irgendeinem der Oligonucleotide SEQ ID NO 25, SEQ ID NO 62, SEQ ID NO 63 und irgendeinem der Oligonucleotide SEQ ID NO 26, SEQ ID NO 64, SEQ ID NO 65 nach Anspruch 1 oder
- irgendeinem der Oligonucleotide SEQ ID NO 70, SEQ ID NO 71, SEQ ID NO 72 und irgendeinem der Oligonucleotide SEQ ID NO 73, SEQ ID NO 74, SEQ ID NO 75 nach Anspruch 1 oder
- irgendeinem der Oligonucleotide SEQ ID NO 27, SEQ ID NO 66, SEQ ID NO 67 und irgendeinem der Oligonucleotide SEQ ID NO 28, SEQ ID NO 68, SEQ ID NO 69 nach Anspruch 1
und vorteilhafterweise gebildet sind aus dem Paar von Oligonucleotiden, ausgewählt aus den folgenden Paaren:
- SEQ ID NO 1 und SEQ ID NO 5,
- SEQ ID NO 2 und SEQ ID NO 5,
- SEQ ID NO 1 und SEQ ID NO 4,
- SEQ ID NO 2 und SEQ ID NO 4,
- SEQ ID NO 3 und SEQ ID NO 5,
- SEQ ID NO 11 und SEQ ID NO 12,
- SEQ ID NO 14 und SEQ ID NO 15,
- SEQ ID NO 16 und SEQ ID NO 17,
- SEQ ID NO 18 und SEQ ID NO 19,
- SEQ ID NO 23 und SEQ ID NO 24,
- SEQ ID NO 25 und SEQ ID NO 26,
- SEQ ED NO 72 und SEQ ID NO 75,
- SEQ ID NO 27 und SEQ ID NO 28.

3. Verfahren zum Nachweis des Vorhandenseins biologischer Materialien, die von Vögeln, insbesondere Anseriformes, Galliformes oder Struthioniformes stammen, in einer Probe von organischem Material, **dadurch gekennzeichnet, dass** man das Vorhandensein von DNA, die von Vögeln stammt, in diesem organischen Material durch Amplifikation einer DNA-Sequenz bestimmt, die für das Genom der Vögel spezifisch und in der aus der Probe extrahierten DNA enthalten ist, nämlich einer Sequenz, die im Genom der Vögel vorhanden, aber im Genom anderer Tiergattungen, insbesondere anderer Tierspezies, nicht vorhanden ist,
wobei diese Sequenz eine Sequenz mitochondrialer DNA ist, die für das Genom der Vögel spezifisch ist, amplifiziert mit Hilfe von Oligonucleotiden, die aus den in Anspruch 1 definierten Oligonucleotiden ausgewählt sind.

4. Verfahren nach Anspruch 3, welches auch die Identifizierung der Gattung, insbesondere der Spezies der Vögel, insbesondere Anseriformes, Galliformes oder Struthioniformes umfasst, die in der Probe vorhanden ist, **dadurch gekennzeichnet, dass** man die für das Genom der Vögel spezifische DNA-Sequenz, die wie im Anspruch 3 definiert amplifiziert wurde, mit anderen DNA-Sequenzen des Genoms der Vögel vergleicht, wobei die so amplifizierte für das Genom der Vögel spezifische DNA-Sequenz mindestens 50 % Identität, insbesondere 60 % Identität mit den anderen DNA-Sequenzen des Genoms der Vögel aufweist.

5. Verfahren nach Anspruch 3 oder Anspruch 4, **dadurch gekennzeichnet, dass** es den Nachweis und/oder die Identifikation des Vorhandenseins von Anseriformes erlaubt, insbesondere von Anatidae, die ausgewählt sind aus der Gruppe bestehend aus Enten, wie der Spießente *(Anas acuta*), der Mandarinente (*Aix galericulata*), der Löffelente *(Anas clypeata*), der Knäkente *(Anas querquedula*), der Eiderente (*Somateria mollissima*), Gänsen, wie der Zwergblässgans *(Anser erythropus*), der Schneegans *(Anser caerulescens*), der Kanadagans *(Branta canadensis*), und Schwänen, wie dem Singschwan *(Cygnus cygnus*), dem Trauerschwan *(Cygnus atratus*), dem Höckerschwan *(Cygnus color*), und insbesondere den domestizierten Entenspezies, wie Stockente *(Anas platyrhynchos*), Moschusente *(Cairina moschata*), oder domestizierten Gänsespezies, wie Graugans *(Anser anser*).

6. Verfahren nach einem der Ansprüche 3 bis 5, **dadurch gekennzeichnet, dass** die Amplifikation der für das Genom der Vögel, insbesondere der Anseriformes, Galliformes oder Struthioniformes, spezifischen DNA-Sequenz nach dem Amplifizierungsverfahren der Polymerase-Kettenreaktion (PCR) erfolgt, umfassend die Wiederholung des Zyklus der folgenden Schritte:
- Erwärmen der aus der Probe organischen Materials extrahierten DNA, um die DNA in zwei Einzelstränge zu trennen,
- Hybridisieren von Oligonucleotid-Primern nach Anspruch 1 oder 2 mit den Einzelsträngen der DNA bei einer geeigneten Temperatur und
- Verlängern der Oligonucleotid-Primer durch eine Polymerase bei einer geeigneten Temperatur, um eine amplifizierte DNA-Sequenz oder ein amplifiziertes DNA-Fragment zu erhalten, die/das für das Genom der Vögel, insbesondere Anseriformes, Galliformes oder Struthioniformes, spezifisch ist.

7. Verfahren nach einem der Ansprüche 3 bis 6, **dadurch gekennzeichnet, dass** das erhaltene amplifizierte DNA-Fragment identifiziert wird:
- durch Sequenzierung des amplifizierten Fragments,
- durch Gelelektrophorese der erhaltenen Fragmente nach Verdauung des amplifizierten Fragments mit Restriktionsenzymen,
- direkt durch Visualisierung des Vorhandenseins des amplifizierten Fragments mittels Gelelektrophorese.

8. Verfahren nach einem der Ansprüche 3 bis 7, **dadurch gekennzeichnet, dass** die aus der Probe organischen Materials extrahierte DNA ist:
- nicht abgebaute DNA, insbesondere aus einer frischen Probe stammend,
- abgebaute DNA, insbesondere aus einer transformierten Probe stammend, insbesondere gekocht, lyophilisiert, getrocknet, gepökelt, appertisiert oder pasteurisiert.

9. DNA-Fragment, amplifiziert nach dem Verfahren nach einem der Ansprüche 3 bis 8, **dadurch gekennzeichnet, dass** es eine Sequenzidentität von mindestens 80 %, vorzugsweise 90 % und vorteilhafterweise 95 % aufweist mit:
- der folgenden SEQ ID NO 6: ACYAGCTTCA GGCCCATACG TTCCCCCTAA ACCCCTCGCC CTCCTCACAT TTTTGCGCCT 
CTGGTTCCTC GGTCAGGGCC ATCMATTGGG TTCACTCACC YCYMYTYGCC YTTCAAAGTG
GCATCTGTGG ANKACBTYCA CCWYYYCRRT GCGTWATCGC GGCATBYTYM ASYWTTTWSM
CGCCTYTGGT TCYMYTTHTY TYT
 worin Y gleich C oder T ist, M gleich A oder C ist, K gleich G oder T ist, B gleich C, G oder T ist, R gleich A oder G ist, W gleich A oder T ist, S gleich C oder G ist, H gleich A, C oder T ist, N gleich A, C, G oder T ist,
wobei diese Sequenz SEQ ID NO 6 203 Basenpaare aufweist,
- oder der folgenden Sequenz SEQ ID NO 7: 
ACYAGCTTCA GGCCCATACG TTCCCCCTAA ACCCCTCGCC CTCCTCACAT TTTTGCGCCT
CTGGTTCCTC GGTCAGGGCC ATCMATTGGG TTCACTCACC YCYMYTYGCC YTTCAAAGTG
GCATCTGTGG ANKACBT
 worin Y gleich C oder T ist, M gleich A oder C ist, N gleich A, C, G oder T ist, K gleich G oder T ist, B gleich C, G oder T ist,
wobei diese Sequenz SEQ ID NO 7 137 Basenpaare aufweist,
- oder der folgenden Sequenz SEQ ID NO 8: 
AACCCCTCGC CCTCCTCACA TTTTTGCGCC TCTGGTTCCT CGGTCAGGGC CATCMATTGG
GTTCACTCAC CYCYMYTYGC CYTTCAAAGT GGCATCTGTG GANKACBTYC ACCWYYYCRR
TGCGTWATCG CGGCATBYTY MASYWTTTWS MCGCCTYTGG TTCYMYTTHT YTYT
 worin Y gleich C oder T ist, M gleich A oder C ist, N gleich A, C, G oder T ist, K gleich G oder T ist, B gleich C, G oder T ist, R gleich A oder G ist, W gleich A oder T ist, S gleich C oder G ist, H gleich A, C oder T ist,
wobei diese Sequenz SEQ ID NO 8 174 Basenpaare aufweist,
- oder der folgenden Sequenz SEQ ID NO 9: 
AACCCCTCGC CCTCCTCACA TTTTTGCGCC TCTGGTTCCT CGGTCAGGGC CATCMATTGG
GTTCACTCAC CYCYMYTYGC CYTTCAAAGT GGCATCTGTG GANKACBT
 worin Y gleich C oder T ist, M gleich A oder C ist, N gleich A, C, G oder T ist, K gleich G oder T ist, B gleich C, G oder T ist,
wobei diese Sequenz SEQ ID NO 9 108 Basenpaare aufweist,
- oder der folgenden Sequenz SEQ ID NO 10: 
ATCMATTGGG TTCACTCACC YCYMYTYGCC YTTCAAAGTG GCATCTGTGG ANKACBTYCA
CCWYYYCRRT GCGTWATCGC GGCATBYTYM ASYWTTTWSM CGCCTYTGGT TCYMYTTHTY
TYT
 worin Y gleich C oder T ist, M gleich A oder C ist, N gleich A, C, G oder T ist, K gleich G oder T ist, B gleich C, G oder T ist, R gleich A oder G ist, W gleich A oder T ist, S gleich C oder G ist, H gleich A, C oder T ist,
wobei diese Sequenz SEQ ID NO 10 123 Basenpaare aufweist,
- oder der folgenden Sequenz SEQ ID NO 13: 
YMYYKRYACT GATGCACTTK GDYCDCATTY RGTTRWKGYY MYNTCCRCHV HYCYABNBWH
WVRTGYTRYY NRGTGAATGC TYGHHGGACA TADYWYWAWM MAWHWWMHYW YYHWSYRYWW
YYYHYRSRMH HHMNRAAYDM AACCARHRDW YTTYMDCHRW THWDHWWHMM WHYWYVTWDY
RTMWWHYHHV HMHHHHMHWM HHBHAWWHHH HNHNHYNHWA YYBCHNNYHH HWAWYNYSYY
WHHYNWBVHW HHNBYBDMDM DWCHDWMMNR WWHDMAYDDH DYVDHHYAWM HTTATTAKAG
RAACWCCAGY ACYARVVVHD VWHNHAMHYN RRHRWHHMYY AYWAHYYHVH YNTYNMNAMH
HNBNVYHYRD HYHRYYARYB RMHYVHHHYR YYCDCMTWSY WYMMHNHH
 worin Y gleich C oder T ist, M gleich A oder C ist, N gleich A, C, G oder T ist, K gleich G oder T ist, B gleich C, G oder T ist, R gleich A oder G ist, W gleich A oder T ist, S gleich C oder G ist, D gleich A, G oder T ist, V gleich A, C oder G ist, H gleich A, C oder T ist,
wobei diese Sequenz SEQ ID NO 13 408 Basenpaare aufweist,
- oder der folgenden Sequenz SEQ ID NO 77: 
YMYYKRCTGA TGCACTTKGD YCDCATTYRG TTRWKGYYMY CCRCHVHYCY ABNBWHWVRT
GYTRYYNRGT GAATGCTYGH HGGACATADY WYWAWMMAWH WMHYWYYHWS YRYWWYYYHY
RSRMHHHMRA AYDMAACCAR HRDWYTTYMD CHRWTHDYRT MWWHYHVHMH HNHWAYYBCH
NNYHDMDWCH DWMMRWWHDM AYDDHDYVDH HYAWHTTATT AKAGRAACWC CAGYACYARV
VVHDVWHNHA MHYNRRHRWH MYYAYWAHYY HVHYNTYNMN AMHHNBNVYH YRDHYHRYYA
RYBRMHYVHH HYRYYCDCMT WSYWYMMHNH H
 worin Y gleich C oder T ist, M gleich A oder C ist, N gleich A, C, G oder T ist, K gleich G oder T ist, B gleich C, G oder T ist, R gleich A oder G ist, W gleich A oder T ist, S gleich C oder G ist, D gleich A, G oder T ist, V gleich A, C oder G ist, H gleich A, C oder T ist,
wobei diese Sequenz SEQ ID NO 77 331 Basenpaare aufweist,
- oder der folgenden Sequenz SEQ ID NO 78: 
YMYYKRYACT GATGCACTTK GDYCDCATTY RGTTRWKGYY MYNTCCRCHV HYCYABNBWH
WVRTGYTRYY NRGTGAATGC TYGHHGGACA TAMHHHMNRA AYDMAACCAY DMAACHRHYH
HVHMHHHNHN HYNHHNNYRH HWAYYBCHNN YHHWAWYMYS YYWHHYNWBV HWHNBYBDMD
 
MDWCHDWMNR WWHDMAYDDH DYVDHHYAWM HTTATTAKAG RAACWCCAGY ACYARVVVHD
VWHNHAMHYN RRHRWHHMYY AYWAHYYHVH YNTYNMNAMH HNBNVYHYRD HYHRYYARYB
RMHYVHHHYR YYCDCMTWSY WYMMHNHH
 worin Y gleich C oder T ist, M gleich A oder C ist, N gleich A, C, G oder T ist, K gleich G oder T ist, B gleich C, G oder T ist, R gleich A oder G ist, W gleich A oder T ist, S gleich C oder G ist, D gleich A, G oder T ist, V gleich A, C oder G ist, H gleich A, C oder T ist,
wobei diese Sequenz SEQ ID NO 78 328 Basenpaare aufweist,
- oder der folgenden Sequenz SEQ ID NO 79: 
YMYYKRYACT GATGCACTTK GDYCDCATTY RGTTRWKGYY MYNTCCRCHV HYCYABNBWH
WVRTGYTRYY NRGTGAATGC TYGHHGGACA TAMHHHMNRA AYDMAACHRH YHHVHMHHHN
HNHYNHHNNY HHHWAYWBVH WHWCHDWMMN RWWHDMAYDD HDYVDHHYAW MHTTATTAKA
GRAACWCCAG YACYARVVVH DVWHNHAMHY NRRHRWHHMY YAYWAHYYHV HYNTYNMNMH
HNBNVYHYRD HYHRYYARYB RMHYVHHHYR YYCDCMTWSY WYMMHNHH
 worin Y gleich C oder T ist, M gleich A oder C ist, N gleich A, C, G oder T ist, K gleich G oder T ist, B gleich C, G oder T ist, R gleich A oder G ist, W gleich A oder T ist, S gleich C oder G ist, D gleich A, G oder T ist, V gleich A, C oder G ist, H gleich A, C oder T ist,
wobei diese Sequenz SEQ ID NO 79 288 Basenpaare aufweist,
- oder der folgenden Sequenz SEQ ID NO 20: 
HWBDNHMRMG RNCATHHHHN NYHVHBDNAH NNNHCHHCCA VVRMHHHNNV RTGMAYGHYC
YHRNDVHHHH NHVNHVVMHV VMHVNVHNHN NHHHNHVHVV RWNMACMARR RCMMHDHNNR
VTGAATGCTH NMHRRACAWR DMNYNDVHAH HMHTCYDDNB YYBHHTCCAC CCRMMCATWH
 worin Y gleich C oder T ist, M gleich A oder C ist, N gleich A, C, G oder T ist, B gleich C, G oder T ist, R gleich A oder G ist, W gleich A oder T ist, D gleich A, G oder T ist, V gleich A, C oder G ist, H gleich A, C oder T ist,
welche Sequenz SEQ ID NO 20 180 Basenpaare aufweist,
- oder der folgenden Sequenz SEQ ID NO 21: 
WKGYYMYNTC CRCHVHYCYA BNBWHWVRTG YTRYYNRGTG AATGCTYGHH GGACATAMHH
HMNRAAYDMA ACHRHYHHVH MHHHNHNHNH HNNYHHHWAY WBVHWHWCHD WMMNRWWHDM
AYDDHDYVDH HYAWMHTTAT TAKAGRAACW CCAGYACYAR VVVHDVWHNH AMHYNRRHRW
HHMYYAYWAH Y
 worin Y gleich C oder T ist, M gleich A oder C ist, N gleich A, C, G oder T ist, B gleich C, G oder T ist, R gleich A oder G ist, W gleich A oder T ist, D gleich A, G oder T ist, V gleich A, C oder G ist, H gleich A, C oder T ist, K gleich G oder T ist,
wobei diese Sequenz SEQ ID NO 21 191 Basenpaare aufweist,
- oder der folgenden Sequenz SEQ ID NO 22: 
RTGAAYGHYC YHRNDVHHNH MVHNHNVMHV VNHNHHNHNH VVVSWNMARD SCMNHNNTTM
VTGAATGCTH NMHRRACAWD MNYNDVHAHH MHTCYNDNBH YBHHTCCACC CRMMCATWHM
TYATGRAVYY NCGTAYCARR TGGATTTATT RRYCGTACWC CTCACGTGAA ATCAGC
 worin Y gleich C oder T ist, M gleich A oder C ist, N gleich A, C, G oder T ist, B gleich C, G oder T ist, R gleich A oder G ist, W gleich A oder T ist, D gleich A, G oder T ist, V gleich A, C oder G ist, H gleich A, C oder T ist, S gleich C oder G ist,
wobei diese Sequenz SEQ ID NO 22 176 Basenpaare aufweist,
- oder der folgenden Sequenz SEQ ID NO 29: 
KMAWVNGGHM WTVACHYATA TTCCMCWTYY CKYMCHAHVB MCHYTVDATG MAYSMTSHHRK
RCMHVCTMHN TTHCVYDHMC CATAGABAGT TCCWAACHAC WATCRRKVMM CMTAACTATG
AATGGTTRCVG HACATAAATC TCACTCTCAT GTTCTCCCCC CAACAAGTC CCTAACTATG
AATGGTTACAG GACATACATT TDACTAYCAT GTTCTAACCS ATTTGGTT
 worin Y gleich C oder T ist, M gleich A oder C ist, N gleich A, C, G oder T ist, B gleich C, G oder T ist, R gleich A oder G ist, W gleich A oder T ist, D gleich A, G oder T ist, V gleich A, C oder G ist, H gleich A, C oder T ist, S gleich C oder G ist, K gleich G oder T ist,
wobei diese Sequenz SEQ ID NO 29 231 Basenpaare aufweist,
- oder der folgenden Sequenz SEQ ID NO 30: 
TACAWACYMW CGYAWWRRWD CCCTYAMAYY AYAHAAMCGY YTATCGYWTA DTATATATAC
ATTRYWRYDY AYYMYAYMAT TATTAKAGAA ACYCCMCTAC CRAAACMAWC AWHARARRCW
WAHADHWAHA TKCMRCDYAW VBYHCYTCAS AWRCMWWBRT KRTWTATATT GTTAA
 worin Y gleich C oder T ist, M gleich A oder C ist, B gleich C, G oder T ist, R gleich A oder G ist, W gleich A oder T ist, D gleich A, G oder T ist, V gleich A, C oder G ist, H gleich A, C oder T ist, S gleich C oder G ist, K gleich G oder T ist,
wobei diese Sequenz SEQ ID NO 30 175 Basenpaare aufweist,
- oder der folgenden Sequenz SEQ ID NO 76: MCAGTGMSKK YGSCRGRGTV CYAYTCAAKY KRAGCCTGGA YTACWCCTGC GTTRCGYSCT 
ATYCTAKWYY TCHVGKRTYM CTCRATGAKA CGGTTKGCGT RTATKKGGWA TCAYYTTGAC
ACTGATGCAC TTTGGAYCRC ATTYRGTTAW KGYTCTYCCR CMSYYCYNKW WARTRGKGYT
ATWTAGTGAA TGCTYGHYGG ACATAYYTTA WSYNAAWWYY CMCYYCMWCY A
 worin M gleich A oder C ist, S gleich C oder G ist, K gleich G oder T ist, Y gleich C oder T ist, R gleich A oder G ist, V gleich A, C oder G ist, W gleich A oder T ist, N gleich A, C, T oder G ist,
wobei diese Sequenz SEQ ID NO 76 231 Basenpaare aufweist,
- oder der folgenden Sequenz SEQ ID NO 31: 
TGAATGCTTG YYGGACATWW TWYMAYYWAT TWTCACTTCC WCTGAYTTTY YTMACAMMAC
YAGGAYWMAW YTYYTWYDTY DKTWYWAAYW YWTKWTTTVW TYAYWTYAAW MACATYTTTT
AMYTAWAYTA MCYWTACAWA CYMWCMGYAY WRRWTDTYAM AYYAAHAAMC GYYCGYWTAD
TATATATACA TARYWRYTGY RYYTMYAYCM ATTATT
 worin Y gleich C oder T ist, M gleich A oder C ist, R gleich A oder G ist, W gleich A oder T ist, D gleich A, G oder T ist, V gleich A, C oder G ist, H gleich A, C oder T ist,
wobei diese Sequenz SEQ ID NO 31 216 Basenpaare aufweist.

10. Verwendung von Nucleotidsequenzen, ausgewählt aus den Oligonucleotiden, wie sie in einem der Ansprüche 1 oder 2 definiert sind, oder DNA-Fragmenten nach Anspruch 9 zur Durchführung eines Verfahrens zum Nachweis des Vorhandenseins biologischer Materialien, die von Vögeln stammen, insbesondere von Anseriformes, Galliformes oder Struthioniformes, und/oder zur Identifizierung der vorhandenen Vogelspezies, insbesondere der Anseriformes, Galliformes oder Struthioniformes, in einer Probe organischen Materials, die solche biologischen Materialien enthalten kann.

11. Verwendung nach Anspruch 10 zur Durchführung eines Verfahrens zum Nachweis des Vorhandenseins biologischer Materialien, die von Vögeln stammen, insbesondere von Anseriformes, Galliformes oder Struthioniformes, und/oder zur Identifizierung der Spezies, insbesondere der Anseriformes, Galliformes oder Struthioniformes, in frischen Produkten oder in transformierten Produkten, wie Nahrungsmittelprodukten, insbesondere Stopfleber, Pasteten, Fleisch, Fetten, zubereiteten Gerichten, Eiern, Fertigerzeugnissen, wie jenen auf Basis von Federn.
